# EUROPEAN PATENT APPLICATION

(11) **EP 4 339 213 A1**
(43) Date of publication of application: **20.03.2024**
(21) Application number: 22806844.1
(22) Date of filing: 12.05.2022
(51) Int. Cl.: C07K 16/46, C07K 16/28, A61K 39/395, A61K 38/17, A61P 35/00, C12N 15/62, G01N 33/53, C07K 16/00, C12N 15/12

(54) **ANTIGEN-BINDING MOLECULE**

(30) Priority: 14.05.2021 CN 202110527339
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: YING, Hua, Shanghai 200245 (CN); HU, Qiyue, Shanghai 200245 (CN); JIN, Xinsheng, Shanghai 200245 (CN); SHI, Jinping, Shanghai 200245 (CN); ZHANG, Ling, Shanghai 200245 (CN); MAO, Langyong, Shanghai 200245 (CN); YE, Xin, Shanghai 200245 (CN); TAO, Weikang, Shanghai 200245 (CN)
(74) Representative: Regimbeau
(86) International application number: PCT/CN2022/092529
(87) International publication number: WO 2022/237882

(57) **Abstract**

Provided is an antigen-binding molecule, particularly a domain-engineered antibody, wherein at least one constant region domain CH1/CL of the antibody is replaced by a Titin T-chain/Obscurin-O chain.

## Description

### TECHNICAL FIELD

The present disclosure relates to the field of antibody drugs, and specifically includes an antigen-binding molecule in which CH1 and CL structures are replaced and use thereof.

### BACKGROUND

The statements herein merely provide background information related to the present disclosure and may not necessarily constitute the prior art.

With the continuous improvement of antibody humanization technology, monoclonal antibodies have been developed rapidly in recent years. Various monoclonal antibodies have been used to treat major diseases such as malignant tumors and autoimmune diseases. However, the immune escape of tumors is often accompanied by a variety of different mechanisms, and a single monoclonal antibody can only bind to one specific target, which can greatly reduce the therapeutic effect of the monoclonal antibody.

The bispecific antibody (BsAb) is an artificial antibody formed by combining antibodies targeting two different antigens or two different epitopes together by using a genetic engineering means. Different from the monoclonal antibody, the bispecific antibody has the ability to target two different antigens or two different epitopes simultaneously, and can play special biological functions, such as immune cell recruitment, receptor co-stimulation or co-inhibition, multivalent virus neutralization, etc., and are expected to obtain better clinical therapeutic effects than single monoclonal antibodies, even antibody combinations.

A wide variety of recombinant bispecific antibodies have been developed, such as tetravalent bispecific antibodies formed by fusion of, for example, an IgG antibody and a single-chain domain (see Coloma, M.J., et al., Nature Biotech.15 (1997) 159-163; Morrison, S.L., Nature Biotech. 25 (2007) 1233-1234). There are other bispecific forms, such as DVD-Ig, CrossMab, and BiTE (Spiess et al., Molecular Immunology, 67 (2), pp. 95-106 (2015)).

Various structural models for bispecific antibodies have also been disclosed, for example, the introduction of the mutation "knobs-into-holes" into the Fc region (Ridgway et al., Protein Engineering, 9 (7), pp. 617-621 (1996)); the introduction of electrostatic design (Gunasekaran et al., Journal of Biological Chemistry, 285, (25), pp. 19637-19646, (2010)), or the introduction of negative state design (Kreudenstein et al., mAbs, 5 (5), pp. 646-654 (2013); Leaver-Fay et al., Structure, 24 (4), pp. 641-651 (2016)), the exchange of CH1 and CL domains (CrossMab platform) (Schaefer et al., Proceedings of the National Academy of Sciences of the United States of America, 108 (27), pp. 11187-11192 (2011)), fusion with the TCR constant region (WO2019057122A1), etc.

### SUMMARY

During the preparation of a multispecific antibody, random combinations of light and heavy chains of different antigens result in a variety of different products, only one of which is desired, and the resulting byproducts comprise an antibody lacking a light chain, a half-antibody, a heavy chain polymer, light chain and heavy chain mispairings, and the like, causing significant challenges to the development of downstream processes. To this end, the present disclosure provides a dimerized polypeptide and an antigen-binding molecule comprising the dimerized polypeptide.

In some embodiments, the present disclosure provides a dimerized polypeptide, which comprises a Titin-T chain and an Obscurin-O chain, or a Titin-T chain and an Obscurin-like-O chain, wherein,
i) the Titin-T chain is a variant of SEQ ID NO: 32, wherein the variant has amino acid residue substitutions at one or more positions selected from the group consisting of positions 60 and 64, and/or
ii) the Obscurin-O chain is a variant of SEQ ID NO: 33, wherein the variant has amino acid residue substitutions at one or more positions selected from the group consisting of positions 13, 32, 48, 66, 82, and 93;
with the proviso that:
a) when the variant does not have an amino acid residue substitution at position 13, 48, 66, 82, or 93, and has an amino acid residue substitution at position 32, the amino acid substitution at position 32 is not 32P;
b) when the variant does not have an amino acid residue substitution at position 32, 48, 66, 82, or 93, and has an amino acid residue substitution at position 13, the amino acid substitution at position 13 is not 13Y; and
c) when the variant does not have an amino acid residue substitution at position 48, 66, 82, or 93, and has amino acid residue substitutions at positions 13 and 32, the amino acid residue substitution at position 13 is not 13Y, and the amino acid residue substitution at position 32 is not 32P.

In some embodiments, provided is the dimerized polypeptide according to any one of the above, wherein,
i) the Titin-T chain is a variant of SEQ ID NO: 32, wherein the variant has amino acid residue substitutions at one or more positions selected from the group consisting of positions 60 and 64, and/or
ii) the Obscurin-O chain is a variant of SEQ ID NO: 33, wherein the variant has one or more amino acid residue substitutions selected from the group consisting of 13S, 32F, an amino acid residue substitution at position 48, an amino acid residue substitution at position 66, an amino acid residue substitution at position 82, and an amino acid residue substitution at position 93.

In some embodiments, provided is the dimerized polypeptide according to any one of the above, wherein the variant of SEQ ID NO: 32 has one or more amino acid substitutions selected from the group consisting of 60S and 64T, and/or the variant of SEQ ID NO: 33 has one or more amino acid residue substitutions selected from the group consisting of 13S, 32F, 48V, 66C, 82H, and 93C.

In some embodiments, provided is the dimerized polypeptide according to any one of the above, wherein the variant of SEQ ID NO: 32 has amino acid residue substitutions selected from the group consisting of 60S and 64T, and/or the variant of SEQ ID NO: 33 has amino acid residue substitutions selected from any one of a) to c):
a) 32F and 48V;
b) 13S, 32F, 48V, and 82H; and
c) 13S, 32F, 48V, 66C, 82H, and 93C.

In some embodiments, the present disclosure provides a dimerized polypeptide, which consists of a Titin-T chain and an Obscurin-O chain, or a Titin-T chain and an Obscurin-like-O chain, wherein,
i) the Titin-T chain is a variant of SEQ ID NO: 32, wherein the variant comprises at least amino acid residue substitutions at positions 60 and/or 64 as compared to SEQ ID NO: 32, and/or
ii) the Obscurin-O chain is a variant of SEQ ID NO: 33, wherein the variant comprises at least one or more amino acid residue substitutions at positions selected from the group consisting of positions 13, 32, 48, 66, 82, and 93 as compared to SEQ ID NO: 33;
with the proviso that:
a) when the variant does not have an amino acid residue substitution at position 13, 48, 66, 82, or 93, and has an amino acid residue substitution at position 32, the amino acid substitution at position 32 is not 32P;
b) when the variant does not have an amino acid residue substitution at position 32, 48, 66, 82, or 93, and has an amino acid residue substitution at position 13, the amino acid substitution at position 13 is not 13Y; and
c) when the variant does not have an amino acid residue substitution at position 48, 66, 82, or 93, and has amino acid residue substitutions at positions 13 and 32, the amino acid residue substitution at position 13 is not 13Y, and the amino acid residue substitution at position 32 is not 32P.

In some embodiments, provided is the dimerized polypeptide according to any one of the above, wherein,
i) the Titin-T chain is a variant of SEQ ID NO: 32, wherein the variant comprises at least one or more amino acid residue substitutions at positions 60 and/or 64 as compared to SEQ ID NO: 32, and/or
ii) the Obscurin-O chain is a variant of SEQ ID NO: 33 having one or more amino acid residue substitutions selected from the group consisting of 13S, 32F, an amino acid residue substitution at position 48, an amino acid residue substitution at position 66, an amino acid residue substitution at position 82, and an amino acid residue substitution at position 93, as compared to SEQ ID NO: 33.

In some embodiments, provided is the dimerized polypeptide according to any one of the above, wherein the variant of SEQ ID NO: 32 comprises at least amino acid residue substitutions of 60S and/or 64T as compared to SEQ ID NO: 32, and/or the variant of SEQ ID NO: 33 comprises at least one or more amino acid residue substitutions selected from the group consisting of 13S, 32F, 48V, 66C, 82H, and 93C as compared to SEQ ID NO: 33.

In some embodiments, provided is the dimerized polypeptide according to any one of the above, wherein the variant of SEQ ID NO: 32 comprises at least amino acid residue substitutions of 60S and 64T as compared to SEQ ID NO: 32, and/or the variant of SEQ ID NO: 33 comprises at least amino acid residue substitutions selected from any one of a) to c) as compared to SEQ ID NO: 33:
a) 32F and 48V;
b) 13S, 32F, 48V, and 82H; and
c) 13S, 32F, 48V, 66C, 82H, and 93C.

In some embodiments, provided is the dimerized polypeptide according to any one of the above, wherein the variant of SEQ ID NO: 32 further has amino acid residue substitutions at one or more positions selected from the group consisting of positions 3, 8, 11, 13, 20, 22, 25, 26, 39, 40, 42, 45, 47, 49, 56, 58, 66, 70, 75, 77, 79, 81, 82, 83, and 84, for example, further has 1, 2, 3, 4, 5, 6, 7, 8, 9, 10, 11, 12, 13, 14, 15, 16, 17, 18, 19, 20, 21, 22, 23, 24, or 25 amino acid residue substitutions, as compared to SEQ ID NO: 32. In some embodiments, provided is the dimerized polypeptide according to any one of the above, wherein the variant of SEQ ID NO: 32 further comprises one or more amino acid residue substitutions selected from the group consisting of 3W, 8C, 11I, 13L, 20C, 22M/22C, 25S, 26C, 39T, 40S, 42K, 45S, 47E, 49G, 56S, 58E, 66S/66K, 70R, 75V, 77S, 79T, 81R, 82M, 83D, and 84L, as compared to SEQ ID NO: 32.

In some embodiments, provided is the dimerized polypeptide according to any one of the above, the variant of SEQ ID NO: 32 further comprises amino acid residue substitutions at one or more positions selected from the group consisting of positions 8, 11, 20, 25, 26, 39, 66, 79, and 81 as compared to SEQ ID NO: 32; in some embodiments, the variant of SEQ ID NO: 32 further has one or more amino acid residue substitutions selected from the group consisting of 8C, 11I, 20C, 25S, 26C, 39T, 66K, 79T, and 81R as compared to SEQ ID NO: 32. In some embodiments, the variant of SEQ ID NO: 32 further comprises amino acid residue substitutions at positions 8, 11, 25, 39, 66, 79, and 81 as compared to SEQ ID NO: 32; in some embodiments, the variant of SEQ ID NO: 32 further comprises amino acid residue substitutions of 8C, 11I, 25S, 39T, 66K, 79T, and 81R as compared to SEQ ID NO: 32; in some embodiments, the variant of SEQ ID NO: 32 further comprises amino acid residue substitutions of 8C, 11I, 25S, 39T, 60S, 64T, 66K, 79T, and 81R, and amino acid residue substitutions of 20C and/or 26C as compared to SEQ ID NO: 32.

In some embodiments, provided is the dimerized polypeptide according to any one of the above, the variant of SEQ ID NO: 32 further comprises amino acid residue substitutions selected from any one of a) to l) as compared to SEQ ID NO: 32:
a) 8C, 25S, and 39T;
b) 20C, 25S, and 39T;
c) 25S, 26C, and 39T;
d) 22C, 25S, and 39T;
e) 8C, 25S, 39T, 66S, and 77S;
f) 8C, 25S, 39T, 66K, 70R, 79T, and 81R;
g) 3W, 8C, 11I, 13L, 22M, 25S, 39T, and 82M;
h) 8C, 11I, 25S, 39T, 66K, 79T, and 81R;
i) 8C, 25S, 39T, 40S, 42K, 45S, 47E, 49G, 56S, 58E, 75V, 83D, and 84L;
j) 8C, 25S, 39T, 47E, 49G, 56S, 58E, and 75V;
k) 8C, 25S, 39T, 56S, 58E, and 75V; and
I) 8C, 25S, 39T, 56S, 58E, 66S, and 77S;

In some embodiments, provided is the dimerized polypeptide according to any one of the above, wherein the variant of SEQ ID NO: 32 has amino acid residue substitutions selected from any one of a) to c) as compared to SEQ ID NO: 32:
A) 8C, 11I, 25S, 39T, 60S, 64T, 66K, 79T, and 81R;
B) 8C, 11I, 20C, 25S, 39T, 60S, 64T, 66K, 79T, and 81R, and
C) 8C, 11I, 25S, 26C, 39T, 60S, 64T, 66K, 79T, and 81R.

In some embodiments, provided is the dimerized polypeptide according to any one of the above, wherein the variant of SEQ ID NO: 33 further has amino acid residue substitutions at one or more positions selected from the group consisting of positions 2, 3, 7, 9, 11, 12, 13, 14, 17, 20, 22, 25, 30, 32, 34, 36, 41, 42, 44, 45, 53, 58, 62, 67, 69, 76, 88, 89, 92, 94, and 97 as compared to SEQ ID NO: 33; in some embodiments, the variant of SEQ ID NO: 33 further has one or more amino acid residue substitutions selected from the group consisting of 2E, 3C, 7K/7R, 9C, 11L, 12S, 13Y, 14T, 17E, 20L, 22M/22S, 25S, 30D, 32P, 34E, 36T, 41K, 42L, 441, 45T, 53L, 58V, 62E/62K/62H, 67Q/67T, 69S, 76S, 88C, 89L, 92E, 94G, and 97G as compared to SEQ ID NO: 33.

In some embodiments, provided is the dimerized polypeptide according to any one of the above, wherein the variant of SEQ ID NO: 33 has amino acid residue substitutions selected from any one of A) to R) as compared to SEQ ID NO: 33:
A) 88C;
B) 3C;
C) 9C;
D) 25S, 76S, and 88C;
E) 25S, 76S, and 3C;
F) 25S, 76S, and 9C;
G) 7K, 25S, 62K, 76S, and 88C;
H) 7K, 25S, 62H, 76S, and 88C;
I) 7R, 25S, 62K, 76S, and 88C;
G) 7R, 25S, 62H, 76S, and 88C;
K) 11L, 25S, 62K, 76S, and 88C;
L) 11L, 25S, 62H, 76S, and 88C;
M) 12S, 13Y, 14T, 22S, 25S, 62K, 76S, and 88C;
N) 2E, 11L, 17E, 25S, 30D, 32P, 34E, 36T, 441, 45T, 58V, 62E, 67Q, 69S, 76S, 88C, and 97G;
O) 11L, 20L, 22M, 25S, 53L, 62K, 76S, and 88C;
P) 11L, 25S, 41K, 45T, 62K, 67Q, 69S, 76S, 88C, and 89L;
Q) 11L, 25S, 42L, 45T, 62K, 67T, 69S, 76S, 88C, 92E, and 94G; and
R) 11L, 12S, 13Y, 22S, 25S, 42L, 45T, 62K, 67Q, 69S, 76S, 88C, 92E, and 94G.

In some embodiments, provided is the dimerized polypeptide according to any one of the above, wherein the variant of SEQ ID NO: 33 further has amino acid residue substitutions at one or more positions selected from the group consisting of positions 3, 9, 25, 41, 45, 62, 67, 69, 76, 88, and 89 as compared to SEQ ID NO: 33; in some embodiments, the variant of SEQ ID NO: 33 further has one or more amino acid residue substitutions selected from the group consisting of 3C, 9C, 25S, 41K, 45T, 62K, 67Q, 69S, 76S, 88C, and 89L as compared to SEQ ID NO: 33.

In some embodiments, the dimerized polypeptide according to any one of the above, wherein the variant of SEQ ID NO: 33 further has amino acid residue substitutions at one or more positions selected from the group consisting of positions 41, 45, 62, 67, 69, 88, and 89 as compared to SEQ ID NO: 33; in some embodiments, the variant of SEQ ID NO: 33 further has one or more amino acid residue substitutions selected from the group consisting of 41K, 45T, 62K, 67Q, 69S, 88C, and 89L as compared to SEQ ID NO: 33; in some embodiments, the variant of SEQ ID NO: 33 has amino acid residue substitutions of 41K, 45T, 62K, 67Q, 69S, 88C, and 89L.

In some embodiments, provided is the dimerized polypeptide according to any one of the above, wherein the variant of SEQ ID NO: 33 has amino acid residue substitutions of 13S, 32F, 48V, and 82H, and one or more amino acid residue substitutions selected from the group consisting of 3C, 9C, 25S, 41K, 45T, 62K, 67Q, 69S, 76S, 88C, and 89L as compared to SEQ ID NO: 33. In some embodiments, the variant of SEQ ID NO: 33 further has one or more amino acid residue substitutions selected from the group consisting of 41K, 45T, 62K, 67Q, 69S, and 89L, for example, has amino acid residue substitutions of 41K, 45T, 62K, 67Q, 69S, and 89L, as compared to SEQ ID NO: 33; in some embodiments, the variant of SEQ ID NO: 33 further has amino acid residue substitutions at one or more positions selected from the group consisting of 3C, 9C, 25S, 66C, 76S, 88C, and V93C, for example, has amino acid residue substitutions selected from any one of a) to j), as compared to SEQ ID NO: 33: a) 25S, 76S, and 88C; b) 3C, 25S, 76S, and 88C; c) 9C, 25S, 76S, and 88C; d) 88C; e) 3C and 88C; f) 9C and 88C; g) 25S, 66C, 76S, 88C, and 93C; h) 9C, 25S, 66C, 76S, 88C, and 93C; i) 3C, 25S, 66C, 76S, 88C, and 93C; and j) 3C, 9C, 25S, 66C, 76S, and 93C.

In some embodiments, provided is the dimerized polypeptide according to any one of the above, wherein the variant of SEQ ID NO: 33 further has amino acid residue substitutions at one or more positions selected from the group consisting of positions 25, 41, 45, 62, 67, 69, 76, 88, and 89 as compared to SEQ ID NO: 33; in some embodiments, the variant of SEQ ID NO: 33 further has one or more amino acid residue substitutions selected from the group consisting of 25S, 41K, 45T, 62K, 67Q, 69S, 76S, 88C, and 89L as compared to SEQ ID NO: 33.

In some embodiments, provided is the dimerized polypeptide according to any one of the above, wherein the variant of SEQ ID NO: 33 has amino acid residue substitutions selected from any one of a) to j) as compared to SEQ ID NO: 33:
a) 25S, 32F, 41K, 45T, 48V, 62K, 67Q, 69S, 76S, 88C, and 89L;
b) 13S, 25S, 32F, 41K, 45T, 48V, 62K, 67Q, 69S, 76S, 82H, 88C, and 89L;
c) 3C, 13S, 25S, 32F, 41K, 45T, 48V, 62K, 67Q, 69S, 76S, 82H, 88C, and 89L;
d) 9C, 13S, 25S, 32F, 41K, 45T, 48V, 62K, 67Q, 69S, 76S, 82H, 88C, and 89L;
e) 13S, 32F, 41K, 45T, 48V, 62K, 67Q, 69S, 82H, 88C, and 89L;
f) 3C, 13S, 32F, 41K, 45T, 48V, 62K, 67Q, 69S, 82H, 88C, and 89L;
g) 9C, 13S, 32F, 41K, 45T, 48V, 62K, 67Q, 69S, 82H, 88C, and 89L;
h) 13S, 25S, 32F, 41K, 45T, 48V, 62K, 66C, 67Q, 69S, 76S, 82H, 88C, 89L, and 93C;
i) 3C, 13S, 25S, 32F, 41K, 45T, 48V, 62K, 66C, 67Q, 69S, 76S, 82H, 88C, 89L, and 93C; and
j) 9C, 13S, 25S, 32F, 41K, 45T, 48V, 62K, 66C, 67Q, 69S, 76S, 82H, 88C, 89L, and 93C.

In some embodiments, provided is the dimerized polypeptide according to any one of the above, wherein the Obscurin-like-O chain is SEQ ID NO: 34 or a variant thereof, wherein the variant of SEQ ID NO: 34 has amino acid residue substitutions at one or more positions selected from the group consisting of positions 6, 26, 74, 77, 84, and 86 as compared to SEQ ID NO: 34; the positions for the amino acid residue substitutions of the variant of SEQ ID NO: 34 are natural sequence numbering positions relative to the sequence SEQ ID NO: 34. In some embodiments, the variant of SEQ ID NO: 34 has one or more amino acid residue substitutions selected from the group consisting of 6E, 26S, 74C, 77S, 84C, and 86C as compared to SEQ ID NO: 33; in some embodiments, the variant of SEQ ID NO: 34 has amino acid residue substitutions selected from any one of A) to F) as compared to SEQ ID NO: 33:
A) 6E and 74C;
B) 6E and 84C;
C) 6E and 86C;
D) 6E, 26S, 77S, and 74C;
E) 6E, 26S, 77S, and 84C; and
F) 6E, 26S, 77S, and 86C.

In some embodiments, provided is the dimerized polypeptide according to any one of the above, wherein the Titin-T chain is a variant of SEQ ID NO: 32, 68, or 127, wherein the variant has one or more amino acid residue substitutions selected from the group consisting of 60S and 64T, and the Obscurin-O chain is a variant of SEQ ID NO: 33, 80, or 128, and the variant has one or more amino acid residue substitutions selected from the group consisting of 13S, 32F, 48V, 66C, 82H, and 93C. The residue positions in the Titin-T chain are natural sequence numbering positions relative to a sequence SEQ ID NO: 32; the residue positions in the Obscurin-O chain are natural sequence numbering positions relative to a sequence SEQ ID NO: 33.

In some embodiments, provided is the dimerized polypeptide according to any one of the above, wherein the Titin-T chain is a variant of SEQ ID NO: 32, 68, or 127, wherein the variant and has amino acid residue substitutions selected from the group consisting of 60S and/or 64T as compared to SEQ ID NO: 32, 68, or 127; the Obscurin-O chain is a variant of SEQ ID NO: 33, 80, or 128, wherein the variant has one or more amino acid residue substitutions selected from the group consisting of 13S, 32F, 48V, 66C, 82H, and 93C as compared to SEQ ID NO: 33, 80, or 128. The amino acid residue positions in the Titin-T chain are natural sequence numbering positions relative to a sequence SEQ ID NO: 32; the amino acid residue positions in the Obscurin-O chain are natural sequence numbering positions relative to a sequence SEQ ID NO: 33.

In some embodiments, provided is the dimerized polypeptide according to any one of the above, wherein the Titin-T chain has at least 85% (e.g., at least 86%, 87%, 88%, 89%, 90%, 91%, 92%, 93%, 94%, 95%, 96%, 97%, 98%, or 99%) sequence identity to any one of amino acid sequences of SEQ ID NO: 132 to SEQ ID NO: 141, and the Obscurin-O chain has at least 85% (e.g., at least 86%, 87%, 88%, 89%, 90%, 91%, 92%, 93%, 94%, 95%, 96%, 97%, 98%, or 99%) sequence identity to any one of amino acid sequences of SEQ ID NO: 129 to SEQ ID NO: 131. In some embodiments, provided is the dimerized polypeptide according to any one of the above, wherein the Titin-T chain has an amino acid sequence set forth in any one of SEQ ID NO: 129 to SEQ ID NO: 131, and the Obscurin-O chain has an amino acid sequence set forth in any one of SEQ ID NO: 132 to SEQ ID NO: 141. In some embodiments, provided is the dimerized polypeptide according to any one of the above, wherein the Titin-T chain has an amino acid sequence set forth in SEQ ID NO: 129, and the Obscurin-O chain has an amino acid sequence set forth in SEQ ID NO: 133; or the Titin-T chain has an amino acid sequence set forth in SEQ ID NO: 129, and the Obscurin-O chain has an amino acid sequence set forth in SEQ ID NO: 135; or the Titin-T chain has an amino acid sequence set forth in SEQ ID NO: 129, and the Obscurin-O chain has an amino acid sequence set forth in SEQ ID NO: 136.

In some embodiments, provided is the dimerized polypeptide according to any one of the above, wherein the Titin-T chain is capable of associating with the Obscurin-O chain to form a dimerized polypeptide; in some embodiments, provided is the dimerized polypeptide according to any one of the above, wherein the Titin-T chain is capable of associating with an Obscurin-like-O chain to form a dimerized polypeptide.

In some embodiments, provided is the dimerized polypeptide according to any one of the above, wherein one or more residues at positions 7-15, 19-24, 26, 55, 59, and 60 in the Titin-T chain is associated with one or more residues at positions 3-6, 9, 41, 73, 75, and 80-90 in the Obscurin-O chain, and one or more residues at positions 1, 7-10, 13-16, 19-26, 59-60, and 96 in the Titin-T chain is associated with one or more residues at positions 4-5, 10, 12-13, 74, 76, 78, and 82-91 in the Obscurin-like-O chain. In some embodiments, the Titin-T chain comprises amino acids at positions 7-60 of SEQ ID NO: 32, and/or further comprises amino acid residue substitutions at corresponding positions according to any one of the above; the Obscurin-O chain comprises amino acids at positions 3-90 of SEQ ID NO: 33, and/or further comprises amino acid residues at positions according to any one of the above. In some embodiments, the Titin-T chain comprises amino acids at positions 1-96 of SEQ ID NO: 32, and/or further comprises amino acid residue substitutions at corresponding positions according to any one of the above; the Obscurin-like-O chain comprises amino acids at positions 4-91 of SEQ ID NO: 34, and/or further comprises amino acid residues at positions according to any one of the above.

Provided is the dimerized polypeptide according to any one of the above, wherein the residue positions in the Titin-T chain are natural sequence numbering positions relative to a sequence SEQ ID NO: 32; the residue positions in the Obscurin-O chain are natural sequence numbering positions relative to a sequence SEQ ID NO: 33; the residue positions in the Obscurin-like-O chain are natural sequence numbering positions relative to a sequence SEQ ID NO: 34.

In some embodiments, provided is the dimerized polypeptide according to any one of the above, wherein the Titin-T chain described above has one or more amino acid residue substitutions selected from the group consisting of 60S and 64T in SEQ ID NO: 32, 68, or 127, and the Obscurin-O chain has one or more amino acid residue substitutions selected from the group consisting of 13S, 32F, 48V, 66C, 82H, and 93C in SEQ ID NO: 33, 80, or 128. The residue positions in the Titin-T chain are natural sequence numbering positions relative to a sequence SEQ ID NO: 32; the residue positions in the Obscurin-O chain are natural sequence numbering positions relative to a sequence SEQ ID NO: 33.

In some embodiments, the variant of SEQ ID NO: 32 comprises an amino acid sequence having at least 70%, 75%, 80%, 85%, 90%, or 95% sequence identity to SEQ ID NO: 32. In some embodiments, the variant of SEQ ID NO: 32 has only the amino acid residue substitutions according to any one of the above as compared to SEQ ID NO: 32. In some embodiments, the variant of SEQ ID NO: 33 comprises an amino acid sequence having at least 70%, 75%, 80%, 85%, 90%, or 95% sequence identity to SEQ ID NO: 33. In some embodiments, the variant of SEQ ID NO: 33 has only the amino acid residue substitutions according to any one of the above as compared to SEQ ID NO: 33. In some embodiments, the variant of SEQ ID NO: 34 comprises an amino acid sequence having at least 70%, 75%, 80%, 85%, 90%, or 95% sequence identity to SEQ ID NO: 34. In some embodiments, the variant of SEQ ID NO: 34 has only the amino acid residue substitutions according to any one of the above as compared to SEQ ID NO: 34. In some embodiments, the present disclosure provides an antigen-binding molecule, which comprises the dimerized polypeptide according to any one of the above.

In some embodiments, provided is the antigen-binding molecule according to any one of the above, which comprises a first antigen-binding moiety, wherein the first antigen-binding moiety comprises a domain-engineered Fab comprising a heavy chain variable region VH1, a light chain variable region VL1, and the dimerized polypeptide, but not comprising a light chain constant region CL and a heavy chain constant region CH1, wherein the VH1 and the VL1 are each linked to any one of the peptide chains of the dimerized polypeptide via a linker. In some embodiments, the C-terminus of the VH1 is fused to the N-terminus of the Titin-T chain via a linker, and the C-terminus of the VL1 is fused to the N-terminus of the Obscurin-O chain or Obscurin-like-O chain via a linker; in some embodiments, the C-terminus of the VL1 is fused to the N-terminus of the Titin-T chain via a linker, and the C-terminus of the VH1 is fused to the N-terminus of the Obscurin-O chain or Obscurin-like-O chain via a linker.

In some embodiments, the present disclosure provides an antigen-binding molecule, which comprises the dimerized polypeptide according to any one of the above that replaces the light chain constant region CL and the heavy chain constant region CH1.

In some embodiments, provided is the antigen-binding molecule according to any one of the above, which comprises a first antigen-binding moiety, wherein the first antigen-binding moiety comprises a domain-engineered Fab comprising a heavy chain variable region VH1, a light chain variable region VL1, and the dimerized polypeptide described above, wherein the VH1 and the VL1 are each linked to any one of the peptide chains of the dimerized polypeptide via a linker. In some embodiments, the C-terminus of the VH1 is fused to the N-terminus of the Titin-T chain via a linker, and the C-terminus of the VL1 is fused to the N-terminus of the Obscurin-O chain or Obscurin-like-O chain via a linker; in some embodiments, the C-terminus of the VL1 is fused to the N-terminus of the Titin-T chain via a linker, and the C-terminus of the VH1 is fused to the N-terminus of the Obscurin-O chain or Obscurin-like-O chain via a linker.

In some embodiments, provided is the antigen-binding molecule according to any one of the above, which comprises a first antigen-binding moiety comprising:
a. a peptide chain of [VH1]-[linker 1]-[Titin-T chain] in order from the N-terminus to the C-terminus, and a peptide chain of [VL1]-[linker 2]-[Obscurin-O chain or Obscurin-like-O chain] in order from the N-terminus to the C-terminus; or
b. a peptide chain of [VH1]-[linker 1]-[Obscurin-O chain or Obscurin-like-O chain] in order from the N-terminus to the C-terminus, and a peptide chain of [VL1]-[linker 2]-[Titin-T chain] in order from the N-terminus to the C-terminus;
wherein the linker 1 and the linker 2 are identical or different. In some embodiments, the peptide linker is a flexible peptide linker. In some embodiments, the peptide linker is 3-15 amino acid residues in length. In some embodiments: A) the linker 1 and the linker 2 are both (GₓS)_{y} linkers, wherein x is selected from the group consisting of integers from 1 to 5 (e.g., 1, 2, 3, 4, or 5) and y is selected from the group consisting of integers from 0 to 6 (e.g., 0, 1, 2, 3, 4, 5, or 6), wherein when y is 0, the linker is a bond, or B) the linker 1 is a C-terminal truncated sequence of CH1, and the linker 2 is a C-terminal truncated sequence of CL. In some other embodiments, wherein: A) the linker 1 has a sequence set forth in SEQ ID NO: 173; the linker 2 has a sequence set forth in SEQ ID NO: 174; or B) the linker 1 and the linker 2 both have sequences set forth in SEQ ID NO: 175; or C) the linker 1 and the linker 2 both have sequences set forth in SEQ ID NO: 176.

In some embodiments, the present disclosure provides an antigen-binding molecule comprising a first antigen-binding moiety and a second antigen-binding moiety, wherein the first antigen-binding moiety is as defined above, the second antigen-binding moiety comprises a heavy chain variable region VH2 and a light chain variable region VL2, and the first antigen-binding moiety and the second antigen-binding moiety bind to different antigens or different epitopes on the same antigen;

In some embodiments, the second antigen-binding moiety comprises a Fab.

In some embodiments, wherein the antigen-binding molecule further comprises an Fc region, wherein the Fc region comprises a first subunit Fc1 and a second subunit Fc2 capable of associating with each other; in some embodiments, the Fc region is an Fc region of an IgG; in some embodiments, the Fc region is an Fc region of IgGi; in some embodiments, the Fc region has one or more amino acid substitutions that reduce homodimerization; and/or the Fc region has one or more amino acid substitutions capable of reducing the binding of the Fc region to an Fc receptor. In some embodiments, the Fc region has a YTE mutation (M252Y, S254T, and T256E), L234A and L235A mutations, and/or an S228P mutation, wherein the mutations are numbered according to the EU index. In some embodiments, the Fc comprises a first subunit and a second subunit capable of associating with each other, wherein the first subunit and/or the second subunit have one or more amino acid substitutions that reduce homodimerization. In some embodiments, the first subunit has a protuberance structure according to the knob-into-hole technique and the second subunit has a pore structure according to the knob-into-hole technique, or the first subunit has a pore structure according to the knob-into-hole technique and the second subunit has a protuberance structure according to the knob-into-hole technique. In some embodiments, the first subunit has one or more amino acid substitutions at positions selected from the group consisting of 354, 356, 358, and 366, and the second subunit has one or more amino acid substitutions at positions selected from the group consisting of 349, 356, 358, 366, 368, and 407; in some embodiments, the second subunit has one or more amino acid substitutions at positions selected from the group consisting of 354, 356, 358, and 366, and the first subunit has one or more amino acid substitutions at positions selected from the group consisting of 349, 356, 358, 366, 368, and 407. In some embodiments, the first subunit has one or more amino acid substitutions selected from the group consisting of 354C, 356E, 358M, and 366W, and the second subunit has one or more amino acid substitutions selected from the group consisting of 349C, 356E, 358M, 366S, 368A, and 407V. In some embodiments, the second subunit has one or more amino acid substitutions selected from the group consisting of 354C, 356E, 358M, and 366W, and the first subunit has one or more amino acid substitutions selected from the group consisting of 349C, 356E, 358M, 366S, 368A, and 407V. In some embodiments, the first subunit comprises amino acid substitutions of 354C, 356E, 358M, and 366W, and the second subunit comprises amino acid substitutions of 349C, 356E, 358M, 366S, 368A, and 407V. In some embodiments, the second subunit comprises amino acid substitutions of 354C, 356E, 358M, and 366W, and the first subunit comprises amino acid substitutions of 349C, 356E, 358M, 366S, 368A, and 407V In some embodiments, the Fc1 has a sequence set forth in SEQ ID NO: 177 and the Fc2 has a sequence set forth in SEQ ID NO: 178; or the Fc2 has a sequence set forth in SEQ ID NO: 177 and the Fc1 has a sequence set forth in SEQ ID NO: 178.

In some embodiments, provided is the antigen-binding molecule according to any one of the above, which comprises a first heavy chain, a first light chain, a second heavy chain, and a second light chain, wherein,
a. the first heavy chain comprises [VH1]-[linker 1]-[Titin-T chain]-[linker 3]-[Fc1] in order from the N-terminus to the C-terminus,
   the first light chain comprises [VL1]-[linker 2]-[Obscurin-0 chain or Obscurin-like-O chain] in order from the N-terminus to the C-terminus,
   the second heavy chain comprises [VH2]-[CH1]-[Fc2] in order from the N-terminus to the C-terminus, and
   the second light chain comprises [VL2]-[CL] in order from the N-terminus to the C-terminus; or
b. the first heavy chain comprises [VH1]-[linker 1]-[Obscurin-O chain or Obscurin-like-O chain]-[linker 3]-[Fc1] in order from the N-terminus to the C-terminus, the first light chain comprises [VL1]-[linker 2]-[Titin-T chain] in order from the N-terminus to the C-terminus,
   the second heavy chain comprises [VH2]-[CH1]-[Fc2] in order from the N-terminus to the C-terminus, and
   the second light chain comprises [VL2]-[CL] in order from the N-terminus to the C-terminus;
   wherein the linker 1, the linker 2, and the linker 3 are identical or different. In some embodiments, the peptide linker is a flexible peptide linker. In some embodiments, the peptide linker is 3-15 amino acid residues in length. In some embodiments, the Fc1 and the Fc2 each independently have one or more amino acid substitutions that reduce homodimerization. In some embodiments, provided are the linker 1, the linker 2, and the linker 3, wherein: A) the linker 1, the linker 2, and the linker 3 are all (GₓS)_{y} linkers, wherein x is selected from the group consisting of integers from 1 to 5 (e.g., 1, 2, 3, 4, or 5), and y is selected from the group consisting of integers from 0 to 6 (e.g., 0, 1, 2, 3, 4, 5, or 6), (wherein: when y is 0, the linker is a bond); or B) the linker 1 is a C-terminal truncated sequence of CH1, the linker 2 is a C-terminal truncated sequence of CL, and the linker 3 is a (GₓS)_{y} linker, wherein x is selected from the group consisting of integers from 1 to 5 (e.g., 1, 2, 3, 4, or 5) and y is selected from the group consisting of integers from 0 to 6 (e.g., 0, 1, 2, 3, 4, 5, or 6). In some embodiments, the linker 3 is a bond, and the linker 1 and the linker 2 are: A) linker 1 having a sequence set forth in SEQ ID NO: 173; and linker 2 having a sequence set forth in SEQ ID NO: 174; or B) linker 1 and linker 2 both having sequences set forth in SEQ ID NO: 175; or C) linker 1 and linker 2 both having sequences set forth in SEQ ID NO: 176. In some embodiments, the Fc1 has a protuberance structure according to the knob-into-hole technique and the Fc2 has a pore structure according to the knob-into-hole technique, or the Fc1 has a pore structure according to the knob-into-hole technique and the Fc2 has a protuberance structure according to the knob-into-hole technique. In some embodiments, the Fc1 has one or more amino acid substitutions at positions selected from the group consisting of 354, 356, 358, and 366, and the Fc2 has one or more amino acid substitutions at positions selected from the group consisting of 349, 356, 358, 366, 368, and 407. In some embodiments, the Fc2 has one or more amino acid substitutions at positions selected from the group consisting of 354, 356, 358, and 366 and the Fc1 has one or more amino acid substitutions at positions selected from the group consisting of 349, 356, 358, 366, 368, and 407. In some embodiments, the Fc1 has one or more amino acid substitutions selected from the group consisting of 354C, 356E, 358M, and 366W, and the Fc2 has one or more amino acid substitutions selected from the group consisting of 349C, 356E, 358M, 366S, 368A, and 407V. In some embodiments, the Fc2 has one or more amino acid substitutions selected from the group consisting of 354C, 356E, 358M, and 366W, and the Fc1 has one or more amino acid substitutions selected from the group consisting of 349C, 356E, 358M, 366S, 368A, and 407V. In some embodiments, the Fc1 comprises amino acid substitutions of 354C, 356E, 358M, and 366W, and the Fc2 comprises amino acid substitutions of 349C, 356E, 358M, 366S, 368A, and 407V In some embodiments, the Fc2 comprises amino acid substitutions of 354C, 356E, 358M, and 366W, and the Fc1 comprises amino acid substitutions of 349C, 356E, 358M, 366S, 368A, and 407V In some embodiments, the Fc1 is set forth in SEQ ID NO: 177 and the Fc2 is set forth in SEQ ID NO: 178; or the Fc2 is set forth in SEQ ID NO: 177 and the Fc1 is set forth in SEQ ID NO: 178.

In some embodiments, the antigen-binding molecule according to any one of the above is a multispecific antibody.

In some embodiments, the antigen-binding molecule according to any one of the above is a bispecific antibody, a trispecific antibody, or a tetraspecific antibody.

In some embodiments, the antigen-binding molecule according to any one of the above is a bispecific antibody.

In some embodiments, the antigen-binding molecule according to any one of the above, wherein:
(I) the antigen-binding molecule is capable of binding to NGF and RANKL; in some embodiments, the antigen-binding molecule comprises a first heavy chain, a first light chain, a second heavy chain, and a second light chain, wherein:
   the first heavy chain comprises [VH1]-[linker 1]-[Obscurin-O chain]-[linker 3]-[Fc1] in order from the N-terminus to the C-terminus,
   the first light chain comprises [VL1]-[linker 2]-[Titin-T chain] in order from the N-terminus to the C-terminus,
   the second heavy chain comprises [VH2]-[CH1]-[Fc2] in order from the N-terminus to the C-terminus, and
   the second light chain comprises [VL2]-[CL] in order from the N-terminus to the C-terminus; wherein the VH1 forms a first antigen-binding moiety binding to NGF with the VL1, and the VH2 forms a second antigen-binding moiety binding to RANKL with the VL2; or
   the VH1 forms a first antigen-binding moiety binding to RANKL with the VL1, and the VH2 forms a second antigen-binding moiety binding to NGF with the VL2;
   in some embodiments, the VH1 has a sequence set forth in SEQ ID NO: 26, the VL1 has a sequence set forth in SEQ ID NO: 27, the VH2 has a sequence set forth in SEQ ID NO: 24, and the VL2 has a sequence set forth in SEQ ID NO: 25; or
   the VH1 has a sequence set forth in SEQ ID NO: 24, the VL1 has a sequence set forth in SEQ ID NO: 25, the VH2 has a sequence set forth in SEQ ID NO: 26, and the VL2 has a sequence set forth in SEQ ID NO: 27;
   and the Obscurin-O chain has a sequence set forth in any one of SEQ ID NOs: 132-141, and the Titin-T chain has a sequence set forth in any one of SEQ ID NOs: 129-131;
   in some embodiments, the Fc1 has a sequence set forth in SEQ ID NO: 177; the Fc2 has a sequence set forth in SEQ ID NO: 178; the CH1 has a sequence set forth in SEQ ID NO: 179; the CL has a sequence set forth in SEQ ID NO: 4; the linker 3 is a bond; the linker 1 and the linker 2 are selected from the group consisting of: a) linker 1 and linker 2 both having sequences set forth in SEQ ID NO: 175; and b) linker 1 having a sequence set forth in SEQ ID NO: 173 and linker 2 having a sequence set forth in SEQ ID NO: 174;
(II) the antigen-binding molecule is capable of binding to PDL1 and CTLA4; in some embodiments, the antigen-binding molecule comprises a first heavy chain, a first light chain, a second heavy chain, and a second light chain, wherein:
   the first heavy chain comprises [VH1]-[linker 1]-[Obscurin-O chain]-[linker 3]-[Fc1] in order from the N-terminus to the C-terminus,
   the first light chain comprises [VL1]-[linker 2]-[Titin-T chain] in order from the N-terminus to the C-terminus,
   the second heavy chain comprises [VH2]-[CH1]-[Fc2] in order from the N-terminus to the C-terminus, and
   the second light chain comprises [VL2]-[CL] in order from the N-terminus to the C-terminus; wherein the VH1 forms a first antigen-binding moiety binding to PDL1 with the VL1, and the VH2 forms a second antigen-binding moiety binding to CTLA4 with the VL2; or
   the VH1 forms a first antigen-binding moiety binding to CTLA4 with the VL1, and the VH2 forms a second antigen-binding moiety binding to PDL1 with the VL2;
   in some embodiments, the VH1 has a sequence set forth in SEQ ID NO: 156, the VL1 has a sequence set forth in SEQ ID NO: 155, the VH2 has a sequence set forth in SEQ ID NO: 169, and the VL2 has a sequence set forth in SEQ ID NO: 170; or
   the VH1 has a sequence set forth in SEQ ID NO: 169, the VL1 has a sequence set forth in SEQ ID NO: 170, the VH2 has a sequence set forth in SEQ ID NO: 156, and the VL2 has a sequence set forth in SEQ ID NO: 155;
   and the Obscurin-O chain has a sequence set forth in any one of SEQ ID NOs: 132-141, and the Titin-T chain has a sequence set forth in any one of SEQ ID NOs: 129-131;
   in some embodiments, the Fc1 has a sequence set forth in SEQ ID NO: 178; the Fc2 has a sequence set forth in SEQ ID NO: 177; the CH1 has a sequence set forth in SEQ ID NO: 179; the CL has a sequence set forth in SEQ ID NO: 4; the linker 3 is a bond; the linker 1 and the linker 2 are selected from the group consisting of: a) linker 1 and linker 2 both having sequences set forth in SEQ ID NO: 175; and b) linker 1 having a sequence set forth in SEQ ID NO: 173 and linker 2 having a sequence set forth in SEQ ID NO: 174; or
(III) the antigen-binding molecule is capable of binding to IL5 and TSLP; in some embodiments, the antigen-binding molecule comprises a first heavy chain, a first light chain, a second heavy chain, and a second light chain, wherein:
   the first heavy chain comprises [VH1]-[linker 1]-[Titin-T chain]-[linker 3]-[Fc1] in order from the N-terminus to the C-terminus;
   the first light chain comprises [VL1]-[linker 2]-[Obscurin-O chain] in order from the N-terminus to the C-terminus;
   the second heavy chain comprises [VH2]-[CH1]-[Fc2] in order from the N-terminus to the C-terminus; and
   the second light chain comprises [VL2]-[CL] in order from the N-terminus to the C-terminus; wherein:
      the VH1 forms a first antigen-binding moiety binding to IL5 with the VL1, and the VH2 forms a second antigen-binding moiety binding to TSLP with the VL2; or
      the VH1 forms a first antigen-binding moiety binding to TSLP with the VL1, and the VH2 forms a second antigen-binding moiety binding to IL5 with the VL2;
      in some embodiments, the VH1 has a sequence set forth in SEQ ID NO: 16, the VL1 has a sequence set forth in SEQ ID NO: 17, the VH2 has a sequence set forth in SEQ ID NO: 171, and the VL2 has a sequence set forth in SEQ ID NO: 172, or
      the VH1 has a sequence set forth in SEQ ID NO: 171, the VL1 has a sequence set forth in SEQ ID NO: 172, the VH2 has a sequence set forth in SEQ ID NO: 16, and the VL2 has a sequence set forth in SEQ ID NO: 17;
      and the Obscurin-O chain has a sequence set forth in any one of SEQ ID NOs: 132-141, and the Titin-T chain has a sequence set forth in any one of SEQ ID NOs: 129-131;
      in some embodiments, the Fc1 has a sequence set forth in SEQ ID NO: 178; the Fc2 has a sequence set forth in SEQ ID NO: 177; the CH1 has a sequence set forth in SEQ ID NO: 179; the CL has a sequence set forth in SEQ ID NO: 4; the linker 3 is a bond; the linker 1 and the linker 2 are selected from the group consisting of: a) linker 1 and linker 2 both having sequences set forth in SEQ ID NO: 175; and b) linker 1 having a sequence set forth in SEQ ID NO: 173 and linker 2 having a sequence set forth in SEQ ID NO: 174.

In some embodiments, provided is the antigen-binding molecule according to any one of the above, which comprises:
a. a first heavy chain comprising [VH1]-[linker 1]-[Titin-T chain]-[linker 3]-[VH2]-[CH1]-[Fc1] in order from the N-terminus to the C-terminus;
   a second heavy chain comprising [VH1]-[linker 1]-[Titin-T chain]-[linker 3]-[VH2]-[CH1]-[Fc2] in order from the N-terminus to the C-terminus;
   a first light chain comprising [VL1]-[linker 2]-[Obscurin-O chain or Obscurin-like-O chain] in order from the N-terminus to the C-terminus; and
   a second light chain comprising [VL2]-[CL] in order from the N-terminus to the C-terminus; or
b. a first heavy chain comprising [VH1]-[linker 1]-[Obscurin-O chain or Obscurin-like-O chain]-[linker 3]-[VH2]-[CH1]-[Fc1] in order from the N-terminus to the C-terminus;
   a second heavy chain comprising [VH1]-[linker 1]-[Obscurin-O chain or Obscurin-like-O chain]-[linker 3]-[VH2]-[CH1]-[Fc2] in order from the N-terminus to the C-terminus,
   a first light chain comprising [VL1]-[linker 2]-[Titin-T chain] in order from the N-terminus to the C-terminus; and
   a second light chain comprising [VL2]-[CL] in order from the N-terminus to the C-terminus;
   wherein the linker 1, the linker 2, and the linker 3 are identical or different; in some embodiments, the linker 1, the linker 2, and the linker 3 are A) or B):

A) the linker 1, the linker 2, and the linker 3 are all (GₓS)_{y} linkers, wherein x is selected from the group consisting of integers from 1 to 5, and y is selected from the group consisting of integers from 0 to 6, and
B) the linker 1 is a C-terminal truncated sequence of CH1, the linker 2 is a C-terminal truncated sequence of CL, the linker 3 is a (GₓS)_{y} linker, wherein x is selected from the group consisting of integers from 1 to 5, and y is selected from the group consisting of integers from 0 to 6.

In some embodiments, the linker 1, the linker 2, and the linker 3 are selected from any one of A) to C):
A) linker 1 having a sequence set forth in SEQ ID NO: 173; linker 2 having a sequence set forth in SEQ ID NO: 174; linker 3 having a sequence set forth in SEQ ID NO: 175 or SEQ ID NO: 176,
B) linker 1, linker 2, and linker 3 all having sequences set forth in SEQ ID NO: 175, and
C) linker 1, linker 2, and linker 3 all having sequences set forth in SEQ ID NO: 176.

In some embodiments, the Fc1 and the Fc2 each independently have one or more amino acid substitutions that reduce homodimerization. In some embodiments, the Fc1 and the Fc2 are identical. In some embodiments, the antigen-binding molecule comprises 1 first heavy chain and 1 second heavy chain, and 2 first light chains and 2 second light chains, wherein the first heavy chain and the second heavy chain have identical amino acid sequences.

In some embodiments, the antigen-binding molecule specifically binds to PDL1 and TIGIT. In some embodiments, the first antigen is PDL1, and the second antigen is TIGIT. In some embodiments, the first antigen is TIGIT, and the second antigen is PDL1. In some embodiments, the antigen-binding molecule comprises a first antigen-binding moiety capable of specifically binding to PDL1 and a second antigen-binding moiety capable of specifically binding to TIGIT, wherein the first antigen-binding moiety comprises a heavy chain variable region VH1 and a light chain variable region VL1, and the second antigen-binding moiety comprises a heavy chain variable region VH2 and a light chain variable region VL2; wherein:
the VH1 comprises HCDR1, HCDR2, and HCDR3 set forth in SEQ ID NOs: 163, SEQ ID NO: 164, and SEQ ID NO: 165, respectively, and the VL1 comprises LCDR1, LCDR2, and LCDR3 set forth in SEQ ID NO: 166, SEQ ID NO: 167, and SEQ ID NO: 168, respectively; and/or
the VH2 comprises HCDR1, HCDR2, and HCDR3 set forth in SEQ ID NO: 157, SEQ ID NO: 158, and SEQ ID NO: 159, respectively, and the VL2 comprises LCDR1, LCDR2, and LCDR3 set forth in SEQ ID NO: 160, SEQ ID NO: 161, and SEQ ID NO: 162, respectively.

In some embodiments, the VH1 has a sequence set forth in SEQ ID NO: 156 or a sequence having at least 90% (e.g., has at least 90%, 91%, 92%, 93%, 94%, 95%, 96%, 97%, 98%, or 99%) sequence identity to SEQ ID NO: 156, and the VL1 has a sequence set forth in SEQ ID NO: 155 or a sequence having at least 90% (e.g., at least 90%, 91%, 92%, 93%, 94%, 95%, 96%, 97%, 98%, or 99%) sequence identity to SEQ ID NO: 155; and/or
the VH2 has a sequence set forth in SEQ ID NO: 154 or a sequence having at least 90% (e.g., at least 90%, 91%, 92%, 93%, 94%, 95%, 96%, 97%, 98%, or 99%) sequence identity to SEQ ID NO: 154, and the VL2 has a sequence set forth in SEQ ID NO: 153 or a sequence having at least 90% (e.g., at least 90%, 91%, 92%, 93%, 94%, 95%, 96%, 97%, 98%, or 99%) sequence identity to SEQ ID NO: 153.

In some embodiments, the antigen-binding molecule comprises:
a heavy chain having a sequence set forth in SEQ ID NO: 148 or a sequence having at least 90% sequence identity to SEQ ID NO: 148; a first light chain having a sequence set forth in SEQ ID NO: 146 or a sequence having at least 90% sequence identity to SEQ ID NO: 146; and a second light chain having a sequence set forth in SEQ ID NO: 147 or a sequence having at least 90% sequence identity to SEQ ID NO: 147; in some embodiments, the antigen-binding molecule has 2 said heavy chains, 2 said first light chains, and 2 said second light chains.

In some embodiments, the present disclosure provides an antigen-binding molecule, which comprises a first antigen-binding moiety capable of specifically binding to PDL1 and a second antigen-binding moiety capable of specifically binding to TIGIT, wherein the first antigen-binding moiety comprises a heavy chain variable region VH1 and a light chain variable region VL1, and the second antigen-binding moiety comprises a heavy chain variable region VH2 and a light chain variable region VL2; wherein:
the VH1 comprises HCDR1, HCDR2, and HCDR3 set forth in SEQ ID NOs: 163, SEQ ID NO: 164, and SEQ ID NO: 165, respectively, and the VL1 comprises LCDR1, LCDR2, and LCDR3 set forth in SEQ ID NO: 166, SEQ ID NO: 167, and SEQ ID NO: 168, respectively; and/or
the VH2 comprises HCDR1, HCDR2, and HCDR3 set forth in SEQ ID NO: 157, SEQ ID NO: 158, and SEQ ID NO: 159, respectively, and the VL2 comprises LCDR1, LCDR2, and LCDR3 set forth in SEQ ID NO: 160, SEQ ID NO: 161, and SEQ ID NO: 162, respectively.

In some embodiments, the VH1 has a sequence set forth in SEQ ID NO: 156 or a sequence having at least 90% (e.g., has at least 90%, 91%, 92%, 93%, 94%, 95%, 96%, 97%, 98%, or 99%) sequence identity to SEQ ID NO: 156, and the VL1 has a sequence set forth in SEQ ID NO: 155 or a sequence having at least 90% (e.g., at least 90%, 91%, 92%, 93%, 94%, 95%, 96%, 97%, 98%, or 99%) sequence identity to SEQ ID NO: 155; and/or
the VH2 has a sequence set forth in SEQ ID NO: 154 or a sequence having at least 90% (e.g., at least 90%, 91%, 92%, 93%, 94%, 95%, 96%, 97%, 98%, or 99%) sequence identity to SEQ ID NO: 154, and the VL2 has a sequence set forth in SEQ ID NO: 153 or a sequence having at least 90% (e.g., at least 90%, 91%, 92%, 93%, 94%, 95%, 96%, 97%, 98%, or 99%) sequence identity to SEQ ID NO: 153.

In some embodiments, the antigen-binding molecule has:
a heavy chain having a sequence set forth in SEQ ID NO: 148 or a sequence having at least 90% sequence identity to SEQ ID NO: 148; a first light chain having a sequence set forth in SEQ ID NO: 146 or a sequence having at least 90% sequence identity to SEQ ID NO: 146; and a second light chain having a sequence set forth in SEQ ID NO: 147 or a sequence having at least 90% sequence identity to SEQ ID NO: 147. In some embodiments, the antigen-binding molecule has 2 heavy chains, 2 first light chains, and 2 second light chains.

In some embodiments, the present disclosure provides a domain-engineered antibody, which is an antibody in which a heavy chain constant region CH1 and a light chain constant region CL are replaced with the dimerized polypeptide according to any one of the above. In some embodiments, provided is the domain-engineered antibody, wherein the heavy chain constant region CH1 is replaced with a Titin-T chain and the light chain constant region CL is replaced with an Obscurin-O chain. In some embodiments, provided is the domain-engineered antibody, wherein the light chain constant region CL is replaced with a Titin-T chain, and the heavy chain constant region CH1 is replaced with an Obscurin-O chain. In some embodiments, provided is the domain-engineered antibody, wherein the heavy chain constant region CH1 is replaced with a Titin-T chain, and the light chain constant region CL is replaced with an Obscurin-like-O chain. In some embodiments, provided is the domain-engineered antibody, wherein the light chain constant region CL is replaced with a Titin-T chain, and the heavy chain constant region CH1 is replaced with an Obscurin-like-O chain.

In some embodiments, the present disclosure provides a pharmaceutical composition, which comprises the antigen-binding molecule or the domain-engineered antibody according to any one of the above, and one or more pharmaceutically acceptable carriers, diluents, or excipients.

In some embodiments, the present disclosure provides use of the dimerized polypeptide according to any one of the above in the reduction of antibody light chain/heavy chain mispairings.

In some embodiments, the present disclosure provides use of the dimerized polypeptide according to any one of the above in the reduction of antibody light chain/heavy chain mispairings during preparation of a bispecific antibody.

In some embodiments, the present disclosure provides use of the dimerized polypeptide according to any one of the above in the reduction of light chain/heavy chain mispairings during preparation of a multispecific antibody.

In some embodiments, the present disclosure provides use of the dimerized polypeptide according to any one of the above in the reduction of light chain/heavy chain mispairings during preparation of a tetraspecific antibody, a trispecific antibody, and a bispecific antibody.

In some embodiments, the present disclosure provides a nucleic acid molecule encoding the dimerized polypeptide, the antigen-binding molecule, or the domain-engineered antibody according to any one of the above.

In some embodiments, the present disclosure provides an expression vector comprising the nucleic acid molecule according to any one of the above.

In some embodiments, the present disclosure further provides a host cell comprising the nucleic acid molecule according to any one of the above.

In some embodiments, provided is the host cell according to any one of the above, which is obtained by transformation (or transduction or transfection) with the vector described above; the host cell is selected from the group consisting of prokaryotic cells and eukaryotic cells, preferably eukaryotic cells, and more preferably mammalian cells. The host cell does not include any animal or plant cells capable of developing into a whole individual, such as human embryonic stem cells, fertilized eggs, or germ cells. In some embodiments, the host cell is a eukaryotic cell, and more preferably a mammalian cell including, but not limited to, CHO, 293, NSO, and a cell in which gene editing can be performed to alter the glycosylation modification of an antibody or an antigen-binding fragment thereof, thereby altering the ADCC function of the antibody or the antigen-binding fragment thereof, e.g., knocking out genes such as FUT8 or GnT-III.

In some embodiments, the present disclosure provides a method for preparing the dimerized polypeptide, the antigen-binding molecule, or the domain-engineered antibody according to any one of the above, which comprises the steps of culturing the host cell described above, and purifying and isolating the dimerized polypeptide, the antigen-binding molecule, or the domain-engineered antibody.

In some embodiments, the present disclosure also provides use of the antigen-binding molecule, the domain-engineered antibody, or the pharmaceutical composition according to any one of the above in the preparation of a medicament for treating or preventing a disease or condition.

In some embodiments, the present disclosure provides a method for treating or preventing a disease or condition, which comprises administering to a subject in need thereof an effective amount of the antigen-binding molecule, the domain-engineered antibody, or the pharmaceutical composition according to any one of the above.

In some embodiments, the present disclosure provides the antigen-binding molecule, the domain-engineered antibody, or the pharmaceutical composition according to any one of the above for use as a medicament; in some embodiments, the medicament is for use in treating or preventing a disease or condition.

In some embodiments, the disease or condition according to any one of the above is a bone-related disease, such as a disease or disorder of osteoporosis, osteopenia or osteoarthritis, rheumatoid arthritis, periodontal disease or multiple myeloma. In some embodiments, the tumor is selected from the group consisting of carcinoma, lymphoma, blastoma, sarcoma, leukemia, and lymphoid malignancies. In some embodiments, the tumor is selected from the group consisting of: squamous cell carcinoma, myeloma, small-cell lung cancer, non-small cell lung cancer (NSCLC), head and neck squamous cell carcinoma (HNSCC), neuroglioma, Hodgkin's lymphoma, non-Hodgkin's lymphoma, diffuse large B-cell lymphoma (DLBCL), follicular lymphoma, acute lymphoblastic leukemia (ALL), acute myeloid leukemia (AML), chronic lymphocytic leukemia (CLL), chronic myeloid leukemia (CML), primary mediastinal large B-cell lymphoma, mantle cell lymphoma (MCL), small lymphocytic lymphoma (SLL), large B-cell lymphoma rich in T-cells/histiocytes, multiple myeloma, myeloid cell leukemia-1 protein (Mcl-1), myelodysplastic syndrome (MDS), gastrointestinal (tract) cancer, kidney cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, gastric cancer, bone cancer, Ewing's sarcoma, cervical cancer, brain cancer, gastric cancer, bladder cancer, hepatoma, breast cancer, colon cancer, hepatocellular carcinoma (HCC), clear cell renal cell carcinoma (RCC), head and neck cancer, laryngeal cancer, hepatobiliary cancer, central nervous system cancer, esophageal cancer, malignant pleural mesothelioma, systemic light chain amyloidosis, lymphoplasmacytic lymphoma, myelodysplastic syndrome, myeloproliferative tumors, neuroendocrine tumors, Merkel cell carcinoma, testicular cancer, and skin cancer. In some embodiments, the inflammatory disease is selected from the group consisting of: rheumatoid arthritis, psoriasis, Crohn's disease, ankylosing spondylitis, multiple sclerosis, type I diabetes, hepatitis (e.g., hepatitis B, hepatitis A, hepatitis C), myocarditis, Sjogren's syndrome, autoimmune hemolytic anemia after transplant rejection, bullus pemphigoid, Graves' disease, Hashimoto's thyroiditis, systemic lupus erythematosus (SLE), myasthenia gravis, pemphigus, and pernicious anemia. In some embodiments, the immune disease may be selected from the group consisting of: rheumatoid arthritis, psoriasis, joint psoriasis, dermatitis, systemic scleroderma and sclerosis, inflammatory bowel disease (IBD), Crohn's disease, ulcerative colitis, respiratory distress syndrome, meningitis, encephalitis, uveitis, glomerulonephritis, eczema, asthma, arteriosclerosis, leukocyte adhesion-deficient disease, multiple sclerosis, Raynaud's syndrome, Sjogren's syndrome, juvenile diabetes, Reiter's disease, Behcet's disease, immune complex nephritis, IgA nephropathy, IgM polyneuropathy, immune-mediated thrombocytopenic symptoms (such as acute idiopathic thrombocytopenic purpura and chronic idiopathic thrombocytopenic purpura), hemolytic anemia, myasthenia gravis, lupus nephritis, systemic lupus erythematosus, rheumatoid arthritis (RA), atopic dermatitis, pemphigus, Graves' disease, Hashimoto's thyroiditis, Wegener's granulomatosis, Omenn's syndrome, chronic renal failure, acute infectious mononucleosis, HIV and herpes virus related diseases, severe acute respiratory syndrome, choreoretinitis and immunological diseases caused by virus infection (such as diseases caused or mediated by B cell infection by Epstein-Barr Virus (EBV)).

### BRIEF DESCRIPTION OF THE DRAWINGS

FIGs. 1A and 1B show diagrams of the interface structure of the interaction between a Titin-T chain and an Obscurin-O chain or an Obscurin-like-O chain to form a dimerized polypeptide complex, wherein FIG. 1A shows a diagram of the interface structure of the interaction between the Titin-T chain and the Obscurin-O chain; FIG. 1B shows a diagram of the interface structure of the interaction between the Titin-T chain and the Obscurin-like-O chain;
FIG. 2 shows a schematic diagram of a Fab in which CH1 and CL are replaced;
FIG. 3 shows a schematic structural diagram of an IgG monospecific antibody in which CH1 and CL on both sides are replaced;
FIG. 4 shows a schematic structural diagram of an IgG bispecific antibody in which CH1 and CL on one side are replaced;
FIG. 5 shows a schematic structural diagram of a DI-1 bispecific antibody;
FIGs. 6A-6C show mass spectrometry spectra of DI-1; wherein FIG. 6A shows mass spectrometry spectra of LC1/LC2, and FIG. 6B shows mass spectrometry spectra of HC1/HC2; FIG. 6C shows mass spectrometry spectra of LC1 + LC2 + HC1 + HC2;
FIG. 7 shows schematic structural diagrams of antibody light/heavy chain cross-mispaired molecules;
FIG. 8 shows a schematic structural diagram of a BU5 bispecific antibody;
FIGs. 9A-9C show mass spectrometry spectra, wherein FIG. 9A shows a mass spectrometry spectrum of B0, FIG. 9B shows a mass spectrometry spectrum of U0, and FIG. 9C shows a mass spectrometry spectrum of BU5;
FIG. 10 shows results for an experiment of osteoclast differentiation of a DI-1 bispecific antibody;
FIG. 11 shows results for an experiment of TF1 cell proliferation of a DI-1 bispecific antibody;
FIG. 12 shows schematic structural diagrams of bispecific antibodies HJ-1, HJ-2, HJ-3, and HJ-4;
FIGs. 13A-13D show mass spectrometry spectra of the HJ bispecific antibodies with four chains co-expressed, wherein FIG. 13A shows a mass spectrometry spectrum of HJ-1 with four chains co-expressed, FIG. 13B shows a mass spectrometry spectrum of HJ-2 with four chains co-expressed, FIG. 13C shows a mass spectrometry spectrum of HJ-3 with four chains co-expressed, and FIG. 13D shows a mass spectrometry spectrum of HJ-4 with four chains co-expressed;
FIGs. 14A-14B show mass spectrometry spectra of the bispecific antibody HJ-1 with three chains of HJ-1-H1, HJ-1-H2, and HJ-1-L2 co-expressed, wherein FIG. 14A shows a mass spectrometry spectrum of a 120000-130000 (amu) deconvolution portion, and
FIG. 14B shows a mass spectrometry spectrum of a 140000-160000 (amu) deconvolution portion;
FIGs. 15A-15B show mass spectrometry spectra of the bispecific antibody HJ-3 with three chain of HJ-3-H1, HJ-3-H2, and HJ-3-L2 co-expressed; wherein, FIG. 15A shows a mass spectrometry spectrum of a 122600-125200 (amu) deconvolution portion, and
FIG. 15B shows a mass spectrometry spectrum of a 120000-148000 (amu) deconvolution portion;
FIGs. 16A-16B: wherein FIG. 16A shows the interaction sites between the residues of Titin-T chain/Obscurin-O chain, and FIG. 16B shows the interaction sites between the residues of Titin-T chain/Obscurin-like-O chain;
FIG. 17 shows a schematic diagram of the bispecific antibody structure in a (FabV)₂-IgG form constructed by the domain-engineered Fab;
FIG. 18 shows the experimental results for the blocking of the binding of PD-L1 to PD-1 and the binding of TIGIT to CD155 by the PDL1-TIGIT bispecific antibodies;
FIG. 19 shows the experimental results for the promotion of IFN-γ secretion by the PDL1-TIGIT bispecific antibodies; and
FIG. 20 shows the experimental results for the inhibition of xenograft tumors in MC38-HL1 mice by the PDL1-TIGIT bispecific antibodies.

### DETAILED DESCRIPTION

To facilitate the understanding of the present disclosure, some technical and scientific terms are described below. Unless otherwise specifically defined herein, all technical and scientific terms used herein have the same meaning as commonly understood by those of ordinary skill in the art.

As used in the description and in the claims, the singular forms of "a", "an", and "the" include plural referents unless otherwise clearly indicated in the context.

Unless otherwise clearly stated in the context, throughout the description and the claims, the words "comprise", "have", "include", and the like, should be construed in an inclusive sense as opposed to an exclusive or exhaustive sense.

The term "and/or" is intended to include two meanings, "and" and "or". For example, the phrase "A, B, and/or C" is intended to encompass each of the following: A, B, and C; A, B, or C; A or C; A or B; B or C; A and C; A and B; B and C; A (alone); B (alone); and C (alone).

The three-letter and single-letter codes for amino acids used herein are as described in J. biol. chem, 243, p3558 (1968).

The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a different structure from amino acids, but function in a manner similar to naturally occurring amino acids.

The term "amino acid mutation" includes amino acid substitutions (also known as amino acid replacements), deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., replacement of one amino acid with another amino acid having different structural and/or chemical properties. Amino acid substitutions include replacement with non-naturally occurring amino acids or with derivatives of the 20 native amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 366W indicates that an amino acid residue at position 366 is W. T366W indicates that an amino acid residue at position 366 is mutated from the original T to W.

The term "antibody" is used in the broadest sense and encompasses a variety of antibody structures, including but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) so long as they exhibit the desired antigen-binding activity. "Natural antibody" refers to a naturally-occurring immunoglobulin molecule. For example, a natural IgG antibody is a heterotetrameric glycoprotein of about 150,000 Daltons composed of two identical light chains and two identical heavy chains linked by a disulfide bond. From the N-terminus to the C-terminus, each heavy chain has one variable region (VH), also known as variable heavy domain or heavy chain variable region, followed by one heavy chain constant region. A heavy chain constant region generally comprises three constant domains (CH1, CH2, and CH3). Similarly, from the N-terminus to the C-terminus, each light chain has one variable region (VL), also known as variable light domain or light chain variable domain, followed by one constant light domain (light chain constant region, CL). The term "full-length antibody", "intact antibody", and "whole antibody" are used herein interchangeably and refer to an antibody having a substantially similar structure to a native antibody structure or having heavy chains in an Fc region as defined herein. In a natural intact antibody, the light chain comprises light chain variable region VL and constant region CL, wherein the VL is positioned at the amino terminus of the light chain, and the light chain constant region comprises a κ chain and a λ chain; the heavy chain comprises a variable region VH and a constant region (CH1, CH2, and CH3), wherein the VH is positioned at the amino terminus of the heavy chain, and the constant region is positioned at the carboxyl terminus, wherein the CH3 is closest to the carboxyl terminus of the polypeptide, and the heavy chain can be of any isotype, including IgG (including subtypes IgG1, IgG2, IgG3, and IgG4), IgA (including subtypes IgA1 and IgA2), IgM, and IgE.

The term "variable region" or "variable domain" of an antibody refers to a domain in an antibody heavy or light chain that is involved in binding of the antibody to an antigen. Herein, the heavy chain variable region (VH) and light chain variable region (VL) of the antibody each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDR regions: HCDR1, HCDR2, and HCDR3; a VL comprises 3 CDR regions: LCDR1, LCDR2, and LCDR3. Each VH and VL is composed of three CDRs and four FRs arranged from the amino terminus (also known as N-terminus) to the carboxyl terminus (also known as C-terminus) in the following order: FR1, CDR1, FR2, CDR2, FR3, CDR3, and FR4.

The amino acid sequence boundaries of the CDRs can be determined by a variety of well-known schemes, for example, the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol. 2018 Oct 16; 9: 2278), and the like. The corresponding relationships among the various numbering schemes are well known to those skilled in the art and are exemplary, as shown in Table 1 below.

**Table 1. Relationships among CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the sequences of the variable regions and CDRs herein.

The term "antibody fragment" refers to a molecule different from an intact antibody, which comprises a moiety of an intact antibody that binds to an antigen to which the intact antibody binds. Examples of antibody fragments include, but are not limited to, Fv, Fab, Fab', Fab'-SH, F(ab')₂, single-domain antibodies, single-chain Fab (scFab), diabodies, linear antibodies, single-chain antibody molecules (e.g., scFv), and multispecific antibodies formed from antibody fragments. In some embodiments, the antigen-binding fragment of the antibody is a monovalent Fab (i.e., Fab), a divalent Fab (F(ab)₂), a trivalent Fab fragment (F(ab)₃), a multivalent Fab (two or more Fabs), or may be a monospecific or multispecific antigen-binding fragment comprising at least one Fab fragment.

The term "antigen-binding molecule" refers to a protein that is capable of specifically binding to an antigen.

The term "Fc region" or "fragment crystallizable region" is used to define the C-terminal region of the heavy chain of an antibody, including native Fc regions and modified Fc regions. In some embodiments, the Fc region comprises two subunits, which may be identical or different. In some embodiments, the Fc region of the human IgG heavy chain is defined as extending from the amino acid residue at position Cys226 or from Pro230 to its carboxyl terminus. Suitable Fc regions used for the antibodies described herein include Fc regions of human IgG1, IgG2 (IgG2A and IgG2B), IgG3, and IgG4. In some embodiments, the boundaries of the Fc region may also be varied, for example, by deleting the C-terminal lysine of the Fc region (residue 447 according to the EU numbering scheme) or deleting the C-terminal glycine and lysine of the Fc region (residues 446 and 447 according to the EU numbering scheme). Unless otherwise stated, the numbering scheme for the Fc region is the EU numbering scheme, also known as the EU index.

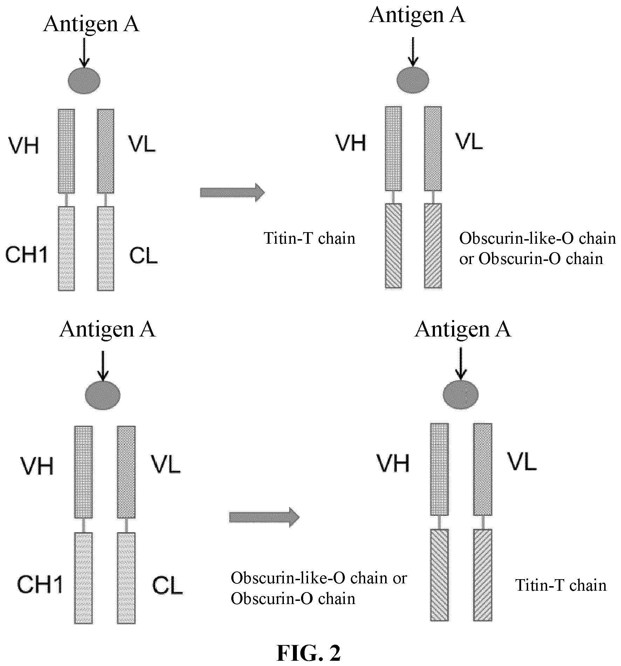

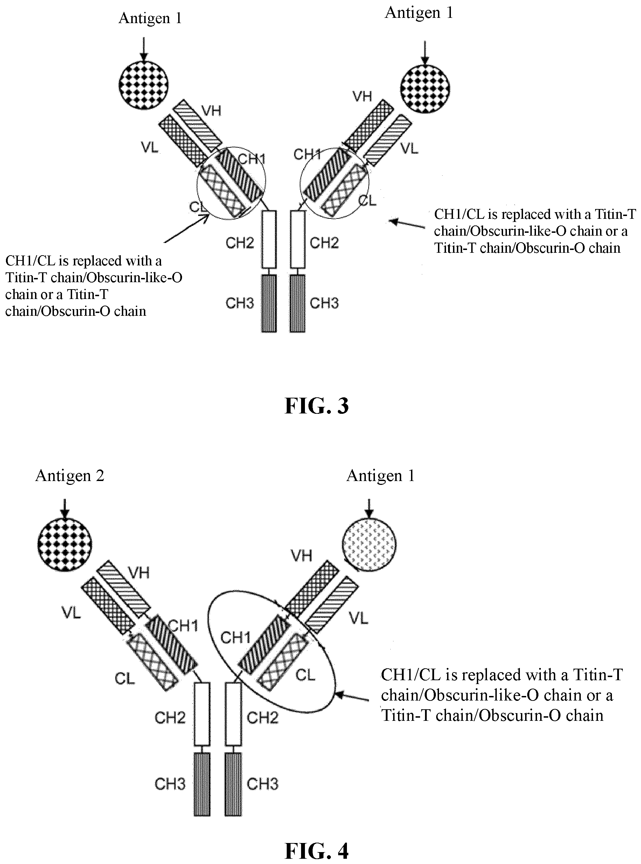

The term "domain-engineered antibody" described herein refers to an antibody in which CH1 and/or CL is replaced with other domains or peptide fragments, for example, CH1/CL is replaced with a Titin-T chain/Obscurin-O chain or with a Titin-T chain/Obscurin-like-O chain. In some embodiments, the domain-engineered antibody is a monospecific antibody or a multispecific antibody; in some embodiments, the domain-engineered antibody is a monovalent antibody, a bivalent antibody, or a multivalent antibody; in some embodiments, the domain-engineered antibody is an intact antibody or an antigen-binding fragment thereof; in some embodiments, a schematic structural diagram of a domain-engineered Fab in which CH1/CL of a Fab is replaced with a Titin-T chain/Obscurin-O chain or a Titin-T chain/Obscurin-like-O chain is shown in FIG. 2; a schematic structural diagram of a domain-engineered antibody in which CH1/CL of a monoclonal antibody is replaced with a Titin-T chain/Obscurin-O chain or a Titin-T chain/Obscurin-like-O chain is shown in FIG. 3; a schematic structural diagram of a domain-engineered antibody in which CH1/CL on one side of a bispecific antibody is replaced with a Titin-T chain/Obscurin-O chain or a Titin-T chain/Obscurin-like-O chain is shown in FIG. 4. Other domain-engineered antibodies in which at least one CH1 and at least one CL are replaced with a Titin-T chain/Obscurin-O chain or a Titin-T chain/Obscurin-like-O chain include, but are not limited to, a bivalent antibody in which CH1 and CL on one side are replaced with a Titin-T chain/Obscurin-O chain or a Titin-T chain/Obscurin-like-O chain, a bispecific antibody in which CH1 and CL on both sides are replaced with a Titin-T chain/Obscurin-O chain or a Titin-T chain/Obscurin-like-O chain, an F(ab)₂ fragment in which 2 CH1/CL are replaced with a Titin-T chain/Obscurin-O chain or a Titin-T chain/Obscurin-like-O chain, or a F(ab)₃ fragment in which 1 or 2 or 3 CH1/CL are replaced with a Titin-T chain/Obscurin-O chain or with a Titin-T chain/Obscurin-like-O chain.

"Titin" is a large sarcomeric protein with a complex molecular folding structure. It is known to have the functions of linking thick myofilaments to a Z-wire, maintaining the integrity and stability of myofibrils, and the like. Titin is the third most abundant protein in skeletal muscle fibers, with a molecular weight of 2700 kDa (more than 25000 amino acids) and a length of 1 µm, which is about half of the sarcomere.

"Titin Ig-like 152 domain" is an Ig-like domain on a Titin protein named as the Titin Ig-like 152 domain, which is capable of binding to an Obscurin Ig-like 1 domain or an Obscurin-like Ig-like 1 domain to form a complex (available from the RCSB PDB database).

"Titin-T chain" or "T chain" refers to a peptide fragment of 78-118 amino acids in length comprising a Titin Ig-like 152 domain in a Titin protein or a functional variant thereof, wherein the Titin-T chain is capable of binding to the Obscurin Ig-like 1 domain or the Obscurin-like Ig-like 1 domain to form a dimerized complex. The functional variant of the T chain is obtained through the mutation on a portion of amino acids of a wild-type T chain, but still has a peptide that binds to the Obscurin Ig-like 1 domain or the Obscurin-like Ig-like 1 domain to form a dimerized complex. In the present disclosure, the Titin-T chain can be used to replace the CH1 or CL domain of an antibody without affecting the binding of the antibody to an antigen. A portion of amino acids of the Titin Ig-like 152 domain may be mutated while still retaining its function of binding to the Obscurin Ig-like 1 domain or the Obscurin-like Ig-like 1 domain to form a complex. For example, amino acids of suitable length are added or truncated at the C-terminus and/or the N-terminus of the Titin Ig-like 152 domain; 1, 2, 3, 4, 5, 6, 7, 8, 9, or 10 amino acid residues may be added or truncated; for example, 5 amino acids "KAGIR" in 5 wild-type Titin proteins which are immediately adjacent to the N-terminus of the Titin Ig-like 152 domain are added to the N-terminus of the Titin Ig-like 152 domain, which still has the function of binding to the Obscurin Ig-like 1 domain or the Obscurin-like Ig-like 1 domain to form a complex. Other mutations can also be made on amino acids of the Titin Ig-like 152 domain, for example, certain amino acids may be mutated to increase inter-chain disulfide bonds, improve complex stability, and the like. In some embodiments, the Titin-T chain is a polypeptide comprising amino acid residues at positions 7-60 of SEQ ID NO: 32 or a mutated sequence thereof. In some embodiments, the Titin-T chain is a polypeptide comprising amino acid residues at positions 1-96 of SEQ ID NO: 32 or a mutated sequence thereof. In some embodiments, the Titin-T chain is a variant of SEQ ID NO: 32 having amino acid residue substitutions at one or more positions selected from the group consisting of positions 60 and 64, for example, having one or more amino acid residue substitutions selected from the group consisting of 60S and 64T. In some embodiments, the Titin-T chain is a variant of SEQ ID NO: 32 having amino acid residue substitutions at one or more positions selected from the group consisting of positions 60 and 64, and further having amino acid residue substitutions at one or more positions selected from the group consisting of positions 3, 8, 11, 13, 20, 22, 25, 26, 39, 40, 42, 45, 47, 49, 56, 58, 66, 70, 75, 77, 79, 81, 82, 83, and 84; for example, the variant further comprises one or more amino acid residue substitutions selected from the group consisting of 3W, 8C, 11I, 13L, 20C, 22M/22C, 25S, 26C, 39T, 40S, 42K, 45S, 47E, 49G, 56S, 58E, 66S/66K, 70R, 75V, 77S, 79T, 81R, 82M, 83D, and 84L. In some embodiments, the Titin-T chain is a polypeptide comprising amino acid residues at positions 7-60 of SEQ ID NO: 32 or a mutated sequence thereof. In some embodiments, the Titin-T chain is a polypeptide comprising amino acid residues at positions 1-96 of SEQ ID NO: 32 or a mutated sequence thereof.

"Obscurin" is a protein encoded by the OBSCN gene and belongs to the giant sarcosine signaling protein family. Obscurin is expressed in cardiac and skeletal muscle and plays an important role in the organization of myofibrils during sarcomere assembly. Obscurin is the major cytoplasmic ligand of sarcoplasmic reticulin sANK1, which can prevent the degradation of sANK1 (Lange S et al., Molecular Biology of the Cell., 23 (13): 2490-504); Obscurin acts as a signaling link between sarcoplasmic reticulum domain and sarcoplasmic reticulum domain (Bagnato P et al., The Journal of Cell Biology., 160 (2): 245-53); Obscurin participates in the formation of new sarcomere during myofibril assembly (Borisov AB, et al., Biochemical and Biophysical Research Communications., 310 (3): 910-918).

"Obscurin Ig-like 1 domain" is an Ig-like domain in an Obscurin protein named as the Obscurin Ig-like 1 domain, which is capable of binding to a Titin Ig-like 152 domain to form a dimerized complex (available from the RCSB PDB database).

"Obscurin-O chain" or "O chain" refers to a peptide fragment of 87-117 amino acids in length comprising an Obscurin Ig-like 1 domain in an Obscurin protein or a functional variant thereof, which is capable of binding to the Titin Ig-like 152 domain to form a dimerized complex. The functional variant of the Obscurin-O chain is obtained through the mutation on a portion of amino acids of a wild-type O chain, but still has a peptide that binds to the Titin Ig-like 152 domain to form a dimerized complex. In the present disclosure, the Obscurin-O chain can replace the CH1 or CL domain of an antibody without affecting the binding of the antibody to an antigen. A portion of amino acids of the Obscurin Ig-like 1 domain can be mutated while still retaining its function of binding to the Titin Ig-like 152 domain to form a complex. For example, amino acids of suitable length are added or truncated at the C-terminus and/or the N-terminus of the Obscurin-O domain, for example, 1, 2, 3, 4, 5, 6, 7, 8, 9, or 10 amino acids are added or truncated; for example, 5 amino acids "DQPQF" in 5 wild-type Obscurin proteins which are immediately adjacent to the N-terminus of the Obscurin Ig-like 1 domain are added to the N-terminus of the Obscurin-O domain, which still has the function of binding to the Titin Ig-like 152 domain to form a dimerized complex. Other mutations can also be made on a portion of amino acids of the Obscurin Ig-like 1 domain, for example, certain amino acids may be mutated to increase inter-chain disulfide bonds, improve antibody stability, and the like. In some embodiments, the Obscurin-O chain is a variant of SEQ ID NO: 33 having amino acid residue substitutions at one or more positions selected from the group consisting of positions 13, 32, 48, 66, 82, and 93, for example, having one or more amino acid residue substitutions selected from the group consisting of 13S, 32F, 48V, 66C, 82H, and 93C; in some embodiments, the Obscurin-O chain is a variant of SEQ ID NO: 33, further having amino acid residue substitutions at one or more positions selected from the group consisting of positions 2, 3, 7, 9, 11, 12, 13, 14, 17, 20, 22, 25, 30, 32, 34, 36, 41, 42, 44, 45, 53, 58, 62, 67, 69, 76, 88, 89, 92, 94, and 97, for example, further having one or more amino acid residue substitutions selected from the group consisting of 2E, 3C, 7K/7R, 9C, 11L, 12S, 13Y, 14T, 17E, 20L, 22M/22S, 25S, 30D, 32P, 34E, 36T, 41K, 42L, 44I, 45T, 53L, 58V, 62E/62K/62H, 67Q/67T, 69S, 76S, 88C, 89L, 92E, 94G, and 97G. In some embodiments, the Obscurin-O chain is a polypeptide comprising amino acids at positions 3-90 of SEQ ID NO: 33 or a mutated sequence thereof.

"Obscurin-like 1" is a protein encoded by the OBSL1 gene located in SPEG of human chromosome 2q35 and is closely related to Obscurin. Alternative splicing of "Obscurin-like 1" produces multiple isoforms with a predicted molecular weight ranging from 130 kD to 230 kD (Geisler SB et al., (2007)., Genomics., 89 (4): 521-531). "Obscurin-like Ig-like 1 domain" is an Ig-like domain in an Obscurin-like 1 protein named as the Obscurin-like Ig-like 1 domain, which is capable of binding to a Titin Ig-like 152 domain to form a complex (available from the RCSB PDB database).

"Obscurin-like-O chain" or "OL chain" refers to a peptide fragment of 78-118 amino acids in length comprising an Obscurin-like Ig-like 1 domain in an Obscurin-like 1 protein or a functional variant thereof. The Obscurin-like-O chain is capable of binding to the Titin Ig-like 152 domain to form a dimerized complex. The functional variant of the Obscurin-like-O chain is obtained through the mutation on a portion of amino acids of a wild-type OL chain, but still has a peptide that binds to the Titin Ig-like 152 domain to form a dimerized polypeptide. In the present disclosure, the Obscurin-like-O chain can replace the CH1 or CL domain of an antibody without affecting the formation of an antigen-binding site by the VH and VL of the antibody and the binding of the antibody to an antigen. A portion of amino acids of the Obscurin-like Ig-like 1 domain can be mutated while still retaining its function of binding to the Titin Ig-like 152 domain to form a dimerized complex. For example, amino acids of a suitable length are added or truncated at the C-terminus and/or the N-terminus of the Obscurin-O domain, for example, 1, 2, 3, 4, 5, 6, 7, 8, 9, or 10 amino acids are added or truncated. Other mutations can also be made on a portion of amino acids of the Obscurin-like Ig-like 1 domain, for example, certain amino acids may be mutated to increase inter-chain disulfide bonds, improve antibody stability, and the like. In some embodiments, the Obscurin-like-O chain is a variant of SEQ ID NO: 34 having amino acid residue substitutions at one or more positions selected from the group consisting of positions 6, 26, 74, 77, 84, and 86, for example, having one or more amino acid residue substitutions selected from the group consisting of 6E, 26S, 74C, 77S, 84C, and 86C. In some embodiments, the Obscurin-like-O chain is a polypeptide comprising amino acid residues at positions 4-91 of SEQ ID NO: 34 or a mutated sequence thereof.

In some embodiments, a portion of amino acids in the Titin Ig-like 152 domain, the Obscurin Ig-like 1 domain, and/or the Obscurin-like Ig-like 1 domain are mutated, which still have the ability to associate the Titin Ig-like 152 domain with the Obscurin Ig-like 1 domain to form a complex, or have the ability to associate the Titin Ig-like 152 domain with the Obscurin-like 1 domain to form a complex. In some embodiments, disclosed is a Titin-T chain/Obscurin-O chain dimerized polypeptide and a Titin-T chain/Obscurin-like-O chain dimerized polypeptide, wherein one or more residues at positions 7-15, 19-24, 26, 55, 59, and 60 in the Titin-T chain are linked to one or more residues at positions 3-6, 9, 41, 73, 75, and 80-90 in the Obscurin-O chain, and one or more residues at positions 1, 7-10, 13-16, 19-26, 59-60, and 96 in the Titin-T chain are linked to one or more residues at positions 4-5, 10, 12-13, 74, 76, 78, and 82-91 in the Obscurin-like-O chain; the residue positions in the Titin-T chain are natural sequence numbering positions relative to a sequence SEQ ID NO: 32; the residue positions in the Obscurin-O chain are natural sequence numbering positions relative to a sequence SEQ ID NO: 33; the residue positions in the Obscurin-like-O chain are natural sequence numbering positions relative to a sequence SEQ ID NO: 34.

"Fab" refers to a protein consisting of VH and CH1 (Fab heavy chain) and VL and CL (Fab light chain) of an immunoglobulin.

"Domain-engineered Fab" or "FabV" refers to a polypeptide fragment of a Fab in which CL and/or CH1 has been replaced with other domains or peptide fragments. In the domain-engineered Fab, VH and VL can still interact to form an antigen-binding site and retain the ability to bind to an antigen. In some embodiments, the domain-engineered Fab may be part of a multivalent antibody (e.g., a divalent antibody or a trivalent antibody). In some other embodiments, the domain-engineered Fab fragment is a single antigen-binding molecule. In some embodiments, the CH1/CL of the Fab is replaced with a Titin-T chain/Obscurin-O chain or a Titin-T chain/Obscurin-like-O chain, and a schematic structural diagram of the domain-engineered Fab is shown in FIG. 2.

The term "specific antibody" or "specifically bound antibody" refers to an antibody capable of specifically binding to a target antigen or epitope. Antibodies are classified into monospecific antibodies, bispecific antibodies, trispecific antibodies, tetraspecific antibodies, ..., multispecific (binding to two or more different target antigens or different epitopes of the same antigen) antibodies according to the number of different target antigens or different epitopes to which the antibodies bind. For example, "bispecific antibody" refers to an antibody that is capable of specifically binding to two different antigens or two different epitopes of the same antigen. Bispecific antibodies of various structures have been disclosed in the prior art; the bispecific antibodies can be classified into IgG-like bispecific antibodies and antibody-fragment-type bispecific antibodies according to the integrity of IgG molecules; the bispecific antibodies can be classified into bivalent, trivalent, tetravalent, ..., multispecific (bivalent or above) antibodies according to the number of the antigen-binding regions; the bispecific antibodies can be classified into symmetric bispecific antibodies and asymmetric bispecific antibodies according to whether their structures are symmetric or asymmetric. Among them, the bispecific antibodies based on antibody fragments, such as Fab fragments lacking Fc fragments, formed by linking 2 or more Fab fragments in one molecule, have low immunogenicity, small molecular weight and high tumor tissue permeability, and typical antibody structures of this type comprise bispecific antibodies, such as F(ab)₂, scFv-Fab and (scFv)₂-Fab; IgG-like bispecific antibodies (e.g., antibodies having Fc fragments) have relatively large molecular weight, and the Fc fragments facilitate later purification of the antibody and increase their solubility and stability, and the Fc fragments may further bind to the receptor FcRn and increase the serum half-life of the antibody, and typical structural models of bispecific antibodies comprise bispecific antibodies such as KiH, CrossMAb, Triomab quadroma, FcΔAdp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP- DART, CODV-Fab-TL, HLE-BiTE, F(ab)₂-CrossMAb, IgG-(scFv)₂, Bs4Ab, DVD-Ig, Tetravalent-DART-Fc, (scFv)₄-Fc, CODV-Ig, mAb2, and F(ab)₄-CrossMAb (see Aran F. Labrijn et al., Nature Reviews Drug Discovery, volume 18, pages 585-608 (2019); Chen S1 et al., J Immunol Res., Feb. 11, 2019: 4516041).

The terms "monovalent", "bivalent", "trivalent", or "multivalent" antibody refer to an antibody in which a specified number of antigen-binding sites are present in the antibody. For example, "monovalent antibody" indicates the presence of one antigen-binding site in an antibody, "divalent antibody" indicates the presence of two antigen-binding sites in an antibody, "trivalent antibody" indicates the presence of three antigen-binding sites in an antibody, and "multivalent antibody" indicates the presence of multiple (e.g., 2 or more) antigen-binding sites in an antibody.

The terms "polypeptide" and "protein" are used interchangeably herein and refer to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term "antigen-binding domain" refers to a region in an antigen-binding molecule (e.g., an antibody) that specifically binds to an antigen, which may be a moiety of a ligand-binding domain that is capable of directly binding to an antigen, or a domain that comprises a variable region of an antibody that is capable of directly binding to an antigen. The term "antigen-binding moiety" refers to a moiety of an antigen-binding molecule (e.g., an antibody) that comprises an antigen-binding domain.

The term "fusion" or "linkage" means that components (e.g., two polypeptides) are linked directly or by a covalent bond via one or more linkers. When the linker is a peptide linker, the covalent bond is a peptide bond.

The term "interaction domain" refers to a domain of a polypeptide that is capable of facilitating the interaction or association of two or more homologous or heterologous polypeptides. For example, interaction domains are dimerization domains that facilitate association with each other to form a dimer. An inter-protein interaction domain is a domain of a polypeptide that facilitates the interaction or association between two or more proteins, for example, an Obscurin-like Ig-like 1 domain in an Obscurin-like protein that is capable of forming a complex with a Titin Ig-like 152 domain of a Titin protein through interactions. In some embodiments, the domain that interacts with the Titin-T chain is an Obscurin-O chain or an Obscurin-like-O chain.

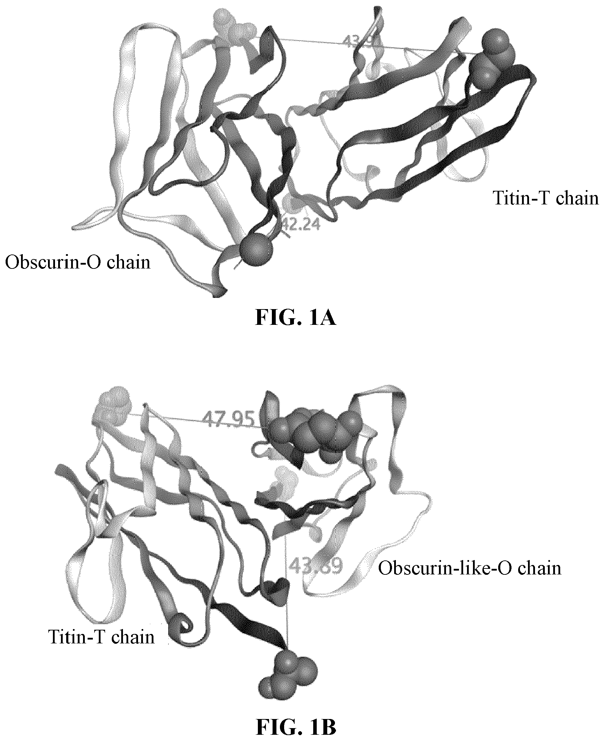

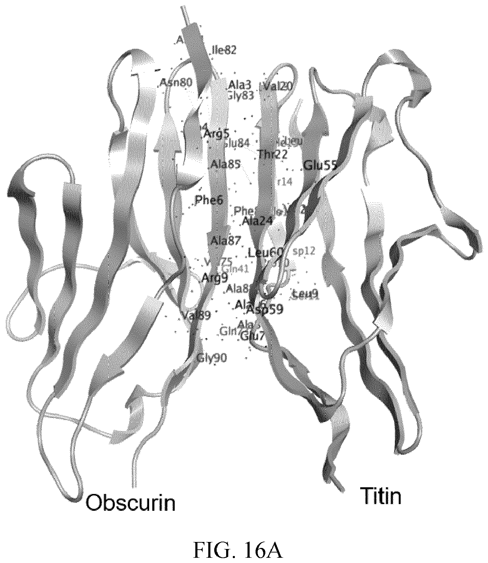

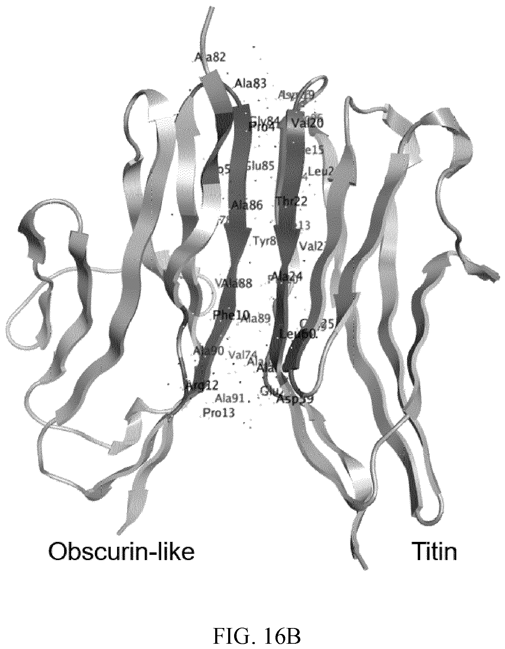

The term "dimerized polypeptide" refers to a dimeric polypeptide (also referred to as a dimerized complex) formed by associating two polypeptides through covalent or non-covalent interactions. A homodimer is a dimer formed from two identical polypeptides, and a heterodimer is a dimer formed from two different polypeptides. The polypeptides may be bound or linked or bonded by any suitable means, for example, through a linker, a disulfide bond, a hydrogen bond, an electrostatic interaction, a salt bridge, a hydrophobic-hydrophilic interaction, or a combination thereof. Illustratively, two polypeptide molecules can form a dimer through natural inter-chain bonds, and can also form a dimer through unnatural inter-chain bonds. In some embodiments of the present disclosure, the dimerized polypeptides Titin-T chain and Obscurin-O chain, or the dimerized polypeptides Titin-T chain and Obscurin-like-O chain, may form a dimer through natural inter-chain bonds. In addition, it is well known to those skilled in the art that, for two domains associated with each other to form a dimer, contact interface residues of the first domain and the second domain that are separated at a distance within 6 angstroms (especially within 4.5 angstroms) play a critical role in maintaining the association of these two domains (Yan, Changhui et al., "Characterization of protein-protein interfaces", The protein journal, vol., 27, 1 (2008): 59-70). In the present disclosure, through analysis of the dimerized complexes formed by associating the Titin-T chain with the Obscurin-O chain and associating the Titin-T chain with the Obscurin-like-O chain by the MOE (molecular operating environment) system (see FIG. 1A and FIG. 1B), it is found that one or more residues at positions 7-15, 19-24, 26, 55, 59, and 60 in the Titin-T chain and one or more residues at positions 3-6, 9, 41, 73, 75, and 80-90 in the Obscurin-O chain interact with each other, and one or more residues at positions 1, 7-10, 13-16, 19-26, 59-60, and 96 in the Titin-T chain and one or more residues at positions 4-5, 10, 12-13, 74, 76, 78, and 82-91 in the Obscurin-like-O chain interact with each other (see FIG. 16A and FIG. 16B for binding sites), wherein the contact interface residues at the above positions of the above dimerized complexes are separated at a distance within 4.5 angstroms, which plays a critical role in maintaining the association of these two domains. In the present disclosure, in some embodiments, the Titin-T chain and the Obscurin-O chain, or the Titin-T chain and the Obscurin-like-O chain, form a dimer through non-natural inter-chain bonds, wherein the dimer comprises 1, 2, 3, 4, 5, 6, 7, 8, 9, 10 or more non-natural inter-chain bonds. The disulfide bond between the Titin-T chain and the Obscurin-O chain or between the Titin-T chain and the Obscurin-like-O chain can be made more stable by mutating some amino acids in the Titin-T chain, the Obscurin-O chain, or the Obscurin-like-O chain, thereby promoting the formation of a stabilized dimer between them. In some embodiments of the present disclosure, the two inter-chain bonds are made more stable by mutating one or more of amino acid residues at positions selected from the group consisting of positions 8, 20, 22, 25, 26, and 39 in the Titin-T chain, and/or at positions selected from the group consisting of positions 3, 9, 25, 66, 76, 88, and 93 in the Obscurin-O chain; or the two inter-chain bonds are made more stable by mutating one or more of amino acid residues at positions selected from the group consisting of positions 8, 20, 22, 25, 26, and 39 in the Titin-T chain, and/or at positions selected from the group consisting of positions 6, 26, 74, 77, 84, and 86 in the Obscurin-like-O chain. In the present disclosure, "mispairing" means that two or more homologous or heterologous polypeptides are interacted or associated to form an undesired dimer or multimer pairing. "Mispairing is less susceptible to occur" means that, for example, when polypeptides A1, B 1, and B2 are co-expressed, it is desirable that an A1-B 1 dimer is produced, and it is not desirable that an A1-B2 dimer is produced, and if the expression amount of the A1-B 1 dimer finally produced is larger than that of the A1-B2 dimer, it is considered that A1 and B 1 are preferentially paired, that is, mispairing is less susceptible to occur between A1 and B2. In the present disclosure, in some embodiments, in the bispecific antibodies comprising the dimerized polypeptides Titin-T chain and Obscurin-O chain or the dimerized polypeptides Titin-T chain and Obscurin-like-O chain of the present disclosure, mispairing is less susceptible to occur between VH1 and VL2, and/or between VL1 and VH2; preferential pairing occurs between VH1 and VL1, and between VH2 and VL2.

"Disulfide bond" refers to a covalent bond formed between sulfur atoms in the structure R-S-S-R'. The amino acid cysteine comprises a thiol group which may form a disulphide bond with a second thiol group, for example with a thiol group of another cysteine residue. Disulfide bonds can be formed between the thiol groups of two cysteine residues located on two polypeptide chains, respectively, thereby forming an inter-chain bridge or an inter-chain bond.

Electrostatic interaction is a non-covalent interaction, plays a critical role in protein folding, stability, flexibility, and function, and includes an ionic interaction, hydrogen bonding, and halogen bonding. The electrostatic interaction may be formed in polypeptides, for example, between Lys and Asp, between Lys and Glu, between Glu and Arg, or between Glu or Trp on a first chain and Arg, Val or Thr on a second chain. Salt bridge is a close-range electrostatic interaction, which is primarily from the anionic carboxylate of Asp or Glu and from the cationic ammonium of Lys or the guanidyl group of Arg, and is a pair of oppositely charged residues in close spatial proximity in the native protein structure. Charged and polar residues in the hydrophobic interface can act as hot spots for binding. Residues having ionizable side chains, such as His, Tyr and Ser, can also participate in the formation of salt bridges.

Hydrophilic interaction means that molecules with polar groups have large affinity to water and can form transient bonding with water through hydrogen bonds. The hydrophobic interaction is a non-covalent interaction between non-polar molecules.

These non-polar molecules (e.g., some neutral amino acid residues, also known as hydrophobic residues) have a tendency to aggregate with each other in an aqueous environment and keep away from water. For example, hydrophobic interactions may be formed between one or more of Val, Tyr and Ala in the first chain and one or more of Val, Leu and Trp in the second chain, or between His and Ala in the first chain and Thr and Phe in the second chain. (See Brinkmann et al., 2017).

The term "hydrogen bond" is formed by electrostatic attraction between two polar groups when a hydrogen atom is covalently bonded to a highly electronegative atom, such as, nitrogen, oxygen or fluorine. Hydrogen bonds can be formed between backbone oxygens (e.g., chalcogen groups) and amide hydrogens (nitrogen groups) of two residues of a polypeptide, for example, a hydrogen bond can be formed between a nitrogen group in Asn and an oxygen group in His, or an oxygen group in Asn and a nitrogen group in Lys. The interactions between hydrogen bonds are stronger than Van der Waals interactions, but weaker than interactions between covalent or ionic bonds, and are critical for maintaining secondary and tertiary structures. For example, an α-helix is formed when the spacing of amino acid residues occurs regularly between positions i and i+4, and a sheet of twisted folds formed by peptide fragments of 3-10 amino acids in length when two peptides are linked through at least two or three backbone hydrogen bonds is a β-sheet.

"Non-natural inter-chain bonds" refer to inter-chain bonds not found in wild-type polypeptide polymers. For example, a non-natural inter-chain bond may be formed between a mutated amino acid residue of one polypeptide and a wild-type amino acid residue or a mutated amino acid residue of another polypeptide. In certain embodiments, at least one non-natural inter-chain bond is a "disulfide bond" formed following an amino acid mutation.

The term "contact interface" refers to a particular region on polypeptides where the polypeptides are in contact with or interact with each other. The contact interface comprises one or more amino acid residues, and when an interaction occurs, amino acid residues of the contact interface on a certain polypeptide are capable of interacting with the corresponding amino acid residues with which they are in contact. The amino acid residues of the contact interface may be in a continuous or discontinuous sequence. For example, when the interface is three-dimensional, amino acid residues within the interface can be separated at different positions on the linear sequence.

The term "linker" refers to a linker unit that links two polypeptide fragments. Linkers generally have some flexibility such that the use of linkers does not result in the loss of the original function of the protein domain. Herein, the linkers present in the same structure may be identical or different. The linker may be a peptide linker comprising one or more amino acids, typically about 1-30, 2-24, or 3-15 amino acids. The linkers used herein may be identical or different. In some embodiments, the linker is selected from a (GₓS)_{y} linker, wherein x is selected from the group consisting of integers from 1 to 5, y is selected from the group consisting of integers from 0 to 6, and when y is 0, the linker is a bond, and the two polypeptide chains are directly linked by the bond; in some embodiments, provided is the (GₓS)_{y} linker, wherein x is an integer from 1 to 5 (e.g., x is 4), and y is an integer selected from 1 to 6 (e.g., 1, 2, 3, 4, 5, or 6); for example, the linker is a "GGGGS" (SEQ ID NO: 175) or "GGGGSGGGGS" (SEQ ID NO: 176) polypeptide. In some other embodiments, the linker is a C-terminal truncated sequence of a heavy chain constant region CH1 (i.e., a peptide sequence formed by truncating a C-terminal portion of CH1 and retaining a N-terminal portion of CH1, wherein for example, the C-terminal truncated sequence of CH1 is a polypeptide formed from amino acid residues at positions 1 to 2, 3, 4, 5, 6, 7, 8, 9, 10, 11, 12, 13, 14, 15, 16, 17, 18, 19, or 20 of the N-terminus of CH1, e.g., the polypeptide "ASTKG" formed from amino acid residues at positions 1 to 5 (SEQ ID NO: 173); or a C-terminal truncated sequence of a light chain constant region CL (i.e., a peptide sequence from by truncating a C-terminal portion of CL and remaining a N-terminal portion of CL, wherein for example, the C-terminal truncated sequence of CL is a polypeptide formed from amino acid residues at positions 1 to 2, 3, 4, 5, 6, 7, 8, 9, 10, 11, 12, 13, 14, 15, 16, 17, 18, 19, or 20 of the N-terminus of CL, e.g., "RTVAS" formed from amino acid residues at positions 1 to 5 (SEQ ID NO: 174). In some embodiments, linker 1 is an "ASTKG" polypeptide and linker 2 is an "RTVAS" polypeptide, or both linker 1 and linker 2 are "GGGGS" polypeptides or "GGGGSGGGGS" polypeptides.

"Tm" is the temperature of dissolution and denaturation (endogenous fluorescence). When the protein is denatured (by heating or by a denaturant), the tertiary structure is opened and the aromatic amino acid microenvironment changes, resulting in a change in the emission fluorescence spectrum. In the present disclosure, Tm1 refers to the temperature at which the fluorescence value changes to half of the maximum value.

"Tonset" is the onset temperature of denaturation. It refers to the temperature at which the protein begins to denature, i.e., the temperature at which the fluorescence value begins to change.

"Tagg" is the onset temperature of aggregation. The temperature at which the sample begins to aggregate is monitored by detecting aggregates at two wavelengths of 266 nm and 473 nm by static light scattering. Tagg 266 refers to the onset temperature of aggregation monitored at 266 nm.

"SEC purity (%)" or "SEC %" refers to the percentage SEC monomer content. SEC % = A monomer/A total × 100% (A monomer represents the peak area of the main peak monomer in the sample, and A total represents the sum of areas of all peaks). In the present disclosure, SEC % of an antibody can be determined by molecular exclusion chromatography (a method for analyzing the separation of a solute by the relative relationship between the pore size of the gel pores and the size of the polymer sample molecule coil.) Instrument for SEC determination is, for example: Agilent 1260; column: waters, XBrige BEH200Å SEC (300 × 7.8 mm, 3.5 µm).

"NR-CE-SDS %" or "NR-CE-SDS purity (%)" refers to the percentage purity by non-reducing capillary electrophoresis. NR-CE-SDS % = A main peak/A total × 100% (A main peak represents the light chain main peak area + the heavy chain main peak area, and A total represents the sum of all peak areas). In the present disclosure, the NR-CE-SDS% of an antibody can be determined by NR-CE capillary gel electrophoresis (a method of electrophoresis in which a gel is transferred into a capillary tube as a supporting medium, and a sample is separated according to its molecular weight under a certain voltage). Instrument for NR-CE-SDS determination is, for example: Beckman model plus 800.

The antibodies of the present disclosure may be antibodies derived from animals (e.g., antibodies derived from murine, fowl, rabbit, camel, and monkey), chimeric antibodies, humanized antibodies and fully human antibodies.

The term "chimeric" antibody refers to an antibody in which a portion of the heavy and/or light chain in the antibody is derived from a particular source or species, while the remainder of the heavy and/or light chain is derived from another different source or species.

The term "humanized" antibody refers to an antibody that retains the reactivity of a non-human antibody while having low immunogenicity in humans. For example, this can be achieved by retaining the non-human CDR regions and replacing the remainder of the antibody with its human counterparts (i.e., the constant regions and the framework region portion of the variable regions).

The terms "human antibody", "human-derived antibody", "fully human antibody", and "complete human antibody" are used interchangeably and refer to antibodies in which the variable and constant regions are human sequences. The term encompasses antibodies that are derived from human genes but have, for example, sequences that have been altered to, e.g., reduce possible immunogenicity, increase affinity, eliminate cysteines that may cause undesired folding, or generate glycosylation sites. The term encompasses antibodies recombinantly produced in non-human cells that may confer glycosylation not characteristic of human cells. The term also encompasses antibodies that have been cultured in transgenic mice comprising some or all of the human immunoglobulin heavy and light chain loci. The meaning of the human antibody specifically excludes humanized antibodies comprising non-human antigen-binding residues.

The term "affinity" refers to the overall strength of a non-covalent interaction between a single binding site of a molecule (e.g., an antibody) and its binding partner (e.g., an antigen). Unless otherwise indicated, as used herein, binding "affinity" refers to an internal binding affinity that reflects a 1:1 interaction between members of a binding pair (e.g., an antibody and an antigen). The affinity of a molecule X for its ligand Y can be generally denoted by the dissociation constant (K_{D}). Affinity can be determined by conventional methods known in the art, including those described herein.

As used herein, the term "k_{assoc}" or "kₐ" refers to the association rate of a particular antibody-antigen interaction, while the term "k_{dis}" or "k_{d}" refers to the dissociation rate of a particular antibody-antigen interaction. The term "K_{D}" refers to the dissociation constant, which is obtained from the ratio of k_{d} to kₐ (i.e., k_{d}/kₐ) and denoted by a molar concentration (M). The K_{D} value of an antibody can be determined using methods well known in the art. For example, surface plasmon resonance is determined using a biosensing system such as a system, or affinity in a solution is determined by solution equilibrium titration (SET).

The term "effector function" refers to biological activities which can be attributed to the Fc region of an antibody (either the native sequence Fc region or the amino acid sequence variant Fc region) and vary with the antibody isotype. Examples of effector functions of an antibody include, but are not limited to: C1q binding and complement-dependent cytotoxicity, Fc receptor binding, antibody-dependent cell-mediated cytotoxicity (ADCC), phagocytosis, down-regulation of cell surface receptors (e.g., B cell receptors), and B cell activation.

The term "monoclonal antibody" refers to a population of substantially homogeneous antibodies, that is, the amino acid sequences of the antibody molecules comprised in the population are identical, except for a small number of natural mutations that may exist. In contrast, polyclonal antibody preparations generally comprise a plurality of different antibodies having different amino acid sequences in their variable domains, which are generally specific for different epitopes. "Monoclonal" refers to the characteristics of an antibody obtained from a substantially homogeneous population of antibodies, and should not be construed as requiring the production of the antibody by any particular method. In some embodiments, the antibody provided by the present disclosure is a monoclonal antibody.

The term "antigen" refers to a molecule or portion of a molecule that is capable of being bound by a selective binding agent, such as an antigen-binding protein (including, for example, an antibody), and that is otherwise capable of being used in an animal to produce an antibody that is capable of binding to the antigen. An antigen may have one or more epitopes capable of interacting with different antigen-binding proteins (e.g., antibodies).

The term "epitope" refers to an area or region on an antigen that is capable of specifically binding to an antibody or an antigen-binding fragment thereof. Epitopes can be formed from contiguous strings of amino acids (linear epitope) or comprise non-contiguous amino acids (conformational epitope), e.g., coming in spatial proximity due to the folding of the antigen (i.e., by the tertiary folding of a proteinaceous antigen). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, including, for example, but not limited to, alanine scanning, peptide blotting, peptide cleavage analysis, epitope excision, epitope extraction, chemical modification of the antigen (Prot. Sci. 9 (2000) 487-496), and cross-blocking.

The term "capable of specifically binding", "specifically binding", or "binding" means that an antibody is capable of binding to a certain antigen or an epitope in the antigen with higher affinity than to other antigens or epitopes. Generally, an antibody binds to an antigen or an epitope in the antigen with an equilibrium dissociation constant (K_{D}) of about 1 × 10⁻⁷ or less (e.g., about 1×10⁻⁸ M or less). In some embodiments, the K_{D} for the binding of an antibody to an antigen is 10% or less (e.g., 1%) of the KD for the binding of the antibody to a non-specific antigen (e.g., BSA or casein). K_{D} may be determined using known methods, for example, by a BIACORE^{®} surface plasmon resonance assay. However, an antibody that specifically binds to an antigen or an epitope within the antigen may have cross-reactivity to other related antigens, e.g., to corresponding antigens from other species (homologous), such as humans or monkeys, e.g., Macaca fascicularis (cynomolgus, cyno), Pan troglodytes (chimpanzee, chimp), or Callithrix jacchus (commonmarmoset, marmoset).

The term "antibody-dependent cellular cytotoxicity", "antibody-dependent cell-mediated cytotoxicity", or "ADCC" is a mechanism for inducing cell death, which depends on the interaction of antibody-coated target cells with effector cells with lytic activity, such as natural killer cells (NK), monocytes, macrophages, and neutrophils, via an Fcy receptor (FcyR) expressed on the effector cells. For example, NK cells express FcyRIIIa, while monocytes express FcyRI, FcγRII, and FcyRIIIa. The ADCC activity of the antibodies provided herein can be assessed by *in vitro* assays, using cells expressing the antigen as target cells and NK cells as effector cells. Cell lysis is detected based on the release of a label (e.g., radioactive substrate, fluorescent dye, or natural intracellular protein) from the lysed cells.

The term "antibody-dependent cellular phagocytosis" ("ADCP") refers to a mechanism by which antibody-coated target cells are eliminated by internalization of phagocytic cells such as macrophages or dendritic cells.

The term "complement-dependent cytotoxicity" or "CDC" refers to a mechanism for inducing cell death in which the Fc effector domain of a target-binding antibody binds to and activates a complement component C1q, and C1q then activates the complement cascade, resulting in the death of the target cell. Activation of a complement may also result in the deposition of complement components on the surface of target cells that promote CDC by binding to complement receptors on leukocytes (e.g., CR3).

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized in a manner similar to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA). "Isolated" nucleic acid refers to a nucleic acid molecule that has been separated from components of its natural environment. The isolated nucleic acid includes a nucleic acid molecule comprised in a cell that generally comprises the nucleic acid molecule, but the nucleic acid molecule is present extrachromosomally or at a chromosomal position different from its natural chromosomal position. An isolated nucleic acid encoding a polypeptide or a fusion protein refers to one or more nucleic acid molecules encoding the polypeptide or fusion protein, including such one or more nucleic acid molecules in a single vector or separate vectors, and such one or more nucleic acid molecules present at one or more positions in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with mixed bases and/or deoxyinosine residues.

The term sequence "identity" refers to the degree (percentage) to which the amino acids/nucleic acids of two sequences are identical at equivalent positions, when the two sequences are optimally aligned, with gaps introduced as necessary to achieve the maximum percent sequence identity, and without considering any conservative substitutions as part of the sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

In the present disclosure, for example, "a position relative to a XX sequence" means refers to a position of a test sequence corresponding to that of a XX sequence, i.e., a relative position of the two sequences, when the test sequence is optimally aligned with the XX sequence to obtain the highest percent identity. For example, in the Titin-T chain, the natural sequence numbering position of SEQ ID NO: 127 relative to the natural sequence numbering position 1 in the sequence SEQ ID NO: 32 is position 6; for another example, in the Obscurin-O chain, the natural sequence numbering position of SEQ ID NO: 128 relative to the natural sequence numbering position 3 in the sequence SEQ ID NO: 33 is position 8.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that can be transformed into a host cell and comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron.

The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progenies derived therefrom, regardless of the number of passages. Progeny may not be exactly the same as parent cells in terms of nucleic acid content and may contain mutations. Mutant progenies that have the same function or biological activity as the cells screened or selected from the group consisting of the initially transformed cells are included herein. Host cells include prokaryotic and eukaryotic host cells, wherein eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichiapastoris*, *Pichia finlandica*, *Pichia trehalophila*, *Pichia koclamae*, *Pichia membranaefaciens*, *Pichia minuta* (*Ogataea minuta*, *Pichia lindneri*), *Pichiaopuntiae*, *Pichia thermotolerans*, *Pichia salictaria*, *Pichia guercuum*, *Pichia pijperi*, *Pichia stiptis*, *Pichia methanolica*, *Pichia*, *Saccharomycescerevisiae*, *Saccharomyces*, *Hansenula polymorpha*, *Kluyveromyces*, *Kluyveromyces lactis*, *Candida albicans*, *Aspergillus nidulans*, *Aspergillus niger*, *Aspergillus oryzae*, *Trichoderma reesei*, *Chrysosporium lucknowense*, *Fusarium sp.*, *Fusarium gramineum*, *Fusarium venenatum*, *Physcomitrella patens*, and *Neurospora crassa. Pichia*, any *Saccharomyces*, *Hansenula polymorpha*, any *Kluyveromyces*, *Candida albicans*, any *Aspergillus*, *Trichoderma reesei*, *Chrysosporium lucknowense*, any *Fusarium*, *Yarrowia lipolytica*, and *Neurospora crassa.*

As used herein, the expressions "cell", "cell line", and "cell culture" are used interchangeably, and all such designations include their progenies. Thus, the words "transformant" and "transformed cell" include the primary subject cell and cultures derived therefrom, regardless of the passage number. It is also understood that not all progeny have precisely identical DNA contents due to deliberate or inadvertent mutations. Variant progeny that have the same function or biological activity as the original transformed cell from which they were selected are included.

"Optional" or "optionally" means that the event or circumstance subsequently described may, but not necessarily, occur, and that the description includes instances where the event or circumstance occurs or does not occur.

The term "pharmaceutical composition" refers to a mixture comprising one or more of the antigen-binding molecules described herein and other chemical components, for example, physiological/pharmaceutically acceptable carriers and excipients.

The term "pharmaceutically acceptable carrier" refers to an ingredient in a pharmaceutical formulation that is different from the active ingredient and is not toxic to the subject. Pharmaceutically acceptable carriers include, but are not limited to, buffers, excipients, stabilizers, or preservatives.

The term "subject" or "individual" includes both human and non-human animals. Non-human animals include all vertebrates (e.g., mammals and non-mammals) such as non-human primates (e.g., cynomolgus monkeys), sheep, dogs, cows, chickens, amphibians, and reptiles. Unless indicated, the terms "patient" and "subject" are used interchangeably herein. As used herein, the term "cynomolgus monkey (cyno)" or "cynomolgus" refers to Macaca fascicularis. In certain embodiments, the individual or subject is a human.

"Administrating" or "giving", when applied to animals, humans, experimental subjects, cells, tissue, organs, or biological fluids, refer to contact of an exogenous drug, a therapeutic agent, a diagnostic agent, or a composition with the animals, humans, subjects, cells, tissue, organs, or biological fluids.

The term "sample" refers to a collection of similar fluids, cells or tissues isolated from a subject, as well as fluids, cells or tissues present within a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura; pericardium; peritoneum; abdominal cavity and other body cavities; fluids collected from bronchial lavage; synovial fluids; liquid solutions in contact with a subject or biological source, e.g., cell and organ culture media (including cell or organ conditioned media); lavage fluids; and the like), tissue biopsy samples, fine needle punctures, surgically excised tissues, organ cultures, or cell cultures.

"Treatment" or "treat" (and grammatical variations thereof) refers to clinical intervention in an attempt to alter the natural course of the treated individual, which may be performed either for prophylaxis or during the course of clinical pathology. Desirable effects of the treatment include, but are not limited to, preventing the occurrence or recurrence of a disease, alleviating symptoms, alleviating/reducing any indirect pathological consequences of the disease, preventing metastasis, decreasing the rate of disease progression, ameliorating or alleviating the disease state, and regressing or improving prognosis. In some embodiments, the antibody of the present disclosure is used to delay the development of or slow the progression of a disease.

"Effective amount" is generally an amount sufficient to reduce the severity and/or frequency of symptoms, eliminate symptoms and/or underlying causes, prevent the appearance of symptoms and/or their underlying causes, and/or ameliorate or improve damage (e.g., lung disease) caused by or associated with a disease state. In some embodiments, the effective amount is a therapeutically effective amount or a prophylactically effective amount. "Therapeutically effective amount" is an amount sufficient to treat a disease state or condition, particularly a state or condition associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health condition of a patient.

### Dimerized Polypeptide and Antigen-Binding Molecule Comprising the Dimerized Polypeptide of the Present Disclosure

In one aspect, in the present disclosure, different dimerized polypeptides are designed. The dimerized polypeptides each comprise a Titin-T chain and an Obscurin-O chain or Obscurin-like-O chain, and can be used to replace CH1/CL of an antibody to alleviate the mispairing between heavy/light chains of a multispecific antibody (e.g., a bispecific antibody) without affecting the binding of the antibody to an antigen.

In some embodiments, the present disclosure provides a dimerized polypeptide, which comprises a Titin-T chain and an Obscurin-O chain or Obscurin-like-O chain.

In some embodiments, the present disclosure provides a dimerized polypeptide, wherein the Titin-T chain described above has one or more amino acid residue replacements at positions selected from the group consisting of positions 8, 20, 22, 25, 26, and 39, and/or the Obscurin-O chain has one or more amino acid residue mutations at positions selected from the group consisting of positions 3, 9, 25, 76, and 88; or the Titin-T chain has one or more amino acid residue mutations at positions selected from the group consisting of positions 8, 20, 22, 25, 26, and 39, and/or the Obscurin-like-O chain has one or more amino acid residue mutations at positions selected from the group consisting of positions 6, 26, 74, 77, 84, and 86; the mutation positions in the Titin-T chain are natural sequence numbering positions relative to a sequence SEQ ID NO: 32; the mutation positions in the Obscurin-O chain are natural sequence numbering positions relative to a sequence SEQ ID NO: 33; the mutation positions in the Obscurin-like-O chain are natural sequence numbering positions relative to a sequence SEQ ID NO: 34.

In some specific embodiments, the Titin-T chain described above has one or more amino acid residue substitutions selected from the group consisting of 8C, 20C, 22C, 25S, 26C, and 39T, and/or the Obscurin-O chain has one or more amino acid residue substitutions selected from the group consisting of 3C, 9C, 25S, 76S, and 88C; or the Titin-T chain has one or more amino acid residue substitutions selected from the group consisting of 8C, 20C, 22C, 25S, 26C, and 39T, and/or the Obscurin-like-O chain has one or more amino acid residue mutations selected from the group consisting of 6E, 26S, 74C, 77S, 84C, and 86C. In some embodiments, the Titin-T chain and the Obscurin-O chain, or the Titin-T chain and the Obscurin-like-O chain, have the following amino acid residue substitutions: substitutions of 25S, 39T, and 8C for the Titin-T chain, and a substitution of 88C for the Obscurin-O chain; substitutions of 25S, 39T, and 20C for the Titin-T chain, and a substitution of 3C for the Obscurin-O chain; substitutions of 25S, 39T, and 26C for the Titin-T chain, and a substitution of 9C for the Obscurin-O chain; substitutions of 25S, 39T, and 8C for the Titin-T chain, and substitutions of 25S, 76S, and 88C for the Obscurin-O chain; substitutions of 25S, 39T, and 20C for the Titin-T chain, and substitutions of 25S, 76S, and 3C for the Obscurin-O chain; substitutions of 25S, 39T, and 26C for the Titin-T chain, and substitutions of 25S, 76S, and 9C for the Obscurin-O chain; substitutions of 25S, 39T, and 8C for the Titin-T chain, and substitutions of 6E and 74C for the Obscurin-like-O chain; substitutions of 25S, 39T, and 20C for the Titin-T chain, and substitutions of 6E and 84C for the Obscurin-like-O chain; substitutions of 25S, 39T, and 22C for the Titin-T chain, and substitutions of 6E and 86C for the Obscurin-like-O chain; substitutions of 25S, 39T, and 8C for the Titin-T chain, and substitutions of 6E, 26S, 77S, and 74C for the Obscurin-like-O chain; substitutions of 25S, 39T, and 20C for the Titin-T chain, and substitutions of 6E, 26S, 77S, and 84C for the Obscurin-like-O chain; or substitutions of 25S, 39T, and 22C for the Titin-T chain, and substitutions of 6E, 26S, 77S, and 86C for the Obscurin-like-O chain. In some embodiments, the Titin-T has the position substitutions described above on the basis of SEQ ID NO: 32 or 127; the Obscurin-O chain has the position substitutions described above on the basis of SEQ ID NO: 33 or 128; the Obscurin-like-O chain has the position substitutions described above on the basis of SEQ ID NO: 34.

In some embodiments, provided is the dimerized polypeptide described above, wherein the Obscurin-O chain described above has one or more amino acid residue mutations at positions selected from the group consisting of positions 7, 11, and 62. In some embodiments, the Obscurin-O chain has one or more amino acid residue substitutions selected from the group consisting of 7R or 7K, 62K or 62H, and 11L; in some embodiments, the Titin-T chain has substitutions of 25S, 39T, and 8C, and the Obscurin-O chain has substitutions of 25S, 76S, 88C, 7K, and 62K; the Titin-T chain has substitutions of 25S, 39T and 8C, and the Obscurin-O chain has substitutions of 25S, 76S, 88C, 7K, and 62H; the Titin-T chain has substitutions of 25S, 39T, and 8C, and the Obscurin-O chain has substitutions of 25S, 76S, 88C, 11L, and 62K; or the Titin-T chain has substitutions of 25S, 39T, and 8C, and the Obscurin-O chain has substitutions of 25S, 76S, 88C, 11L, and 62H. In some embodiments, the Obscurin-O chain has the position substitutions described above on the basis of SEQ ID NO: 33 or 45. The positions for the substitutions in the Titin-T chain are natural sequence numbering positions relative to a sequence SEQ ID NO: 32; the positions for the substitutions in the Obscurin-O chain are natural sequence numbering positions relative to a sequence SEQ ID NO: 33.

In some embodiments, provided is the dimerized polypeptide described above, wherein the Titin-T chain described above has one or more amino acid mutations at positions selected from he group consisting of positions 3, 11, 13, 22, 40, 42, 45, 47, 49, 56, 58, 66, 70, 75, 77, 79, 81, 82, 83, and 84, and/or the Obscurin-O chain has one or more amino acid mutations selected from the group consisting of positions 2, 11, 12, 13, 14, 17, 20, 22, 30, 32, 34, 36, 41, 42, 44, 45, 53, 58, 62, 67, 69, 89, 92, 94, and 97. The mutation positions in the Titin-T chain are natural sequence numbering positions relative to a sequence SEQ ID NO: 35; the mutation positions in the Obscurin-O chain are natural sequence numbering positions relative to a sequence SEQ ID NO: 50. In some embodiments, the Titin-T chain has one or more amino acid substitutions selected from the group consisting of 3W, 11I, 13L, 22M, 40S, 42K, 45S, 47E, 49G, 56S, 58E, 66S or 66K, 70R, 75V, 77S, 79T, 81R, 82M, 83D, and 84L, and/or the Obscurin-O chain has one or more amino acid substitutions selected from the group consisting of 2E, 11K, 12S, 13Y, 14T, 17E, 20L, 22M or 22S, 30D, 32P, 34E, 36T, 41K, 42L, 44I, 45T, 53L, 58V, 62E, 67Q or 67T, 69S, 89L, 92E, 94G, and 97G. In some embodiments, the Titin-T chain has amino acid substitutions of 66S and 77S, and/or the Obscurin-O chain has amino acid substitutions of 11K, 12S, 13Y, 14T, and 22S; the Titin-T chain has amino acid substitutions of 66K, 70R, 79T, and 81R, and/or the Obscurin-O chain has amino acid substitutions of 2E, 17E, 30D, 32P, 34E, 36T, 44I, 45T, 58V, 62E, 67Q, 69S, and 97G; the Titin-T chain has amino acid substitutions of 3W, 11I, 13L, 22M, and 82M, and/or the Obscurin-O chain has amino acid substitutions of 20L, 22M, and 53L; the Titin-T chain has amino acid substitutions of 11I, 66K, 79T, and 81R, and/or the Obscurin-O chain has amino acid substitutions of 41K, 45T, 67Q, 69S, and 89L; the Titin-T chain has amino acid substitutions of 40S, 42K, 45S, 47E, 49G, 56S, 58E, 75V, 83D, and 84L, and/or the Obscurin-O chain has amino acid substitutions of 42L, 45T, 67T, 69S, 92E, and 94G; the Titin-T chain has amino acid substitutions of 47E, 49G, 56S, 58E, and 75V, and/or the Obscurin-O chain has amino acid substitutions of 42L, 45T, 67T, 69S, 92E, and 94G; the Titin-T chain has amino acid substitutions of 56S, 58E, and 75V, and/or the Obscurin-O chain has amino acid substitutions of 42L, 45T, 67T, 69S, 92E, and 94G; or the Titin-T chain has amino acid substitutions of 56S, 58E, 66S, and 77S, and/or the Obscurin-O chain has amino acid substitutions of 12S, 13Y, 22S, 42L, 45T, 67Q, 69S, 92E, and 94G; the positions for the substitutions in the Titin-T chain are natural sequence numbering positions relative to a sequence SEQ ID NO: 35; the positions for the substitutions in the Obscurin-O chain are natural sequence numbering positions relative to a sequence SEQ ID NO: 50. In some embodiments, the Titin-T chain has the amino acid substitutions on the basis of SEQ ID NO: 35, and the Obscurin-O chain has the amino acid substitutions on the basis of SEQ ID NO: 50.

In some embodiments, provided is the dimerized polypeptide described above, wherein the Titin-T chain further comprises one or more amino acid residue mutations at positions selected from the group consisting of positions 60 and 64, and/or the Obscurin-O chain comprises one or more amino acid residue mutations at positions selected from the group consisting of positions 13, 32, 48, 66, 82, and 93; the amino acid residue positions in the Titin-T chain are natural sequence numbering positions relative to a sequence SEQ ID NO: 32; the amino acid residue positions in the Obscurin-O chain are natural sequence numbering positions relative to a sequence SEQ ID NO: 33. In some embodiments, provided is the dimerized polypeptide described above, wherein the Titin-T chain has one or more (e.g., 1 or 2) amino acid residue substitutions selected from the group consisting of 60S and 64T, and/or the Obscurin-O chain has one or more (e.g., 1, 2, 3, 4, 5, 6, or more) amino acid residue substitutions selected from the group consisting of 13S, 32F, 48V, 66C, 82H, and 93C; the amino acid residue positions in the Titin-T chain are natural sequence numbering positions relative to a sequence SEQ ID NO: 32; the amino acid residue positions in the Obscurin-O chain are natural sequence numbering positions relative to a sequence SEQ ID NO: 33. In some embodiments, provided is the dimerized polypeptide described above, wherein the Titin-T chain has amino acid residue substitutions at positions 60S and 64T, and/or the Obscurin-O chain has amino acid residue substitutions selected from any one of a)-c): a) amino acid residue substitutions at positions 13S and 48V, b) amino acid residue substitutions at positions 13S, 32F, 48V, and 82H, and c) amino acid residue substitutions at positions 13S, 32F, 48V, 66C, 82H, and 93C; the amino acid residue positions in the Titin-T chain are natural sequence numbering positions relative to a sequence SEQ ID NO: 32; the amino acid residue positions in the Obscurin-O chain are natural sequence numbering positions relative to a sequence SEQ ID NO: 33. In some embodiments, provided is the dimerized polypeptide described above, wherein the Titin-T chain described above has one or more amino acid residue substitutions selected from the group consisting of 60S and 64T in SEQ ID NO: 32, 68, or 127, and the Obscurin-O chain has one or more amino acid residue substitutions selected from the group consisting of 13S, 32F, 48V, 66C, 82H, and 93C in SEQ ID NO: 33, 80, or 128. The amino acid residue positions in the Titin-T chain are natural sequence numbering positions relative to a sequence SEQ ID NO: 32; the amino acid residue positions in the Obscurin-O chain are natural sequence numbering positions relative to a sequence SEQ ID NO: 33.

In some embodiments, provided is the dimerized polypeptide described above, wherein one or more residues at positions selected from the group consisting of positions 7-15, 19-24, 26, 55, 59, and 60 in the Titin-T chain bind to one or more residues at positions selected from the group consisting of positions 3-6, 9, 41, 73, 75, and 80-90 in the Obscurin-O chain to form a dimerized complex, or one or more residues at positions selected from the group consisting of positions 1, 7-10, 13-16, 19-26, 59-60, and 96 in the Titin-T chain bind to one or more residues at positions selected from the group consisting of positions 4-5, 10, 12-13, 74, 76, 78, and 82-91 in the Obscurin-like-O chain to form a dimerized complex; the residue positions in the Titin-T chain are natural sequence numbering positions relative to a sequence SEQ ID NO: 32; the residue positions in the Obscurin-O chain are natural sequence numbering positions relative to a sequence SEQ ID NO: 33; the residue positions in the Obscurin-like-O chain are natural sequence numbering positions relative to a sequence SEQ ID NO: 34. In some embodiments, the Titin-T chain comprises amino acids at positions 7-60 of SEQ ID NO: 32 or a mutant thereof, and the Obscurin-O chain comprises amino acids at positions 3-90 of SEQ ID NO: 33 or a mutant thereof; or the Titin-T chain comprises amino acids at positions 1-96 of SEQ ID NO: 32 or a mutant thereof, and the Obscurin-like-O chain comprises amino acids at positions 4-91 of SEQ ID NO: 34 or a mutant thereof.

In some embodiments, provided is the dimerized polypeptide, which comprises a Titin-T chain and an Obscurin-O chain, or a Titin-T chain and an Obscurin-like-O chain, wherein: i) the Titin-T chain is a variant of SEQ ID NO: 32, and the variant has amino acid residue substitutions at one or more positions selected from the group consisting of positions 60 and 64 as compared to SEQ ID NO: 32, and/or ii) the Obscurin-O chain is a variant of SEQ ID NO: 33, and the variant is has amino acid residue substitutions at one or more positions selected from the group consisting of positions 13, 32, 48, 66, 82, and 93 as compared to SEQ ID NO: 33; and: a) when the variant does not have an amino acid residue substitution at position 13, 48, 66, 82, or 93, and has an amino acid residue substitution at position 32, the amino acid substitution at position 32 is not 32P; b) when the variant does not have an amino acid residue substitution at position 32, 48, 66, 82, or 93, and has an amino acid residue substitution at position 13, the amino acid substitution at position 13 is not 13Y; and c) when the variant does not have an amino acid residue substitution at position 48, 66, 82, or 93, and has amino acid residue substitutions at positions 13 and 32, the amino acid residue substitution at position 13 is not 13Y, and the amino acid residue substitution at position 32 is not 32P.

In some embodiments, provided is the dimerized polypeptide, wherein the variant of SEQ ID NO: 32 has one or more amino acid residue substitutions selected from the group consisting of 60S and 64T as compared to SEQ ID NO: 32, and/or the variant of SEQ ID NO: 33 has one or more amino acid residue substitutions selected from the group consisting of 13S, 32F, 48V, 66C, 82H, and 93C as compared to SEQ ID NO: 33. In some embodiments, the variant of SEQ ID NO: 32 has amino acid substitutions of 60S and 64T as compared to SEQ ID NO: 32, and/or the variant of SEQ ID NO: 33 has amino acid residue substitutions selected from any one of a) to c) as compared to SEQ ID NO: 32: a) 32F and 48V; b) 13S, 32F, 48V, and 82H; and c) 13S, 32F, 48V, 66C, 82H, and 93C.

In some embodiments, provided is the dimerized polypeptide, wherein the variant of SEQ ID NO: 32 further has amino acid residue substitutions at one or more positions selected from the group consisting of positions 3, 8, 11, 13, 20, 22, 25, 26, 39, 40, 42, 45, 47, 49, 56, 58, 66, 70, 75, 77, 79, 81, 82, 83, and 84 as compared to SEQ ID NO: 32; in some embodiments, the variant of SEQ ID NO: 32 further has one or more amino acid residue substitutions selected from the group consisting of 3W, 8C, 11I, 13L, 20C, 22M/22C, 25S, 26C, 39T, 40S, 42K, 45S, 47E, 49G, 56S, 58E, 66S/66K, 70R, 75V, 77S, 79T, 81R, 82M, 83D, and 84L as compared to SEQ ID NO: 32; in some embodiments, the variant of SEQ ID NO: 32 further comprises amino acid residue substitutions selected from any one of a) to l) as compared to SEQ ID NO: 32: a) 8C, 25S, and 39T; b) 20C, 25S, and 39T; c) 25S, 26C, and 39T; d) 22C, 25S, and 39T; e) 8C, 25S, 39T, 66S, and 77S; f) 8C, 25S, 39T, 66K, 70R, 79T, and 81R; g) 3W, 8C, 11I, 13L, 22M, 25S, 39T, and 82M; h) 8C, 11I, 25S, 39T, 66K, 79T, and 81R; i) 8C, 25S, 39T, 40S, 42K, 45S, 47E, 49G, 56S, 58E, 75V, 83D, and 84L, j) 8C, 25S, 39T, 47E, 49G, 56S, 58E, and 75V; k) 8C, 25S, 39T, 56S, 58E, and 75V; and l) 8C, 25S, 39T, 56S, 58E, 66S, and 77S; in some embodiments, the variant of SEQ ID NO: 32 has amino acid residue substitutions selected from any one of A) to C) as compared to SEQ ID NO: 32: A) 8C, 11I, 25S, 39T, 60S, 64T, 66K, 79T, and 81R; B) 8C, 11I, 20C, 25S, 39T, 60S, 64T, 66K, 79T, and 81R; and C) 8C, 11I, 25S, 26C, 39T, 60S, 64T, 66K, 79T, and 81R.

In some embodiments, provided is the dimerized polypeptide, wherein the variant of SEQ ID NO: 33 further has amino acid residue substitutions at one or more positions selected from the group consisting of positions 2, 3, 7, 9, 11, 12, 13, 14, 17, 20, 22, 25, 30, 32, 34, 36, 41, 42, 44, 45, 53, 58, 62, 67, 69, 76, 88, 89, 92, 94, and 97 as compared to SEQ ID NO: 33. In some embodiments, the variant of SEQ ID NO: 33 further has one or more amino acid residue substitutions selected from the group consisting of 2E, 3C, 7K/7R, 9C, 11L, 12S, 13Y, 14T, 17E, 20L, 22M/22S, 25S, 30D, 32P, 34E, 36T, 41K, 42L, 44I, 45T, 53L, 58V, 62E/62K/62H, 67Q/67T, 69S, 76S, 88C, 89L, 92E, 94G, and 97G as compared to SEQ ID NO: 33. In some embodiments, the variant of SEQ ID NO: 33 further has amino acid residue substitutions selected from any one of A)-R) as compared to SEQ ID NO: 33: A) 88C; B) 3C; C) 9C; D) 25S, 76S, and 88C; E) 25S, 76S, and 3C; F) 25S, 76S, and 9C; G) 7K, 25S, 62K, 76S, and 88C; H) 7K, 25S, 62H, 76S, and 88C; I) 7R, 25S, 62K, 76S, and 88C; J) 7R, 25S, 62H, 76S, and 88C; K) 11L, 25S, 62K, 76S, and 88C; L) 11L, 25S, 62H, 76S, and 88C; M) 12S, 13Y, 14T, 22S, 25S, 62K, 76S, and 88C; N) 2E, 11L, 17E, 25S, 30D, 32P, 34E, 36T, 44I, 45T, 58V, 62E, 67Q, 69S, 76S, 88C, and 97G; O) 11L, 20L, 22M, 25S, 53L, 62K, 76S, and 88C; P) 11L, 25S, 41K, 45T, 62K, 67Q, 69S, 76S, 88C, and 89L; Q) 11L, 25S, 42L, 45T, 62K, 67T, 69S, 76S, 88C, 92E, and 94G; and R) 11L, 12S, 13Y, 22S, 25S, 42L, 45T, 62K, 67Q, 69S, 76S, 88C, 92E, and 94G. In some embodiments, the variant of SEQ ID NO: 33 has amino acid residue substitutions selected from any one of a) to j) as compared to SEQ ID NO: 33: a) 25S, 32F, 41K, 45T, 48V, 62K, 67Q, 69S, 76S, 88C, and 89L; b) 13S, 25S, 32F, 41K, 45T, 48V, 62K, 67Q, 69S, 76S, 82H, 88C, and 89L; c) 3C, 13S, 25S, 32F, 41K, 45T, 48V, 62K, 67Q, 69S, 76S, 82H, 88C, and 89L; d) 9C, 13S, 25S, 32F, 41K, 45T, 48V, 62K, 67Q, 69S, 76S, 82H, 88C, and 89L; e) 13S, 32F, 41K, 45T, 48V, 62K, 67Q, 69S, 82H, 88C, and 89L; f) 3C, 13S, 32F, 41K, 45T, 48V, 62K, 67Q, 69S, 82H, 88C, and 89L, g) 9C, 13S, 32F, 41K, 45T, 48V, 62K, 67Q, 69S, 82H, 88C, and 89L; h) 13S, 25S, 32F, 41K, 45T, 48V, 62K, 66C, 67Q, 69S, 76S, 82H, 88C, 89L, and 93C; i) 3C, 13S, 25S, 32F, 41K, 45T, 48V, 62K, 66C, 67Q, 69S, 76S, 82H, 88C, 89L, and 93C; and j) 9C, 13S, 25S, 32F, 41K, 45T, 48V, 62K, 66C, 67Q, 69S, 76S, 82H, 88C, 89L, and 93C.

In some embodiments, provided is the dimerized polypeptide, wherein the Obscurin-like-O chain is SEQ ID NO: 34 or a variant thereof, wherein the variant of SEQ ID NO: 34 has amino acid residue substitutions at one or more positions selected from the group consisting of positions 6, 26, 74, 77, 84, and 86 as compared to SEQ ID NO: 34. In some embodiments, the variant of SEQ ID NO: 34 has one or more amino acid residue substitutions selected from the group consisting of 6E, 26S, 74C, 77S, 84C, and 86C as compared to SEQ ID NO: 34. In some embodiments, the variant of SEQ ID NO: 34 has amino acid residue substitutions selected from any one of A) to F) as compared to SEQ ID NO: 34: A) 6E and 74C, B) 6E and 84C, C) 6E and 86C, D) 6E, 26S, 77S, and 74C, E) 6E, 26S, 77S, and 84C, F) 6E, 26S, 77S, and 86C.

In some embodiments, provided is the dimerized polypeptide, wherein the Titin-T chain is a variant of SEQ ID NO: 32, 68, or 127, wherein the variant of SEQ ID NO: 32 has one or more amino acid residue substitutions selected from the group consisting of 60S and 64T as compared to SEQ ID NO: 32; the variant of SEQ ID NO: 68 has one or more amino acid residue substitutions selected from the group consisting of 60S and 64T as compared to SEQ ID NO: 68; the variant of SEQ ID NO: 127 has one or more amino acid residue substitutions selected from the group consisting of 60S and 64T as compared to SEQ ID NO: 127; the Obscurin-O chain is a variant of SEQ ID NO: 33, 80, or 128, wherein the variant of SEQ ID NO: 33 has one or more amino acid residue substitutions selected from the group consisting of 13S, 32F, 48V, 66C, 82H, and 93C as compared to SEQ ID NO: 33; the variant of SEQ ID NO: 80 has one or more amino acid residue substitutions selected from the group consisting of 13S, 32F, 48V, 66C, 82H, and 93C as compared to SEQ ID NO: 80; the variant of SEQ ID NO: 128 has one or more amino acid residue substitutions selected from the group consisting of 13S, 32F, 48V, 66C, 82H, and 93C as compared to SEQ ID NO: 128.

In some embodiments, provided is the antigen-binding molecule according to any one of the above, which comprises a first heavy chain, a first light chain, a second heavy chain, and a second light chain, wherein,
a. the first heavy chain comprises [VH1]-[linker 1]-[Titin-T chain]-[Fc1] in order from the N-terminus to the C-terminus,
the first light chain comprises [VL1]-[linker 2]-[Obscurin-O chain] in order from the N-terminus to the C-terminus,
the second heavy chain comprises [VH2]-[CH1]-[Fc2] in order from the N-terminus to the C-terminus, and
the second light chain comprises [VL2]-[CL] in order from the N-terminus to the C-terminus; or
b. the first heavy chain comprises [VH1]-[linker 1]-[Obscurin-O chain]-[Fc1] in order from the N-terminus to the C-terminus,
the first light chain comprises [VL1]-[linker 2]-[Titin-T chain] in order from the N-terminus to the C-terminus,
the second heavy chain comprises [VH2]-[CH1]-[Fc2] in order from the N-terminus to the C-terminus, and
the second light chain comprises [VL2]-[CL] in order from the N-terminus to the C-terminus;
wherein the linker 1 and the linker 2 are identical or different; in some embodiments, the Fc1 and the Fc2 each independently have one or more amino acid substitutions that reduce homodimerization; in some embodiments, provided are the linker 1 and the linker 2, wherein: A) the linker 1 and the linker 2 are both (GₓS)_{y} linkers, wherein x is selected from the group consisting of integers from 1 to 5 (e.g., 1, 2, 3, 4, or 5) and y is selected from the group consisting of integers from 0 to 6 (e.g., 0, 1, 2, 3, 4, 5, or 6) (wherein: when y is 0, the linker is a bond); or B) the linker 1 is a C-terminal truncated sequence of CH1, and the linker 2 is a C-terminal truncated sequence of CL; in some embodiments, the linker 1 and the linker 2 are selected from any one of A) to C): A) linker 1 having a sequence set forth in SEQ ID NO: 173; and linker 2 having a sequence set forth in SEQ ID NO: 174; B) linker 1 and linker 2 both having sequences set forth in SEQ ID NO: 175; and C) linker 1 and linker 2 both having sequences set forth in SEQ ID NO: 176. In some embodiments, the Fc1 has a protuberance structure according to the knob-into-hole technique and the Fc2 has a pore structure according to the knob-into-hole technique, or the Fc1 has a pore structure according to the knob-into-hole technique and the Fc2 has a protuberance structure according to the knob-into-hole technique. In some embodiments, the Fc1 has one or more amino acid substitutions at positions selected from the group consisting of 354, 356, 358, and 366, and the Fc2 has one or more amino acid substitutions at positions selected from the group consisting of 349, 356, 358, 366, 368, and 407. In some embodiments, the Fc2 has one or more amino acid substitutions at positions selected from the group consisting of 354, 356, 358, and 366 and the Fc1 has one or more amino acid substitutions at positions selected from the group consisting of 349, 356, 358, 366, 368, and 407. In some embodiments, the Fc1 has one or more amino acid substitutions selected from the group consisting of 354C, 356E, 358M, and 366W, and the Fc2 has one or more amino acid substitutions selected from the group consisting of 349C, 356E, 358M, 366S, 368A, and 407V. In some embodiments, the Fc2 has one or more amino acid substitutions selected from the group consisting of 354C, 356E, 358M, and 366W, and the Fc1 has one or more amino acid substitutions selected from the group consisting of 349C, 356E, 358M, 366S, 368A, and 407V. In some embodiments, the Fc1 comprises amino acid substitutions of 354C, 356E, 358M, and 366W, and the Fc2 comprises amino acid substitutions of 349C, 356E, 358M, 366S, 368A, and 407V. In some embodiments, the Fc2 comprises amino acid substitutions of 354C, 356E, 358M, and 366W, and the Fc1 comprises amino acid substitutions of 349C, 356E, 358M, 366S, 368A, and 407V In some embodiments, the Fc1 is set forth in SEQ ID NO: 177 and the Fc2 is set forth in SEQ ID NO: 178; or the Fc2 is set forth in SEQ ID NO: 177 and the Fc1 is set forth in SEQ ID NO: 178.

In some embodiments, provided is the antigen-binding molecule according to any one of the above, which comprises a first heavy chain, a first light chain, a second heavy chain, and a second light chain, wherein,
a. the first heavy chain comprises [VH1]-[linker 1]-[Titin-T chain]-[Fc1] in order from the N-terminus to the C-terminus,
the first light chain comprises [VL1]-[linker 2]-[Obscurin-like-0 chain] in order from the N-terminus to the C-terminus,
the second heavy chain comprises [VH2]-[CH1]-[Fc2] in order from the N-terminus to the C-terminus, and
the second light chain comprises [VL2]-[CL] in order from the N-terminus to the C-terminus; or
b. the first heavy chain comprises [VH1]-[linker 1]-[Obscurin-like-O chain]-[Fc1] in order from the N-terminus to the C-terminus,
the first light chain comprises [VL1]-[linker 2]-[Titin-T chain] in order from the N-terminus to the C-terminus,
the second heavy chain comprises [VH2]-[CH1]-[Fc2] in order from the N-terminus to the C-terminus, and
the second light chain comprises [VL2]-[CL] in order from the N-terminus to the C-terminus;
wherein the linker 1 and the linker 2 are identical or different; in some embodiments, the Fc1 and the Fc2 each independently have one or more amino acid substitutions that reduce homodimerization; in some embodiments, provided are the linker 1 and the linker 2, wherein: A) the linker 1 and the linker 2 are both (GₓS)_{y} linkers, wherein x is selected from the group consisting of integers from 1 to 5 (e.g., 1, 2, 3, 4, or 5) and y is selected from the group consisting of integers from 0 to 6 (e.g., 0, 1, 2, 3, 4, 5, or 6) (wherein: when y is 0, the linker is a bond); or B) the linker 1 is a C-terminal truncated sequence of CH1, and the linker 2 is a C-terminal truncated sequence of CL; in some embodiments, the linker 1 and the linker 2 are selected from any one of A) to C): A) linker 1 having a sequence set forth in SEQ ID NO: 173; and linker 2 having a sequence set forth in SEQ ID NO: 174; B) linker 1 and linker 2 both having sequences set forth in SEQ ID NO: 175; and C) linker 1 and linker 2 both having sequences set forth in SEQ ID NO: 176. In some embodiments, the Fc1 has a protuberance structure according to the knob-into-hole technique and the Fc2 has a pore structure according to the knob-into-hole technique, or the Fc1 has a pore structure according to the knob-into-hole technique and the Fc2 has a protuberance structure according to the knob-into-hole technique. In some embodiments, the Fc1 has one or more amino acid substitutions at positions selected from the group consisting of 354, 356, 358, and 366, and the Fc2 has one or more amino acid substitutions at positions selected from the group consisting of 349, 356, 358, 366, 368, and 407. In some embodiments, the Fc2 has one or more amino acid substitutions at positions selected from the group consisting of 354, 356, 358, and 366 and the Fc1 has one or more amino acid substitutions at positions selected from the group consisting of 349, 356, 358, 366, 368, and 407. In some embodiments, the Fc1 has one or more amino acid substitutions selected from the group consisting of 354C, 356E, 358M, and 366W, and the Fc2 has one or more amino acid substitutions selected from the group consisting of 349C, 356E, 358M, 366S, 368A, and 407V. In some embodiments, the Fc2 has one or more amino acid substitutions selected from the group consisting of 354C, 356E, 358M, and 366W, and the Fc1 has one or more amino acid substitutions selected from the group consisting of 349C, 356E, 358M, 366S, 368A, and 407V. In some embodiments, the Fc1 comprises amino acid substitutions of 354C, 356E, 358M, and 366W, and the Fc2 comprises amino acid substitutions of 349C, 356E, 358M, 366S, 368A, and 407V In some embodiments, the Fc2 comprises amino acid substitutions of 354C, 356E, 358M, and 366W, and the Fc1 comprises amino acid substitutions of 349C, 356E, 358M, 366S, 368A, and 407V In some embodiments, the Fc1 is set forth in SEQ ID NO: 177 and the Fc2 is set forth in SEQ ID NO: 178; or the Fc2 is set forth in SEQ ID NO: 177 and the Fc1 is set forth in SEQ ID NO: 178.

### Modification of Fc Region

In one aspect, the Fc region of the present disclosure comprises one or more amino acid substitutions that reduce its binding to an Fc receptor, e.g., its binding to the Fcy receptor, and reduce or eliminate effector function. A native IgG Fc region, specifically an IgG1 Fc region or an IgG4 Fc region, may cause the fusion protein of the present disclosure to target cells expressing an Fc receptor, rather than cells expressing an antigen. In some embodiments, the engineered Fc region of the present disclosure exhibits reduced binding affinity for an Fc receptor and/or reduced effector functions. In some embodiments, the engineered Fc region shows above 50%, 80%, 90%, or 95% reduction in binding affinity for an Fc receptor as compared to the native Fc region. In some embodiments, the Fc receptor is an Fcy receptor. In some embodiments, the Fc receptor is a human Fcγ receptor, e.g., FcyRI, FcyRIIa, FcyRIIB, or FcγRIIIa. In some embodiments, the engineered Fc region also has reduced binding affinity for a complement (e.g., C1q) as compared to the native Fc region. In some embodiments, the engineered Fc region has no reduced binding affinity for neonatal Fc receptor (FcRn) as compared to the native Fc region. In some embodiments, the engineered Fc region has reduced effector functions, which may include, but are not limited to, one or more of the following: reduced complement-dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen presenting cells, reduced binding to NK cells, reduced binding to macrophages, reduced binding to monocytes, reduced binding to polymorphonuclear cells, reduced direct signaling-induced apoptosis, reduced dendritic cell maturation, and reduced T cell priming. For the IgG1 Fc region, amino acid residue substitutions at positions such as positions 238, 265, 269, 270, 297, 327, and 329 can reduce effector functions. In some embodiments, the Fc region is a human IgG1 Fc region, and the amino acid residues at positions 234 and 235 are A, as numbered according to the EU index. For the IgG4 Fc region, amino acid residue substitutions at positions such as position 228 can reduce effector functions.

The antigen-binding molecule may comprise different antigen-binding domains fused to the two subunits of the Fc region and thus may result in undesired homodimerization. To improve yield and purity, it would be advantageous to introduce modifications that promote heterodimerization in the Fc region of the antigen-binding molecule of the present disclosure. In some embodiments, the Fc region of the present disclosure comprises modifications according to the knob-and-hole (KIH) technique, which involves introducing a protuberance structure (knob) at the interface of the first subunit and a pore structure (hole) at the interface of the second subunit; or a protuberance structure (hole) at the interface of the first subunit and a hole structure (knob) at the interface of the second subunit. As such, the protuberance structure can be positioned in the pore structure, thereby promoting the formation of heterodimers and inhibiting the production of homodimers. The protuberance structure is constructed by substituting a small amino acid side chain at the interface of the first subunit with a larger side chain (e.g., tyrosine or tryptophan). The pore structure is created at the interface of the second subunit by substituting a large amino acid side chain with a smaller amino acid side chain (e.g., alanine or threonine). The protuberance and pore structures are prepared by altering the nucleic acid encoding the polypeptide. Illustratively, optional amino acid substitutions are shown in Table 2 below:

**Table 2. Combination of KIH mutations**

| | | | | | | | | |
|---|---|---|---|---|---|---|---|---|
| First subunit | T366Y | T366W | T394W | F405W | T366W | T366Y | T366W | F405W |
| | | | | | | F405A | F405W | Y407A |
| Second subunit | Y407T | Y407A | F405A | T394S | T366S | T394W | T394W | T366W |
| | | | | | L358A | | | |
| | | | | | | Y407T | Y407A | T394S |
| | | | | | Y407V | | | |

In addition to the knob-into-hole technique, other techniques for modifying the CH3 domain of the heavy chain of multispecific antibodies to achieve heterodimerization are also known in the art, e.g., WO 96/27011, WO 98/050431, EP 1870459, WO 2007/110205, WO 007/147901, WO 2009/089004, WO 2010/129304, WO 2011/90754, WO 2011/143545, WO 2012/058768, WO 2013/157954, and WO 013/096291.

The C-terminus of the Fc region may be an intact C-terminus ending with amino acid residues PGK or a shortened C-terminus, e.g., a shortened C-terminus in which one or two C-terminal amino acid residues have been removed. In a preferred aspect, the C-terminus of the heavy chain is a shortened C-terminus ending with PG. Thus, in some embodiments, a composition of intact antibodies may comprise antibody populations with all K447 residues and/or G446 + K447 residues removed. In some embodiments, a composition of intact antibodies may comprise antibody populations without a K447 residue and/or G446 + K447 residues removed. In some embodiments, a composition of intact antibodies comprises antibody populations having a mixture of antibodies with and without a K447 residue and/or G446 + K447 residues.

### Recombination Method

The antigen-binding molecule or polypeptide may be produced using recombination methods. For these methods, one or more isolated nucleic acids encoding the polypeptide or antigen-binding molecule are provided.

In one embodiment, the present disclosure provides an isolated nucleic acid encoding the polypeptide or antigen-binding molecule described above. Such nucleic acids may each independently encode any one of the polypeptide chains described above. In another aspect, the present disclosure provides one or more vectors (e.g., expression vectors) comprising such nucleic acids. In another aspect, the present disclosure provides a host cell comprising such nucleic acids. In one embodiment, provided is a method for preparing a polypeptide or an antigen-binding molecule, which comprises culturing a host cell comprising a nucleic acid encoding the polypeptide or antigen-binding molecule, as provided above, under conditions suitable for expression, and optionally isolating the antibody from the host cell (or a host cell medium).

For recombinant production of the polypeptide or antigen-binding molecule, the nucleic acid encoding the protein is isolated and inserted into one or more vectors for further cloning and/or expression in a host cell. Such nucleic acids can be readily isolated and sequenced using conventional procedures, or produced by recombination methods or obtained by chemical synthesis.

Suitable host cells for cloning or expressing a vector encoding the polypeptide or antigen-binding protein include the prokaryotic or eukaryotic cells described herein. For example, the polypeptide or antigen-binding molecule can be produced in bacteria, particularly when glycosylation and Fc effector functions are not required. After expression, the polypeptide or antigen-binding molecule can be isolated from the bacterial cell paste in a soluble fraction and can be further purified.

In addition to prokaryotes, eukaryotic microorganisms such as filamentous fungi or yeast are suitable cloning or expression hosts for vectors encoding antigen-binding molecules, including fungal and yeast strains. Suitable host cells for expression of the antigen-binding molecule may also be derived from multicellular organisms (invertebrates and vertebrates); examples of invertebrate cells include plant and insect cells. A number of baculovirus strains have been identified, which can be used in combination with insect cells, particularly for transfection of *Spodoptera frugiperda* cells; plant cell cultures may also be used as hosts, see, e.g., US 5959177, US 6040498, US 6420548, US 7125978, and US 6417429; vertebrate cells can also be used as hosts, e.g., mammalian cell lines adapted for growth in a suspension. Other examples of suitable mammalian host cell lines include a SV40-transformed monkey kidney CV1 line (COS-7); a human embryonic kidney line (293 or 293T cells); baby hamster kidney cells (BHK); mouse sertoli cells (TM4 cells); monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRC5 cells; and FS4 cells. Other suitable mammalian host cell lines include Chinese hamster ovary (CHO) cells, including DHFR-CHO cells; and myeloma cell lines such as Y0, NS0, and Sp2/0. For reviews of certain mammalian host cell lines suitable for antibody production see, e.g., Yazaki, P. and Wu, A.M., Methods in Molecular Biology, Vol. 248, Lo, B.K.C. (eds.), Humana Press, Totowa, NJ (2004), pp. 255-268.

### Assay

The polypeptide or antigen-binding molecule provided herein can be identified, screened, or characterized for their physical/chemical characteristics and/or biological activities by a variety of assays known in the art. In one aspect, the polypeptide or antigen-binding molecule of the present disclosure is tested for activity, e.g., by known methods such as ELISA, western blot, and the like.

### Treatment method and route of administration

Any of the antigen-binding molecules provided herein can be used in the treatment method. In yet another aspect, the present disclosure provides use of an antigen-binding molecule in the manufacture or preparation of a medicament. In some embodiments, in one such embodiment, the use further comprises administering to a subject an effective amount of at least one additional therapeutic agent (e.g., one, two, three, four, five, or six additional therapeutic agents). The "subject" according to any of the above embodiments may be a human.

In yet another aspect, provided is a pharmaceutical composition comprising the antigen-binding molecule, e.g., for any of the above pharmaceutical uses or treatment methods. In one embodiment, the pharmaceutical composition comprises any of the antigen-binding molecules provided herein and a pharmaceutically acceptable carrier. In another embodiment, the pharmaceutical composition further comprises at least one additional therapeutic agent.

The antigen-binding molecule of the present disclosure may be used alone or in combination with other agents for the treatment. For example, the antibody of the present disclosure can be co-administered with at least one additional therapeutic agent.

The antigen-binding molecule of the present disclosure (and any additional therapeutic agents) may be administered by any suitable means, including parenteral, intrapulmonary, and intranasal administration, and if required by local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection, depending in part on whether the administration is short-term or long-term. Various administration schedules are contemplated herein, including but not limited to, single administration or multiple administrations at multiple time points, bolus administration, and pulse infusion.

The antigen-binding molecule of the present disclosure will be formulated, administered, and applied in a manner consistent with Good Medical Practice. Factors considered in this context include the specific condition being treated, the specific mammal being treated, the clinical state of the individual patient, the cause of the condition, the site of delivery of the agent, the method of administration, the timing of administration, and other factors known to medical practitioners. The polypeptide or fusion protein may be formulated with or without one or more agents currently used to prevent or treat the condition. The effective amount of such additional agents depends on the amount present in the pharmaceutical composition, the type of condition or treatment, and other factors. These are generally used in the same dosages and routes of administration as described herein, or in about 1% to 99% of the dosages described herein, or in other dosages, and by any route empirically/clinically determined to be appropriate.

For the prevention or treatment of a disease, the appropriate dosage of the antigen-binding molecule of the present disclosure (when used alone or in combination with one or more additional therapeutic agents) will depend on the type of disease to be treated, the type of therapeutic molecule, the severity and course of the disease, purpose of administration (prophylactic or therapeutic), previous treatment, clinical history and response to the therapeutic molecule of the patient, and the discretion of the attending physician. The therapeutic molecule is suitably administered to a patient in one or a series of treatments.

### Article of Manufacture

In another aspect of the present disclosure, provided is an article of manufacture, which comprises materials useful for the treatment, prevention, and/or diagnosis of the above condition. The article of manufacture comprises a container and a label or package insert on or associated with the container. Suitable containers include, for example, bottles, vials, syringes, IV solution bags, and the like. The container may be formed from a variety of materials such as glass or plastic. The container contains a composition effective in the treatment, prevention, and/or diagnosis of a disease, either alone or in combination with another composition, and may have a sterile access port (e.g., the container may be an intravenous solution bag or vial with a stopper pierceable by a hypodermic injection needle). At least one active agent in the composition is the antigen-binding molecule of the present disclosure. The label or package insert indicates that the composition is used to treat the selected condition. Further, the article of manufacture may comprise: (a) a first container containing a composition, wherein the composition comprises the antigen-binding molecule of the present disclosure; and (b) a second container containing a composition, wherein the composition comprises other cytotoxic agents or additional therapeutic agents. The article of manufacture in this embodiment of the present disclosure may further comprise a package insert indicating that the composition may be used to treat a particular condition. Alternatively or additionally, the article of manufacture may further comprise a second (or third) container containing a pharmaceutically acceptable buffer. From a commercial and user standpoint, it may further contain other materials as required, including other buffers, diluents, filters, needles, and syringes.

Although the antibodies used in the examples target specific antigens, those skilled in the art, in light of the teachings of the present disclosure, can understand that the technical effect is achieved independently of the specific CDR sequences and independently of the specific antigen sequences, but with the benefit of substitution of the Titin T chain/Obscurin-O chain, or the Titin T chain/Obscurin-like-O chain for CH1/CL to improve the mispairing between the heavy/light chains.

### Examples and Test Examples

The present disclosure is further described below with reference to the following examples and test examples, which, however, do not limit the present disclosure. The experimental methods in the examples and test examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated are commercially available conventional reagents.

### Example 1: Preparation Method for Antibody or Polypeptide Protein

The method comprises: designing primers for constructing gene fragments (e.g., antibody VH/VK gene fragments) by PCR, subjecting the gene fragments to homologous recombination with an expression vector (such as pHr (with a signal peptide and a constant region gene (such as CH1-Fc/CL) fragment)) to construct an expression vector (such as VH-CH1-Fc-pHr/VK-CL-pHr), introducing the constructed expression vector into a prokaryote or eukaryote for expression, and finally purifying a product to obtain the desired antibodies or polypeptides protein. Without limitation, the antibody constant regions may be selected from the group consisting of light chain constant regions of human κ and λ chains and heavy chain constant regions of IgG1, IgG2, IgG3, and IgG4. Non-limiting examples further include the optimization design for human antibody constant regions, such as mutations at positions L234A/L235A or L234F/L235E of the heavy chain constant region. Illustratively, antibody light/heavy chain constant region sequences are as follows:
> IgG1 heavy chain constant region (abbreviated as hIgG1): 






 (SEQ ID NO: 1).
 Note: In the sequence, the single underlined part indicates CH1, the dotted underlined part indicates CH2, and the italic part denotes CH3.
> knob-IgG1 heavy chain constant region (abbreviated as knob-IgG1): 
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPEAAGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKA
LPAPIEKTISKAKGQPREPQVYTLPPCREEMTKNQVSLWCLVKGFYPSDIAVEWES
NGQPENNYKTTPPVLDSDGSFFLYSKLTVDKSRWQQGNVFSCSVMHEALHNHYTQK
SLSLSPGK (SEQ ID NO:2).
 Note: In the sequence, the single underlined part indicates CH1, the dotted underlined part indicates CH2, and the italic part denotes CH3.
> hole-IgG1 heavy chain constant region (abbreviated as hole-IgG1): 
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPEAAGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKA
LPAPIEKTISKAKGQPREPQVCTLPPSREEMTKNQVSLSCAVKGFYPSDIAVEWESN
GQPENNYKTTPPVLDSDGSFFLVSKLTVDKSRWQQGNVFSCSVMHEALHNHYTQKS
LSLSPGK (SEQ ID NO: 3).
 Note: In the sequence, the single underlined part indicates CH1, the dotted underlined part indicates CH2, and the italic part denotes CH3.
> kappa light chain constant region (abbreviated as kappa or hx): 


(SEQ ID NO: 4).
> lambda light chain constant region (abbreviated as lambda or hλ): 
GQPKAAPSVTLFPPSSEELQANKATLVCLISDFYPGAVTVAWKADSSPVKAGVET
TTPSKQSNNKYAASSYLSLTPEQWKSHRSYSCQVTHEGSTVEKTVAPTECS (SEQ
 ID NO: 5).
> Amino acid sequence of Fc portion of hole-IgG1 heavy chain constant region: 
DKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCK
VSNKALPAPIEKTISKAKGQPREPQVCTLPPSREEMTKNQVSLSCAVKGFYPSDIA
VEWESNGQPENNYKTTPPVLDSDGSFFLVSKLTVDKSRWQQGNVFSCSVMHEALHN
HYTQKSLSLSPGK (SEQ ID NO: 177).
> Amino acid sequence of Fc portion of knob-IgG1 heavy chain constant region: 
DKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCK
VSNKALPAPIEKTISKAKGQPREPQVYTLPPCREEMTKNQVSLWCLVKGFYPSDIA
VEWESNGQPENNYKTTPPVLDSDGSFFLYSKLTVDKSRWQQGNVFSCSVMHEALHN
HYTQKSLSLSPGK (SEQ ID NO: 178).
> Amino acid sequence of CH1 portion of IgG1 heavy chain constant region:

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSC (SEQ ID
NO: 179).

The amino acid sequences of the light and heavy chain variable regions of antibodies against antigens such as B7H3 and CD3 mentioned in the examples or test examples of the present disclosure are as follows:
> Amino acid sequence of VH of F0 antibody: 
QVQLVQSGGGVVQPGTSLRLSCAASGFIFSSSAMHWVRQAPGKGLEWVAVISY
DGSNKYYVDSVKGRFTISRDNSKNTLYLQMNSLRAEDTAVYYCARSARLYASF
DYWGQGALVTVSS (SEQ ID NO: 6).
> Amino acid sequence of VL of F0 antibody: 
DTVVTQEPSFSVSPGGTVTLTCGLSSGSVSTSHYPSWYQQTPGQAPRMLIYNTN
TRSSGVPDRFSGSILGNKAALTITGAQADDESDYYCAIHVDRDIWVFGGGTKLT
VL (SEQ ID NO: 7).
> Amino acid sequence of VH of N0 antibody: 
DVQLVQSGGGLVQPGGSLRLSCAGSGFTSSLSWMSWLRQAPGKGLEWVANINQ
DGSEKNYVDSVKGRFTISRDNAQNSLYLQMSSLRAEDTAVYYCARGLWTFDSW
GQGTLLTVSP (SEQ ID NO: 8).
> Amino acid sequence of VL of N0 antibody: 
EIVLTQSPGTLSLSPGERATLSCRASQNINNRFLAWYQQRPGQAPRLLIYAASSRA
TGVPDRFSGSGSGTDFTLTISRLEAEDFAMYHCQQYGSFPRTFGQGTMVDIK
(SEQ ID NO: 9).
> Amino acid sequence of VH of S0 antibody: 


 (SEQ ID NO: 10).
> Amino acid sequence of VL of S0 antibody: 

 (SEQ
ID NO: 11).
> Amino acid sequence of VH of V0 antibody: 
QVQLQQSAAELARPGASVKMSCKASGYSFTSYTIHWVKQRPGQGLEWIGYINP
NSRNTDYNQKFKDETTLTADRSSSTAYMQLISLTSEDSAVYYCARYSGSTPYWY
FDVWGAGTTVTVSS (SEQ ID NO: 12).
> Amino acid sequence of VL of V0 antibody: 
QIVLTQSPAILSASPGEKVTMTCRASSSVSYMNWYQQKPGSSPKPWIYATSNLAS
GVPARFSVSVSGTSHSLTISRVEAEDAATYYCQQWSSNPLTFGAGTKLELK (SEQ
ID NO: 13).
> Amino acid sequence of VH of J0 antibody: 
EVQLVESGGGLVKPGGSLRLSCAASGFTFSDYWMHWVRQAPGKGLEWVGHIK
SKTDAGTTDYAAPVKGRFTISRDDSKNTLYLQMNSLKTEDTAVYYCAREIYYYA
FDSWGQGTLVTVSS (SEQ ID NO: 14).
> Amino acid sequence of VL of J0 antibody: 
SYELTQPLSVSVALGQTARITCSGDNIGSKYVHWYQQKPGQAPVLVIYGDNERP
SGIPERFSGSNSGNTATLTISRAQAGDEADYYCQAADWVDFYVFGGGTKLTVL
(SEQ ID NO: 15).
> Amino acid sequence of VH of H0 antibody: 
EVQLVESGGGLVQPGGSLRLSCAASGFTFSHYYMAWVRQAPGKGLEWVTSISY
EGDITYYGDSVKGRFTISRDNSKNTLYLQMNSLRAEDTATYYCASQTLRESFDY
WGQGTLVTVSS (SEQ ID NO: 16).
> Amino acid sequence of VL of H0 antibody: 
DIQMTQSPSSVSASVGDRVTITCRASQDIANYLSWYQQKPGKSPKLLIYGTSNLE
VGVPSRFSGSRSGTDFTLTISSLQPEDFATYYCLQDKEFPRTFGGGTKVEIK (SEQ
ID NO: 17).
> Amino acid sequence of VH of R0 antibody: 


 (SEQ ID NO: 18).
> Amino acid sequence of VL of R0 antibody: 


(SEQ ID NO: 19).
> Amino acid sequence of VH of Bmab (abbreviated as B0) antibody: 
QVQLQQSGAEVKKPGSSVRVSCKASGGTFNNNAINWVRQAPGQGLEWMGGIIP
MFGTAKYSQNFQGRVAITADESTGTASMELSSLRSEDTAVYYCARSRDLLLFPH
HALSPWGRGTMVTVSS (SEQ ID NO: 20).
> Amino acid sequence of VL of Bmab (abbreviated as B0) antibody: 
SSELTQDPAVSVALGQTVRVTCQGDSLRSYYASWYQQKPGQAPVLVIYGKNNRP
SGIPDRFSGSSSGNTASLTITGAQAEDEADYYCSSRDSSGNHWVFGGGTELTVL
(SEQ ID NO: 21).
> Amino acid sequence of VH of Umab (abbreviated as U0) antibody: 
EVQLVQSGAEVKKPGESLKISCKGSGYSFTTYWLGWVRQMPGKGLDWIGIMSP
VDSDIRYSPSFQGQVTMSVDKSITTAYLQWNSLKASDTAMYYCARRRPGQGYF
DFWGQGTLVTVSS (SEQ ID NO: 22).
> Amino acid sequence of VL of Umab (abbreviated as U0) antibody: 
DIQMTQSPSSLSASVGDRVTITCRASQGISSWLAWYQQKPEKAPKSLIYAASSLQ
SGVPSRFSGSGSGTDFTLTISSLQPEDFATYYCQQYNIYPYTFGQGTKLEIK (SEQ
ID NO: 23).
> Amino acid sequence of VH of Dmab (abbreviated as D0) antibody: 
EVQLLESGGGLVQPGGSLRLSCAASGFTFSSYAMSWVRQAPGKGLEWVSGITG
SGGSTYYADSVKGRFTISRDNSKNTLYLQMNSLRAEDTAVYYCAKDPGTTVIMS
WFDPWGQGTLVTVSS (SEQ ID NO: 24).
> Amino acid sequence of VL of Dmab (abbreviated as D0) antibody: 
EIVLTQSPGTLSLSPGERATLSCRASQSVRGRYLAWYQQKPGQAPRLLIYGASSR
ATGIPDRFSGSGSGTDFTLTISRLEPEDFAVFYCQQYGSSPRTFGQGTKVEIK (SEQ
ID NO: 25).
> Amino acid sequence of VH of Tmab (abbreviated as 10) antibody: 


 (SEQ ID NO: 26).
> Amino acid sequence of VL of Tmab (abbreviated as 10) antibody: 

 (SEQ
ID NO: 27).
> Amino acid sequence of VH of C0 antibody: 
EVQLVQSGAEVKKPGASVKVSCKASGYTFTDYHMNWVRQAPGQRLEWMGDI
NPDIGGTSYNQNFKGRVTITRDTSASTAYMELSSLRSEDTAVYYCARWDFDSFA
NWGQGTLVTVSS (SEQ ID NO: 28).
> Amino acid sequence of VL of C0 antibody: 
DIVMTQSPDSLAVSLGERATINCRASESVSIIGTNLIHWYQQKPGQPPKLLIYHAS
NLETGVPDRFSGSGSGTDFTLTISSLQAEDVAVYYCLQSRKIPYTFGGGTKVEIK
(SEQ ID NO: 29).
> Amino acid sequence of VH of A0 antibody: 
EVQLVESGGGLVQPGGSLKLSCAASGFTFNKYAMNWVRQAPGKGLEWVARIRS
KYNNYATYYADSVKDRFTISRDDSKNTAYLQMNNLKTEDTAVYYCVRHGNFGN
EYISYWAYWGQGTLVTVSS (SEQ ID NO: 105).
> Amino acid sequence of VL of A0 antibody:

QTVVTQEPSLTVSPGGTVTLTCGSSTGAVTSGNYPNWVQQKPGQAPRGLIGGTK

FLAPGTPARFSGSLLGGKAALTLSGVQPEDEAEYYCVLWYSNRWVFGGGTKLT
VL (SEQ ID NO: 106).

The heavy chain constant region sequences of the antibodies F0, N0, S0, V0, J0, H0, R0, B0, U0, D0, 10, C0, and A0 described above are IgG1 heavy chain constant regions (SEQ ID NO: 1), and the light chain constant region sequences of the antibodies N0, S0, H0, R0, U0, 10, and C0 are kappa light chain constant regions (SEQ ID NO: 4); the light chain constant region sequences of the antibodies F0, V0, J0, B0, D0, and A0 are lambda light chain constant regions (SEQ ID NO: 5).

In addition, the sequence of hB7H3 antigen protein is as follows:

LEVQVPEDPVVALVGTDATLCCSFSPEPGFSLAQLNLIWQLTDTKQLVHSFAEGQ
DQGSAYANRTALFPDLLAQGNASLRLQRVRVADEGSFTCFVSIRDFGSAAVSLQV
AAPYSKPSMTLEPNKDLRPGDTVTITCSSYQGYPEAEVFWQDGQGVPLTGNVT
TSQMANEQGLFDVHSILRVVLGANGTYSCLVRNPVLQQDAHSSVTITPQRSPTG
AVEVQVPEDPVVALVGTDATLRCSFSPEPGFSLAQLNLIWQLTDTKQLVHSFTEG
RDQGSAYANRTALFPDLLAQGNASLRLQRVRVADEGSFTCFVSIRDFGSAAVSLQ
VAAPYSKPSMTLEPNKDLRPGDTVTITCSSYRGYPEAEVFWQDGQGVPLTGNV
TTSQMANEQGLFDVHSVLRVVLGANGTYSCLVRNPVLQQDAHGSVTITGQPMT
DYKDDDDKHHHHHH (SEQ ID NO: 111).

The hCD3 antigen protein is a heterodimer consisting of a δ subunit and an ε subunit of hCD3 antigen, wherein the sequence of the δ subunit of hCD3 antigen is as follows: 
FKIPIEELEDRVFVNCNTSITWVEGTVGTLLSDITRLDLGKRILDPRGIYRCNGTDI
YKDKESTVQVHYRMCQSCVELDPATVASAENAQCEKELQALEKENAQLEWEL
QALEKELAQDYKDDDDK (SEQ ID NO: 112);
 the sequence of the ε subunit of hCD3 antigen is as follows:

DGNEEMGGITQTPYKVSISGTTVILTCPQYPGSEILWQHNDKNIGGDEDDKNIGS
DEDHLSLKEFSELEQSGYYVCYPRGSKPEDANFYLYLRARVCENCMEMDAKN
AQCKKKLQALKKKNAQLKWKLQALKKKLAQHHHHHH (SEQ ID NO: 113).

### Example 2: Replacement of Antibody CH1/CL Domain

The CH1/CL domain of the antibody was replaced with an Ig-like domain possessing natural intermolecular interactions in each of the natural Titin/Obscurin complex, Titin/Obscurin-like O complex, and IL6Ra/IL6Rb complex, so as to obtain CH1/CL domain-engineered antibodies.

In the IL6Ra/IL6Rb complex, the Ig-like domain in the IL6Ra protein for replacing the antibody CH1 or CL is an IL6Ra.0 chain, and the Ig-like domain in the IL6Rb protein for replacing the antibody CL or CH1 is an IL6Rb.0 chain; in the Titin/Obscurin complex, the Ig-like domain (Titin Ig-like 152 domain) in the Titin protein for replacing the antibody CH1 or CL is a T.0 chain, and the Ig-like domain (Obscurin Ig-like-1 domain) in the Obscurin protein for replacing the antibody CL or CH1 is an O.0 chain; in the Titin/Obscurin-like O complex, the Ig-like domain (Obscurin-like-Ig-like-1 domain) in the Obscurin-like protein for replacing the antibody CH1 or CL is an OL.0 chain, and the Ig-like domain (Titin Ig-like 152 domain) in the Titin protein for replacing the antibody CL or CH1 is a T.0 chain, wherein:
> sequence of the IL6Ra.0 chain is: 
GILQPDPPANITVTAVARNPRWLSVTWQDPHSWNSSFYRLRFELRYRAERSKTFT
TWMVKDLQHHCVIHDAWSGLRHVVQLRAQEEFGQGEWSEWSPEAMGTPW
(SEQ ID NO: 30);
> sequence of the IL6Rb.0 chain is: 
FDPVYKVKPNPPHNLSVINSEELSSILKLTWTNPSIKSVIILKYNIQYRTKDASTW
SQIPPEDTASTRSSFTVQDLKPFTEYVFRIRCMKEDGKGYWSDWSEEASGIT
(SEQ ID NO: 31);
> sequence of the T.0 chain is: 
GIPPKIEALPSDISIDEGKVLTVACAFTGEPTPEVTWSCGGRKIHSQEQGRFHIENT
DDLTTLIIMDVQKQDGGLYTLSLGNEFGSDSATVNIHIRSI (SEQ ID NO: 32);
> sequence of the O.0 chain is: 
SGAPRFLTRPKAFVVSVGKDATLSCQIVGNPTPQVSWEKDQQPVAAGARFRLA
QDGDLYRLTILDLALGDSGQYVCRARNAIGEAFAAVGLQVDAEA (SEQ ID NO:
33);
> sequence of the OL.0 chain is:

QGSPPCFLRFPRPVRVVSGAEAELKCVVLGEPPPVVVWEKGGQQLAASERLSFP
ADGAEHGLLLTAALPTDAGVYVCRARNAAGEAYAAAAVTVLEPP (SEQ ID NO:
34).

A plurality of antibodies in which CH1/CL is replaced were constructed, and the purity of the antibodies and the binding activity of the antibodies against corresponding antigens were assayed (see Test Examples 2 and 4 of the present disclosure for the assay method). The experimental results are shown in Table 3 below. The experimental results showed that after CH1/CL domains of antibodies such as F0, V0, S0, N0, J0, H0, R0, and C0 which bound to different antigens were simultaneously replaced with a T.0 chain/O.0 chain or a T.0 chain/OL.0 chain, the antibodies still retained good binding activity and had high purity.

**Table 3. The purity and antigen binding assay results for the antibodies**

| Antibody name | | CH1/CL and peptides replacing same | | Purity of antibody SEC (%) | Antigen binding assay EC50 (nM) |
|---|---|---|---|---|---|
| | | CH1 | CL | | |
| Series F0 | F0 | CH1 | CL | 98 | 1.29 |
| | F1 | IL6Rb.0 | IL6Ra.0 | 47 | 71.6 |
| | F2 | IL6Ra.0 | IL6Rb.0 | 67 | 59.28 |
| | F3 | T.0 | O.0 | 100 | 2.83 |
| | F4 | T.0 | OL.0 | 100 | 2.17 |
| Series V0 | V0 | CH1 | CL | 86 | 1.8 |
| | V1 | IL6Rb.0 | IL6Ra.0 | 38 | 38.4 |
| | V2 | IL6Ra.0 | IL6Rb.0 | NA | - |
| | V3 | T.0 | O.0 | 99 | 3.6 |
| | V4 | T.0 | OL.0 | 97 | 2.9 |
| | V5 | O.0 | T.0 | 98 | 3.2 |
| | V6 | OL.0 | T.0 | 97 | 4.7 |
| Series S0 | S0 | CH1 | CL | 97 | 1.01 |
| | S1 | IL6Rb.0 | IL6Ra.0 | NA | - |
| | S2 | IL6Ra.0 | IL6Rb.0 | NA | - |
| | S3 | T.0 | OL.0 | 83 | 3.5 |
| | S4 | OL.0 | T.0 | 90 | 1.96 |
| Series N0 | N0 | CH1 | CL | 99 | 0.82 |
| | N1 | IL6Rb.0 | IL6Ra.0 | 100 | 1229 |
| | N2 | IL6Ra.0 | IL6Rb.0 | 67 | 766.5 |
| | N3 | T.0 | O.0 | 90 | 6.45 |
| | N4 | T.0 | OL.0 | 83 | 5.93 |
| | N5 | O.0 | T.0 | 90 | 5.24 |
| Series J0 | J0 | CH1 | CL | 95 | 0.08 |
| | J1 | IL6Rb.0 | IL6Ra.0 | NA | - |
| | J2 | IL6Ra.0 | IL6Rb.0 | NA | 179 |
| | J3 | OL.0 | T.0 | 93 | 0.25 |
| Series H0 | H0 | CH1 | CL | NA | 1.74 |
| | H1 | IL6Rb.0 | IL6Ra.0 | 75 | 19.35 |
| | H2 | IL6Ra.0 | IL6Rb.0 | 80 | 24.38 |
| | H3 | T.0 | O.0 | 95 | 2.753 |
| | H4 | T.0 | OL.0 | 95 | 2.702 |
| Series R0 | R0 | CH1 | CL | NA | 1.75 |
| | R1 | IL6Rb.0 | IL6Ra.0 | 75 | 24.45 |
| | R2 | IL6Ra.0 | IL6Rb.0 | 60 | 18.4 |
| | R3 | O.0 | T.0 | 98 | 1.576 |
| | R4 | OL.0 | T.0 | 98 | 1.667 |
| | R5 | T.0 | O.0 | 98 | 1.589 |
| | R6 | T.0 | OL.0 | 97 | 2.016 |
| Series C0 | C0 | CH1 | CL | 96 | 5.81 |
| | C1 | O.0 | T.0 | 95 | 17.7 |
| | C2 | OL.0 | T.0 | 99 | 16.48 |

| | | | | | |
|---|---|---|---|---|---|
| Note: For example, F1 indicates an antibody obtained by replacement of the heavy chain CH1 of the F0 antibody with the IL6Rb.0 chain (SEQ ID NO: 31) and replacement of the light chain CL with the IL6Ra.0 chain (SEQ ID NO: 30), with the other portions remained the same as those of F0, and so on for others. In the table, "-" indicates no detection, and SEC indicates the purity of the antibodies determined by size exclusion chromatography (NA indicates no detection due to underexpression). | | | | | |

### Example 3: Optimization Design for CH1/CL Domain-Engineered Antibody

### I. Mutations on individual amino acid residues of the Titin Ig-like 152 domain (T.0 chain), the Obscurin Ig-like 1 domain (O.0 chain), and the Obscurin-like Ig-like 1 domain (OL.0 chain)

Illustratively, firstly, amino acid residues in the T.0 chain, O.0 chain, and OL.0 chain were mutated to increase inter-chain disulfide bonds; secondly, other individual amino acids in the domains were also mutated, for example, amino acids at positions 7, 62, and 11 of the Obscurin Ig-like 1 domain were mutated; 5 amino acids "KAGIR (SEQ ID NO: 180)" in 5 wild-type Titin proteins which are immediately adjacent to the N-terminus of the Titin Ig-like 152 domain were added to the N-terminus of the Titin Ig-like 152 domain; 5 amino acids "DQPQF (SEQ ID NO: 181)" in 5 wild-type Obscurin proteins which are immediately adjacent to the N-terminus of the Obscurin Ig-like 1 domain were added to the N-terminus of the Obscurin Ig-like 1 domain. The specific domain optimization design is shown in Tables 4-1 to 4-3.

**Table 4-1. Amino acid mutations on the T.0 chain**

| Titin-T chain | | |
|---|---|---|
| Name | Mode of mutation | |
| T.0 | Wild type (SEQ ID NO: 32) | |
| T.1 | C25S, C39T, A8C | |
| T.2 | C25S, C39T, V20C | |
| T.3 | C25S, C39T, A26C | |
| T.4 | C25S, C39T, T22C | |
| T.5 | | C25S, C39T, A8C, N-terminus + Titin KAGIR |
| T.6 | | N-terminus + Titin KAGIR |

| | | |
|---|---|---|
| Note: In the table, for example, the mode of mutation "C25S, C39T, A8C" for T.1 indicates that the amino acid residue at position 25 of the T.0 (SEQ ID NO: 32) sequence was mutated from C to S, the amino acid residue at position 39 was mutated from C to T, and the amino acid residue at position 8 was mutated from C to A; the mode of mutation "N-terminus + Titin KAGIR" for T.6 indicates that 5 amino acids "KAGIR" were added to the N-terminus of the T.0 (SEQ ID NO: 32) sequence; and so on for others. | | |

**Table 4-2. Amino acid mutations on the O.0 chain**

| Name | Mode of mutation |
|---|---|
| O.0 | Wild type (SEQ ID NO: 33) |
| O.1 | A88C |
| O.2 | A3C |
| O.3 | R9C |
| O.4 | C25S, C76S, A88C |
| O.5 | C25S, C76S, A3C |
| O.6 | C25S, C76S, R9C |
| O.7 | C25S, C76S, A88C, L7K, T62K |
| O.8 | C25S, C76S, A88C, L7K, T62H |
| O.9 | C25S, C76S, A88C, K11L, T62K |
| O.10 | C25S, C76S, A88C, K11L, T62H |
| O.11 | C25S, C76S, A88C, N-terminus + Obscurin DQPQF |
| O.12 | C25S, C76S, A88C, L7K, T62K, N-terminus + Obscurin DQPQF |
| O.13 | C25S, C76S, A88C, L7K, T62H, N-terminus + Obscurin DQPQF |
| O.14 | C25S, C76S, A88C, L7R, T62K, N-terminus + Obscurin DQPQF |
| O.15 | C25S, C76S, A88C, L7R, T62H, N-terminus + Obscurin DQPQF |
| O.16 | N-terminus + Obscurin DQPQF |

| | |
|---|---|
| Note: In the table, the mode of mutation "A88C" for O.1 indicates that the amino acid residue at position 88 of the O.0 (SEQ ID NO: 33) sequence was mutated from A to C; the mode of mutation "C25S, C76S, A88C, N-terminus + Obscurin DQPQF" for O.11 indicates that the O.0 (SEQ ID NO: 33) sequence was subjected to C25S, C76S, and A88C amino acid mutations, and 5 amino acids "DQPQF" were added at the N-terminus of O.0; and so on for others. | |

**Table 4-3. Amino acid mutations on the OL.0 chain**

| Obscurin-like-O chain | |
|---|---|
| Name | Mode of mutation |
| OL.0 | Wild type (SEQ ID NO: 34) |
| OL.1 | C6E, V74C |
| OL.2 | C6E, G84C |
| OL.3 | C6E, A86C |
| OL.4 | C6E, C26S, C77S, V74C |
| OL.5 | C6E, C26S, C77S, G84C |
| OL.6 | C6E, C26S, C77S, A86C |

| | |
|---|---|
| Note: In the table, for example, the mode of mutation "C6E, V74C" for OL.1 indicates that the amino acid residue at position 6 of the OL.0 (SEQ ID NO: 34) sequence was mutated from C to E, and the amino acid residue at position 74 was mutated from V to C; and so on for others. | |

The sequences of the Titin-T chain, the Obscurin-O chain, and the Obscurin-like-O chain after mutations are as follows:
> T. 1 (T.0 with mutations of C25S, C39T, and A8C) 
GIPPKIECLPSDISIDEGKVLTVASAFTGEPTPEVTWSTGGRKIHSQEQGRFHIENT
DDLTTLIIMDVQKQDGGLYTLSLGNEFGSDSATVNIHIRSI (SEQ ID NO: 35);
> T.2 (T.0 with mutations of C25S, C39T, and V20C) GIPPKIEALPSDISIDEGKCLTVASAFTGEPTPEVTWSTGGRKIHSQEQGRFHIENT DDLTTLIIMDVQKQDGGLYTLSLGNEFGSDSATVNIHIRSI (SEQ ID NO: 36);
> T.3 (T.0 with mutations of C25S, C39T, and A26C) 
GIPPKIEALPSDISIDEGKVLTVASCFTGEPTPEVTWSTGGRKIHSQEQGRFHIENT
DDLTTLIIMDVQKQDGGLYTLSLGNEFGSDSATVNIHIRSI (SEQ ID NO: 37);
> T.4 (T.0 with mutations of C25S, C39T, and T22C) 
GIPPKIEALPSDISIDEGKVLCVASAFTGEPTPEVTWSTGGRKIHSQEQGRFHIENT
DDLTTLIIMDVQKQDGGLYTLSLGNEFGSDSATVNIHIRSI (SEQ ID NO: 38);
> T.5 (T.0 with mutations of C25S, C39T, and A8C; with KAGIR added to the N-terminus) 
KAGIRGIPPKIECLPSDISIDEGKVLTVASAFTGEPTPEVTWSTGGRKIHSQEQGRF
HIENTDDLTTLIIMDVQKQDGGLYTLSLGNEFGSDSATVNIHIRSI (SEQ ID NO:
39);
> T.6 (T.0 with KAGIR added to the N-terminus) 
KAGIRGIPPKIEALPSDISIDEGKVLTVACAFTGEPTPEVTWSCGGRKIHSQEQGR
FHIENTDDLTTLIIMDVQKQDGGLYTLSLGNEFGSDSATVNIHIRSI (SEQ ID NO:
127);
> T.1-L1 (T.0 with mutations of C25S, C39T, and A8C; with a (G₄S)₁ linker added to the N-terminus) 
GGGGSGIPPKIECLPSDISIDEGKVLTVASAFTGEPTPEVTWSTGGRKIHSQEQGR
FHIENTDDLTTLIIMDVQKQDGGLYTLSLGNEFGSDSATVNIHIRSI (SEQ ID NO:
40);
> T.1-L2 (T.0 with mutations of C25S, C39T, and A8C; with a (G₄S)₂ linker added to the N-terminus) 
GGGGSGGGGSGIPPKIECLPSDISIDEGKVLTVASAFTGEPTPEVTWSTGGRKIHS
QEQGRFHIENTDDLTTLIIMDVQKQDGGLYTLSLGNEFGSDSATVNIHIRSI (SEQ
ID NO: 41);
> O.1 **(O.0** with an A88C mutation) 
SGAPRFLTRPKAFVVSVGKDATLSCQIVGNPTPQVSWEKDQQPVAAGARFRLA
QDGDLYRLTILDLALGDSGQYVCRARNAIGEAFACVGLQVDAEA (SEQ ID NO:
42);
> O.2 **(O.0** with an A3C mutation) 
SGCPRFLTRPKAFVVSVGKDATLSCQIVGNPTPQVSWEKDQQPVAAGARFRLAQ
DGDLYRLTILDLALGDSGQYVCRARNAIGEAFAAVGLQVDAEA (SEQ ID NO:
43);
> O.3 **(O.0** with an R9C mutation) 
SGAPRFLTCPKAFVVSVGKDATLSCQIVGNPTPQVSWEKDQQPVAAGARFRLA
QDGDLYRLTILDLALGDSGQYVCRARNAIGEAFAAVGLQVDAEA (SEQ ID NO:
44);
> O.4 **(O.0** with mutations of C25S, C76S, and A88C) 
SGAPRFLTRPKAFVVSVGKDATLSSQIVGNPTPQVSWEKDQQPVAAGARFRLAQ
DGDLYRLTILDLALGDSGQYVSRARNAIGEAFACVGLQVDAEA (SEQ ID NO:
 45);
> O.5 **(O.0** with mutations of C25S, C76S, and A3C) 
SGCPRFLTRPKAFVVSVGKDATLSSQIVGNPTPQVSWEKDQQPVAAGARFRLAQ
DGDLYRLTILDLALGDSGQYVSRARNAIGEAFAAVGLQVDAEA (SEQ ID NO:
46);
> O.6 **(O.0** with mutations of C25S, C76S, and R9C) 
SGAPRFLTCPKAFVVSVGKDATLSSQIVGNPTPQVSWEKDQQPVAAGARFRLAQ
DGDLYRLTILDLALGDSGQYVSRARNAIGEAFAAVGLQVDAEA (SEQ ID NO:
47);
> O.7 **(O.0** with mutations of C25S, C76S, A88C, L7K, and T62K) 
SGAPRFKTRPKAFVVSVGKDATLSSQIVGNPTPQVSWEKDQQPVAAGARFRLA
QDGDLYRLKILDLALGDSGQYVSRARNAIGEAFACVGLQVDAEA (SEQ ID NO:
48);
> O.8 **(O.0** with mutations of C25S, C76S, A88C, L7K, and T62H) 
SGAPRFKTRPKAFVVSVGKDATLSSQIVGNPTPQVSWEKDQQPVAAGARFRLA
QDGDLYRLHILDLALGDSGQYVSRARNAIGEAFACVGLQVDAEA (SEQ ID NO:
49);
> O.9 **(O.0** with mutations of C25S, C76S, A88C, K11L, and T62K) 
SGAPRFLTRPLAFVVSVGKDATLSSQIVGNPTPQVSWEKDQQPVAAGARFRLAQ
DGDLYRLKILDLALGDSGQYVSRARNAIGEAFACVGLQVDAEA (SEQ ID NO:
50);
> O.10 **(O.0** with mutations of C25S, C76S, A88C, K11L, and T62H) 
SGAPRFLTRPLAFVVSVGKDATLSSQIVGNPTPQVSWEKDQQPVAAGARFRLAQ
DGDLYRLHILDLALGDSGQYVSRARNAIGEAFACVGLQVDAEA (SEQ ID NO:
51);
> O.11 **(O.0** with mutations of C25S, C76S, and A88C; with DQPQF added to the N-terminus) 
DQPQFSGAPRFLTRPKAFVVSVGKDATLSSQIVGNPTPQVSWEKDQQPVAAGAR
FRLAQDGDLYRLTILDLALGDSGQYVSRARNAIGEAFACVGLQVDAEA (SEQ ID
NO: 52);
> 0.12 **(O.0** with mutations of C25S, C76S, A88C, L7K, and T62K; with DQPQF added to the N-terminus) 
DQPQFSGAPRFKTRPKAFVVSVGKDATLSSQIVGNPTPQVSWEKDQQPVAAGA
RFRLAQDGDLYRLKILDLALGDSGQYVSRARNAIGEAFACVGLQVDAEA (SEQ
ID NO: 53);
> 0.13 **(O.0** with mutations of C25S, C76S, A88C, L7K, and T62H; with DQPQF added to the N-terminus) 
DQPQFSGAPRFKTRPKAFVVSVGKDATLSSQIVGNPTPQVSWEKDQQPVAAGA
RFRLAQDGDLYRLHILDLALGDSGQYVSRARNAIGEAFACVGLQVDAEA (SEQ
ID NO: 54);
> 0.14 **(O.0** with mutations of C25S, C76S, A88C, L7R, and T62K; with DQPQF added to the N-terminus) 
DQPQFSGAPRFRTRPKAFVVSVGKDATLSSQIVGNPTPQVSWEKDQQPVAAGAR
FRLAQDGDLYRLKILDLALGDSGQYVSRARNAIGEAFACVGLQVDAEA (SEQ
ID NO: 55);
> 0.15 (O.0 with mutations of C25S, C76S, A88C, L7R, and T62H; with DQPQF added to the N-terminus) 
DQPQFSGAPRFRTRPKAFVVSVGKDATLSSQIVGNPTPQVSWEKDQQPVAAGAR
FRLAQDGDLYRLHILDLALGDSGQYVSRARNAIGEAFACVGLQVDAEA (SEQ
ID NO: 56);
> 0.16 (O.0 with DQPQF added to the N-terminus) 
DQPQFSGAPRFLTRPKAFVVSVGKDATLSCQIVGNPTPQVSWEKDQQPVAAGA
RFRLAQDGDLYRLTILDLALGDSGQYVCRARNAIGEAFAAVGLQVDAEA (SEQ
ID NO: 128);
> O.4-L1 (O.0 with mutations of C25S, C76S, and A88C; with a (G₄S)₁ linker added to the N-terminus) 
GGGGSSGAPRFLTRPKAFVVSVGKDATLSSQIVGNPTPQVSWEKDQQPVAAGA
RFRLAQDGDLYRLTILDLALGDSGQYVSRARNAIGEAFACVGLQVDAEA (SEQ
ID NO: 57);
> O.4-L2 (O.0 with mutations of C25S, C76S, and A88C; with a (G₄S)₂ linker added to the N-terminus) 
GGGGSGGGGSSGAPRFLTRPKAFVVSVGKDATLSSQIVGNPTPQVSWEKDQQPV
AAGARFRLAQDGDLYRLTILDLALGDSGQYVSRARNAIGEAFACVGLQVDAEA
(SEQ ID NO: 58);
> OL. 1 (OL.0 with mutations of C6E and V74C) 
QGSPPEFLRFPRPVRVVSGAEAELKCVVLGEPPPVVVWEKGGQQLAASERLSFP
ADGAEHGLLLTAALPTDAGCYVCRARNAAGEAYAAAAVTVLEPP (SEQ ID NO:
59);
> OL.2 (OL.0 with mutations of C6E and G84C) 
QGSPPEFLRFPRPVRVVSGAEAELKCVVLGEPPPVVVWEKGGQQLAASERLSFP
ADGAEHGLLLTAALPTDAGVYVCRARNAACEAYAAAAVTVLEPP (SEQ ID NO:
60);
> OL.3 (OL.0 with mutations of C6E and A86C) 
QGSPPEFLRFPRPVRVVSGAEAELKCVVLGEPPPVVVWEKGGQQLAASERLSFP
ADGAEHGLLLTAALPTDAGVYVCRARNAAGECYAAAAVTVLEPP (SEQ ID NO:
61);
> OL.4 (OL.0 with mutations of C6E, C26S, C77S, and V74C) 
QGSPPEFLRFPRPVRVVSGAEAELKSVVLGEPPPVVVWEKGGQQLAASERLSFP
ADGAEHGLLLTAALPTDAGCYVSRARNAAGEAYAAAAVTVLEPP (SEQ ID NO:
62);
> OL.5 (OL.0 with mutations of C6E, C26S, C77S, and G84C) QGSPPEFLRFPRPVRVVSGAEAELKSVVLGEPPPVVVWEKGGQQLAASERLSFP 
ADGAEHGLLLTAALPTDAGVYVSRARNAACEAYAAAAVTVLEPP (SEQ ID NO:
63);
> OL.6 (OL.0 with mutations of C6E, C26S, C77S, and A86C) 
QGSPPEFLRFPRPVRVVSGAEAELKSVVLGEPPPVVVWEKGGQQLAASERLSFP
ADGAEHGLLLTAALPTDAGVYVSRARNAAGECYAAAAVTVLEPP (SEQ ID NO:
64).
Note: In the above sequences, the double-underlined part indicates a linker sequence.

The antibodies in which CH1/CL is replaced were constructed using the Titin-T chain, the Obscurin-O chain, and the Obscurin-like-O chain described above (see Table 5 for specific replacements), and the binding activity of each of the mutant antibodies after optimization against the corresponding antigen was assayed by the method of Test Example 4 of the present disclosure. The experimental results are shown in Table 5. The results showed that the modified antibody retained good antigen-binding activity.

**Table 5. Results for antigen binding assay on the antibodies**

| Antibody name | | Peptides replacing CH1/CL | | Binding assay to the corresponding antigen EC50 (nM) |
|---|---|---|---|---|
| | | CH1 | CL | |
| Series F0 | F3 | T.0 | O.0 | 2.64 |
| | F5 | T.1 | O.1 | 1.21 |
| | F6 | T.2 | O.2 | 2.71 |
| | F7 | T.3 | O.3 | 3.38 |
| | F8 | T.1 | O.4 | 3.14 |
| | F9 | T.2 | O.5 | 3.52 |
| | F10 | T.3 | O.6 | 3.94 |
| | F4 | T.0 | OL.0 | 3.01 |
| | F11 | T.1 | OL.1 | 6.15 |
| | F12 | T.2 | OL.2 | 8.19 |
| | F13 | T.4 | OL.3 | 6.08 |
| | F14 | T.1 | OL.4 | 5.15 |
| | F15 | T.2 | OL.5 | 4.44 |
| Series H0 | H3 | T.0 | O.0 | 3.24 |
| | H5 | T.1 | O.1 | 3.40 |
| | H6 | T.2 | O.2 | 2.10 |
| | H7 | T.3 | O.3 | 2.70 |
| | H8 | T.1 | O.4 | 2.11 |
| | H9 | T.2 | O.5 | 2.92 |
| | H10 | T.3 | O.6 | 2.86 |
| | H4 | T.0 | OL.0 | 3.28 |
| | H11 | T.1 | OL.1 | 3.38 |
| | H12 | T.2 | OL.2 | 4.34 |
| | H13 | T.4 | OL.3 | 3.56 |
| | H14 | T.1 | OL.4 | 2.61 |
| | H15 | T.4 | OL.6 | 4.26 |
| Series R0 | R5 | T.0 | O.0 | 4.13 |
| | R7 | T.1 | O.1 | 4.89 |
| | R8 | T.2 | O.2 | 3.84 |
| | R9 | T.3 | O.3 | 3.95 |
| | R10 | T.1 | O.4 | 3.20 |
| | R11 | T.2 | O.5 | 3.30 |
| | R12 | T.3 | O.6 | 4.36 |
| Series N0 | N3 | T.0 | O.0 | 4.5 |
| | N6 | T.2 | O.5 | 8.4 |
| | N4 | T.0 | OL.0 | 4.6 |
| | N7 | T.5 | OL.1 | 2.8 |
| | N8 | T.5 | OL.4 | 2.6 |

| | | | | |
|---|---|---|---|---|
| Note: For example, the antibody "F5" in the table indicates an antibody obtained by replacement of the heavy chain CH1 of the F0 antibody with the T.1 chain (SEQ ID NO: 35) and replacement of the light chain CL with the O.1 chain (SEQ ID NO: 42), with the other portions being the same as those of F0; and so on for others. | | | | |

In addition, the antibodies in which CH1/CL is replaced were constructed by using the mutated or domain-engineered Titin-T chain and Obscurin-O chain described above, and the functional activity of other antibodies in which CH1/CL is replaced was assayed by the methods of Test Example 2 and Test Example 4 of the present disclosure. The experimental results are shown in Table 6. The experimental results showed that the antibodies in which CH1/CL is replaced still retained good antigen binding activity and had relatively high purity SEC (%).

**Table 6. Assay results for purity and binding activity of domain-engineered antibodies**

| Antibody name | | Peptides replacing CH1/CL | | Antibody purity SEC (%) | Binding assay to the corresponding antigen EC50 (nM) |
|---|---|---|---|---|---|
| | | CH1 | CL | | |
| F0 series | F16 | T.5 | O.4 | 65 | 2.0 |
| | F17 | T.5 | O.7 | 72 | 4.8 |
| | F18 | T.1 | O.8 | 70 | 3.9 |
| | F19 | T.5 | O.8 | 68 | 2.2 |
| | F20 | T.1 | O.9 | 71 | 1.9 |
| | F21 | T.5 | O.9 | 68 | 3.4 |
| | F22 | T.1 | O.10 | 74 | 2.4 |
| | F23 | T.5 | O.10 | 73 | 4.1 |
| | F24 | T.1 | O.12 | 71 | 2.4 |
| | F25 | T.5 | O.12 | 66 | 2.6 |
| | F26 | T.1 | O.13 | 73 | 2.6 |
| | F27 | T.5 | O.13 | 71 | 1.2 |
| | F28 | T.1 | O.14 | 75 | 2.4 |
| | F29 | T.5 | O.14 | 68 | 3.6 |
| | F30 | T.1 | O.15 | 75 | 3.4 |
| | F31 | T.5 | O.15 | 69 | 2.4 |
| | F32 | T.1-L1 | O.4-L2 | 72 | 3.69 |
| | F33 | T.1-L2 | O.4-L1 | 69 | 1.92 |
| | F34 | T.1-L2 | O.4-L2 | 73 | 2.38 |
| Series N0 | N10 | T.5 | O.4 | 89 | 3.0 |
| | N11 | T.1 | O.7 | 86 | 5.0 |
| | N12 | T.1 | O.8 | 84 | 5.3 |
| | N13 | T.5 | O.8 | 82 | 2.9 |
| | N14 | T.1 | O.9 | 92 | 1.9 |
| | N15 | T.5 | O.9 | 92 | 2.6 |
| | N16 | T.5 | O.10 | 91 | 3.4 |
| | N17 | T.1 | O.11 | 90 | 2.2 |
| | N18 | T.5 | O.11 | 89 | 3.2 |
| | N19 | T.1 | O.12 | 84 | 4.3 |
| | N20 | T.5 | O.12 | 84 | 2.8 |
| | N21 | T.5 | O.13 | 84 | 5.2 |
| | N22 | T.5 | O.14 | 87 | 4.5 |
| | N23 | T.1 | O.15 | 86 | 9.4 |
| | N24 | T.5 | O.15 | 84 | 6.7 |
| | N25 | T.1-L1 | O.4-L2 | 92 | 0.62 |
| | N26 | T.1-L2 | O.4-L1 | 93 | 0.84 |
| | N27 | T.1-L2 | O.4-L2 | 94 | 0.67 |
| Series R0 | R10 | T.1 | O.4 | 84 | 0.37 |
| | R13 | T.5 | O.4 | 76 | 0.4 |
| | R14 | T.5 | O.7 | 78 | 0.2 |
| | R15 | T.5 | O.8 | 82 | 0.3 |
| | R16 | T.5 | O.9 | 80 | 0.33 |
| | R17 | T.5 | O.10 | 77 | 0.4 |
| | R18 | T.5 | O.11 | 71 | 0.44 |
| | R19 | T.5 | O.12 | 78 | 0.36 |
| | R20 | T.5 | O.13 | 75 | 0.39 |
| | R21 | T.5 | O.14 | 77 | 0.37 |
| | R22 | T.5 | O.15 | 74 | 0.39 |

| | | | | | |
|---|---|---|---|---|---|
| Note: For example, the antibody "F16" in the table indicates an antibody obtained by replacement of the heavy chain CH1 of the F0 antibody with the T.5 chain (SEQ ID NO: 39) and replacement of the light chain CL with the O.4 chain (SEQ ID NO: 45), with the other portions remained the same as those of F0; and so on for others. | | | | | |

### II. Mutations on the other amino acids of the Titin Ig-like 152 domain and the Obscurin Ig-like 1 domain

The specific design for amino acid mutations on the Titin Ig-like 152 domain and the Obscurin Ig-like 1 domain is shown in Table 7.

**Table 7. Design for amino acid mutations on a T.1 chain/O.9 chain**

| Titin-T chain | | Obscurin-O chain | |
|---|---|---|---|
| Name | Mode of mutation | Name | Mode of mutation |
| T.1 | T.1 (SEQ ID NO: 35) | O.9 | O.9 (SEQ ID NO: 50) |
| T.7 | T.1+ (M66S, T77S) | O.17 | O.9+ (L11K, A12S, F13Y, V14T, T22S) |
| T.8 | T.1+ (M66K, K70R, S79T, G81R) | O.18 | O.9+ (G2E, V17E, N30D, T32P, Q34E, S36T, V44I, A45T, L58V, K62E, A67Q, G69S, A97G) |
| T.9 | T.1+ (P3W, S11I, I13L, T22M, N82M) | O.19 | O.9+ (D20L, T22M, A53L) |
| T.10 | T.1+ (S11I, M66K, S79T, G81R) | O.20 | O.9+ (Q41K, A45T, A67Q, G69S, V89L) |
| T.11 | T.1+ (G40S, R42K, H45S, Q47E, Q49G, N56S, D58E, L75V, E83D, F84L) | O.21 | O.9+ (Q42L, A45T, A67T, G69S, Q92E, D94G) |
| T.12 | T.1+ (Q47E, Q49G, N56S, D58E, L75V) | O.22 | O.9+ (A12S, F13Y, T22S, Q42L, A45T, A67Q, G69S, Q92E, D94G) |
| T.13 | T.1+ (N56S, D58E, L75V) | O.23 | O.9 + (A12S, F13Y, T22S, Q42L, A45T, A67Q, G69S, Q92E, D94G), N-terminus + DQPQF |
| T.14 | T.1+ (N56S, D58E, M66S, T77S) | | |
| T.15 | T.1 + (N56S, D58E, M66S, T77S), N-terminus + KAGIR | | |

| | | | |
|---|---|---|---|
| Note: In the table, for example, T.7 indicates a Titin-T chain obtained by amino acid mutations of M66S and T77S on the T. 1 (SEQ ID NO: 35) sequence; O.23 indicates an Obscurin-O chain obtained by amino acid mutations of A12S, F13Y, T22S, Q42L, A45T, A67Q, G69S, Q92E, and D94G on O.9 (SEQ ID NO: 50), followed by the addition of a "DQPQF" sequence at the N-terminus, and so on for others. | | | |

The mutant sequences of the T.1 chain and the O.9 chain are as follows:
> T.7 (T.1 with M66S and T77S mutations) 
GIPPKIECLPSDISIDEGKVLTVASAFTGEPTPEVTWSTGGRKIHSQEQGRFHIENT
DDLTTLIISDVQKQDGGLYSLSLGNEFGSDSATVNIHIRSI (SEQ ID NO: 65);
> T.8 (T.1 with M66K, K70R, S79T, and G81R mutations) 
GIPPKIECLPSDISIDEGKVLTVASAFTGEPTPEVTWSTGGRKIHSQEQGRFHIENT
DDLTTLIIKDVQRQDGGLYTLTLRNEFGSDSATVNIHIRSI (SEQ ID NO: 66);
> T.9 (T.1 with P3W, S11I, I13L, T22M, and N82M mutations) 
GIWPKIECLPIDLSIDEGKVLMVASAFTGEPTPEVTWSTGGRKIHSQEQGRFHIEN
TDDLTTLIIMDVQKQDGGLYTLSLGMEFGSDSATVNIHIRSI (SEQ ID NO: 67);
> T.10 (T.1 with S11I, M66K, S79T, and G81R mutations) 
GIPPKIECLPIDISIDEGKVLTVASAFTGEPTPEVTWSTGGRKIHSQEQGRFHIENT
DDLTTLIIKDVQKQDGGLYTLTLRNEFGSDSATVNIHIRSI (SEQ ID NO: 68);
> T.11 (T.1 with G40S, R42K, H45S, Q47E, Q49G, N56S, D58E, L75V, E83D, and F84L mutations) 
GIPPKIECLPSDISIDEGKVLTVASAFTGEPTPEVTWSTSGKKISSEEGGRFHIESTE
DLTTLIIMDVQKQDGGVYTLSLGNDLGSDSATVNIHIRSI (SEQ ID NO: 69);
> T.12) (T. 1 with mutations of Q47E, Q49G, N56S, D58E, and L75V) 
GIPPKIECLPSDISIDEGKVLTVASAFTGEPTPEVTWSTGGRKIHSEEGGRFHIEST
EDLTTLIIMDVQKQDGGVYTLSLGNEFGSDSATVNIHIRSI (SEQ ID NO: 70);
> T. 13 (T.1 with mutations of N56S, D58E, and L75V) 
GIPPKIECLPSDISIDEGKVLTVASAFTGEPTPEVTWSTGGRKIHSQEQGRFHIEST
EDLTTLIIMDVQKQDGGVYTLSLGNEFGSDSATVNIHIRSI (SEQ ID NO: 71);
> T. 14 (T.1 with mutations of N56S, D58E, M66S, and T77S) 
GIPPKIECLPSDISIDEGKVLTVASAFTGEPTPEVTWSTGGRKIHSQEQGRFHIEST
EDLTTLIISDVQKQDGGLYSLSLGNEFGSDSATVNIHIRSI (SEQ ID NO: 72);
> T. 15 (T.1 with mutations of N56S, D58E, M66S, and T77S, N-terminus + KAGIR) 
KAGIRGIPPKIECLPSDISIDEGKVLTVASAFTGEPTPEVTWSTGGRKIHSQEQGRF
HIESTEDLTTLIISDVQKQDGGLYSLSLGNEFGSDSATVNIHIRSI (SEQ ID NO:
74);
> T.10-L1 (T.1 with mutations of Sill, M66K, S79T, and G81R, + (G₄S)₁ linker sequence) 
GGGGSGIPPKIECLPIDISIDEGKVLTVASAFTGEPTPEVTWSTGGRKIHSQEQGRF
HIENTDDLTTLIIKDVQKQDGGLYTLTLRNEFGSDSATVNIHIRSI (SEQ ID NO:
73);
> T.15-L1 (T.1 with mutations of N56S, D58E, M66S, and T77S, N-terminus + KAGIR, + (G₄S)₁ linker sequence) 
GGGGSKAGIRGIPPKIECLPSDISIDEGKVLTVASAFTGEPTPEVTWSTGGRKIHS
QEQGRFHIESTEDLTTLIISDVQKQDGGLYSLSLGNEFGSDSATVNIHIRSI (SEQ
ID NO: 75);
> T.14-L1 (T.1 with mutations of N56S, D58E, M66S, and T77S, + (G₄S)₁ linker sequence) 
GGGGSGIPPKIECLPSDISIDEGKVLTVASAFTGEPTPEVTWSTGGRKIHSQEQGR
FHIESTEDLTTLIISDVQKQDGGLYSLSLGNEFGSDSATVNIHIRSI (SEQ ID NO:
76);
> 0.17 (O.9 with mutations of L11K, A12S, F13Y, V14T, and T22S) 
SGAPRFLTRPKSYTVSVGKDASLSSQIVGNPTPQVSWEKDQQPVAAGARFRLAQ
DGDLYRLKILDLALGDSGQYVSRARNAIGEAFACVGLQVDAEA (SEQ ID NO:
77);
> 0.18 (O.9 with mutations of G2E, V17E, N30D, T32P, Q34E, S36T, V44I, A45T, L58V, K62E, A67Q, G69S, and A97G) 
SEAPRFLTRPLAFVVSEGKDATLSSQIVGDPPPEVTWEKDQQPITAGARFRLAQD
GDVYRLEILDLQLSDSGQYVSRARNAIGEAFACVGLQVDAEG (SEQ ID NO: 78);
> 0.19 (O.9 with mutations of D20L, T22M, and A53L) 
SGAPRFLTRPLAFVVSVGKLAMLSSQIVGNPTPQVSWEKDQQPVAAGARFRLLQ
DGDLYRLKILDLALGDSGQYVSRARNAIGEAFACVGLQVDAEA (SEQ ID NO:
79);
> O.20 (O.9 with mutations of Q41K, A45T, A67Q, G69S, and V89L) 
SGAPRFLTRPLAFVVSVGKDATLSSQIVGNPTPQVSWEKDKQPVTAGARFRLAQ
DGDLYRLKILDLQLSDSGQYVSRARNAIGEAFACLGLQVDAEA (SEQ ID NO:
80);
> O.21 (O.9 with mutations of Q42L, A45T, A67T, G69S, Q92E, and D94G) 
SGAPRFLTRPLAFVVSVGKDATLSSQIVGNPTPQVSWEKDQLPVTAGARFRLAQ
DGDLYRLKILDLTLSDSGQYVSRARNAIGEAFACVGLEVGAEA (SEQ ID NO:
81);
> O.22 (O.9 with mutations of A12S, F13Y, T22S, Q42L, A45T, A67Q, G69S, Q92E, and D94G) 
SGAPRFLTRPLSYVVSVGKDASLSSQIVGNPTPQVSWEKDQLPVTAGARFRLAQ
DGDLYRLKILDLQLSDSGQYVSRARNAIGEAFACVGLEVGAEA (SEQ ID NO:
82);
> O.23 (O.9 with mutations of A12S, F13Y, T22S, Q42L, A45T, A67Q, G69S, Q92E, and D94G, N-terminus + DQPQF) 
DQPQFSGAPRFLTRPLSYVVSVGKDASLSSQIVGNPTPQVSWEKDQLPVTAGAR
FRLAQDGDLYRLKILDLQLSDSGQYVSRARNAIGEAFACVGLEVGAEA (SEQ ID
NO: 85);
> O.20-L1 (O.9 with mutations of Q41K, A45T, A67Q, G69S, and V89L + (G₄S)₁ linker sequence) 
GGGGSSGAPRFLTRPLAFVVSVGKDATLSSQIVGNPTPQVSWEKDKQPVTAGAR
FRLAQDGDLYRLKILDLQLSDSGQYVSRARNAIGEAFACLGLQVDAEA (SEQ ID
NO: 83);
> O.22-L1 (O.9 with mutations of A12S, F13Y, T22S, Q42L, A45T, A67Q, G69S, Q92E, and D94G, + (G₄S)₁ linker sequence) 
GGGGSSGAPRFLTRPLSYVVSVGKDASLSSQIVGNPTPQVSWEKDQLPVTAGAR
FRLAQDGDLYRLKILDLQLSDSGQYVSRARNAIGEAFACVGLEVGAEA (SEQ ID
NO: 84);
> O.23-L1 (O.9 with mutations of A12S, F13Y, T22S, Q42L, A45T, A67Q, G69S, Q92E, and D94G, N-terminus + DQPQF; + (G₄S)₁ linker sequence) 
GGGGSDQPQFSGAPRFLTRPLSYVVSVGKDASLSSQIVGNPTPQVSWEKDQLPV
TAGARFRLAQDGDLYRLKILDLQLSDSGQYVSRARNAIGEAFACVGLEVGAEA
(SEQ ID NO: 86).
Note: In the above sequences, the double-underlined part indicates a linker sequence.

The antibodies in which CH1/CL is replaced were constructed by using the mutated or domain-engineered Titin-T chain and Obscurin-O chain, and the binding activity of the antibodies in which CH1/CL is replaced for the antigen was assayed by the method of Test Example 4 of the present disclosure. The experimental results are shown in Table 8. The results showed that the antibodies still retained good antigen-binding activity after the CH1/CL was replaced with the Titin-T chain/Obscurin-O chain.

**Table 8. Results for antigen binding assay on the antibodies**

| Antibody | | Antibody CH1/CL and peptides replacing same | | Corresponding antigen binding assay EC50 (nM) |
|---|---|---|---|---|
| | | CH1 | CL | |
| Series B0 | B0 | CH1 | CL | 0.2 |
| | B1 | T.1 | O.9 | 0.28 |
| | B2 | T.7 | O.17 | 0.19 |
| | B3 | T.8 | O.18 | 0.5 |
| | B4 | T.10 | O.20 | 0.25 |
| | B5 | T.11 | O.21 | 0.29 |
| | B6 | T.12 | O.21 | 0.19 |
| | B7 | T.13 | O.21 | 0.35 |
| | B8 | T.14 | O.22 | 0.16 |
| Series U0 | U0 | CH1 | CL | 0.06 |
| | U1 | T.1 | O.9 | 0.03 |
| | U2 | T.7 | O.17 | 0.07 |
| | U3 | T.8 | O.18 | 0.02 |
| | U4 | T.10 | O.20 | 0.06 |
| | U5 | T.11 | O.21 | 0.11 |
| | U6 | T.12 | O.21 | 0.11 |
| | U7 | T.13 | O.21 | 0.07 |
| | U8 | T.14 | O.22 | 0.16 |
| Series D0 | D0 | CH1 | CL | 0.089 |
| | D1 | T.1 | O.9 | 0.026 |
| | D2 | T.8 | O.18 | 0.056 |
| | D3 | T.10 | O.20 | 0.083 |
| | D4 | T.11 | O.21 | 0.103 |
| | D5 | T.12 | O.21 | 0.134 |
| | D6 | T.13 | O.21 | 0.132 |
| | D7 | T.14 | O.22 | 0.102 |
| Series I0 | I0 | CH1 | CL | 1.424 |
| | I2 | T.7 | O.17 | 3.195 |
| | I3 | T.10 | O.20 | 2.989 |
| | I4 | T.11 | O.21 | 4.132 |
| | I5 | T.12 | O.21 | 3.592 |
| | I6 | T.13 | O.21 | 4.121 |
| | I7 | T.14 | O.22 | 3.153 |

| | | | | |
|---|---|---|---|---|
| Note: In the table, for example, the antibody "B1" in the table indicates an antibody obtained by replacement of the heavy chain CH1 of the B0 antibody with the T.1 chain (SEQ ID NO: 35) and replacement of the light chain CL with the O.9 chain (SEQ ID NO: 50), with the other portions being the same as those of B0; and so on for others. | | | | |

In addition, the protein expression level of the domain-engineered antibodies was assayed by the method of Test Example 1 of the present disclosure. The experimental results are shown in Table 9. The results showed that the expression level of the antibodies was greatly improved by some amino acid mutations on the Titin-T chain/Obscurin-O chain.

**Table 9. Expression level of CH1/CL domain-engineered antibodies**

| Antibody | | Peptides replacing CH1/CL | | Expression level of antibody (mg/L) |
|---|---|---|---|---|
| | | CH1 | CL | |
| Series B0 | B1 | T.1 | O.9 | 9.4 |
| | B2 | T.7 | O.17 | 25.6 |
| | B3 | T.8 | O.18 | 13.9 |
| | B4 | T.10 | O.20 | 21.4 |
| Series D0 | D1 | T.1 | O.9 | 5.9 |
| | D2 | T.8 | O.18 | 44.4 |
| | D3 | T.10 | O.20 | 40.5 |
| | D4 | T.11 | O.21 | 17.8 |
| | D5 | T.12 | O.21 | 21.4 |
| | D6 | T.13 | O.21 | 23.3 |
| | D7 | T.14 | O.22 | 34.3 |
| Series I0 | I1 | T.1 | O.9 | 2.1 |
| | I2 | T.7 | O.17 | 11.0 |
| | I3 | T.10 | O.20 | 10.4 |
| | I4 | T.11 | O.21 | 4.0 |
| | I5 | T.12 | O.21 | 4.6 |
| | I6 | T.13 | O.21 | 8.2 |
| | I7 | T.14 | O.22 | 6.9 |

| | | | | |
|---|---|---|---|---|
| Note: In the table, for example, the antibody "B1" in the table indicates an antibody obtained by replacement of the heavy chain CH1 of the B0 antibody with the T.1 chain (SEQ ID NO: 35) and replacement of the light chain CL with the O.9 chain (SEQ ID NO: 50), with the other portions being the same as those of B0; and so on for others. | | | | |

### III. Novel Titin-T chain/Obscurin-O chain

### 1. Design for amino acid mutations on a Titin-T chain/Obscurin-O chain

The following mutations were made to the Titin-T chain/Obscurin-O chain on the basis of T. 10/O.20. The specific amino acid mutations are shown in Table 10:

**Table 10. Design for amino acid mutations on a Titin-T chain/Obscurin-O chain**

| Titin-T chain | | Obscurin-O chain | |
|---|---|---|---|
| Name | Mode of mutation | Name | Mode of mutation |
| T.16 | T.10+ (L60S, I64T) | O.24 | O20+ (L11K, T32F, A48V) |
| T.17 | T.10+ (V20C, L60S, I64T) | O.25 | O20+ (L11K, F13S, T32F, A48V, I82H) |
| T.18 | T.10+ (A26C, L60S, I64T) | O.26 | O20+ (A3C, L11K, F13S, T32F, A48V, I82H) |
| | | O.27 | O20+ (R9C, L11K, F13S, T32F, A48V, I82H) |
| | | O.28 | O20+ (S25C, S76C, L11K, F13S, T32F, A48V, I82H) |
| | | O.29 | O20+ (A3C, S25C, S76C, L11K, F13S, T32F, A48V, I82H) |
| | | O.30 | O20+ (R9C, S25C, S76C, L11K, F13S, T32F, A48V, I82H) |
| | | O.31 | O20+ (L66C, V93C, L11K, F13S, T32F, A48V, I82H) |
| | | O.32 | O20+ (A3C, L66C, V93C, L11K, F13S, T32F, A48V, I82H) |
| | | O.33 | O20+ (R9C, L66C, V93C, L11K, F13S, T32F, A48V, I82H) |

| | | | |
|---|---|---|---|
| Note: In the tables, for example, T.16 indicates a Titin-T chain formed by performing amino acid residue substitutions of L60S and I64T on T. 10 (SEQ ID NO: 68); and so on for others. | | | |

The sequences of the Titin-T chain/Obscurin-O chain after the amino acid residue substitutions are as follows:
> T. 16 (T. 10 with mutations of L60S and I64T) 
GIPPKIECLPIDISIDEGKVLTVASAFTGEPTPEVTWSTGGRKIHSQEQGRFHIENT
DDSTTLTIKDVQKQDGGLYTLTLRNEFGSDSATVNIHIRSI (SEQ ID NO: 129)
> T.17 (T.10 with mutations of V20C, L60S, and I64T) 
GIPPKIECLPIDISIDEGKCLTVASAFTGEPTPEVTWSTGGRKIHSQEQGRFHIENT
DDSTTLTIKDVQKQDGGLYTLTLRNEFGSDSATVNIHIRSI (SEQ ID NO: 130)
> T. 18 (T. 10 with mutations of A26C, L60S, and I64T) 
GIPPKIECLPIDISIDEGKVLTVASCFTGEPTPEVTWSTGGRKIHSQEQGRFHIENT
DDSTTLTIKDVQKQDGGLYTLTLRNEFGSDSATVNIHIRSI (SEQ ID NO: 131)
> O.24 (O.20 with mutations of L11K, T32F, and A48V) 
SGAPRFLTRPKAFVVSVGKDATLSSQIVGNPFPQVSWEKDKQPVTAGVRFRLAQ
DGDLYRLKILDLQLSDSGQYVSRARNAIGEAFACLGLQVDAEA (SEQ ID NO:
132)
> O.25 (O.20 with mutations of L11K, F13S, T32F, A48V, and I82H) 
SGAPRFLTRPKASVVSVGKDATLSSQIVGNPFPQVSWEKDKQPVTAGVRFRLAQ
DGDLYRLKILDLQLSDSGQYVSRARNAHGEAFACLGLQVDAEA (SEQ ID NO:
133)
> O.26 (O.20 with mutations of A3C, L11K, F13S, T32F, A48V, and I82H) 
SGCPRFLTRPKASVVSVGKDATLSSQIVGNPFPQVSWEKDKQPVTAGVRFRLAQ
DGDLYRLKILDLQLSDSGQYVSRARNAHGEAFACLGLQVDAEA (SEQ ID NO:
134)
> O.27 (O.20 with mutations of R9C, L11K, F13S, T32F, A48V, and I82H) 
SGAPRFLTCPKASVVSVGKDATLSSQIVGNPFPQVSWEKDKQPVTAGVRFRLAQ
DGDLYRLKILDLQLSDSGQYVSRARNAHGEAFACLGLQVDAEA (SEQ ID NO:
135)
> O.28 (O.20 with mutations of S25C, S76C, L11K, F13S, T32F, A48V, and I82H) 
SGAPRFLTRPKASVVSVGKDATLSCQIVGNPFPQVSWEKDKQPVTAGVRFRLAQ
DGDLYRLKILDLQLSDSGQYVCRARNAHGEAFACLGLQVDAEA (SEQ ID NO:
136)
O.29 (O.20 with mutations ofA3C, S25C, S76C, L11K, F13S, T32F, A48V, and I82H) 
SGCPRFLTRPKASVVSVGKDATLSCQIVGNPFPQVSWEKDKQPVTAGVRFRLAQ
DGDLYRLKILDLQLSDSGQYVCRARNAHGEAFACLGLQVDAEA (SEQ ID NO:
137)
> O.30 (O.20 with mutations of R9C, S25C, S76C, L11K, F13S, T32F, A48V, and I82H) 
SGAPRFLTCPKASVVSVGKDATLSCQIVGNPFPQVSWEKDKQPVTAGVRFRLAQ
DGDLYRLKILDLQLSDSGQYVCRARNAHGEAFACLGLQVDAEA (SEQ ID NO:
138)
> O.31 (O.20 with mutations of L66C, V93C, L11K, F13S, T32F, A48V, and I82H) 
SGAPRFLTRPKASVVSVGKDATLSSQIVGNPFPQVSWEKDKQPVTAGVRFRLAQ
DGDLYRLKILDCQLSDSGQYVSRARNAHGEAFACLGLQCDAEA (SEQ ID NO:
139)
> O.32 (O.20 with mutations of A3C, L66C, V93C, L11K, F13S, T32F, A48V, and I82H) 
SGCPRFLTRPKASVVSVGKDATLSSQIVGNPFPQVSWEKDKQPVTAGVRFRLAQ
DGDLYRLKILDCQLSDSGQYVSRARNAHGEAFACLGLQCDAEA (SEQ ID NO:
140)
> O.33 (O.20 with mutations of R9C, L66C, V93C, L11K, F13S, T32F, A48V, and I82H)

SGAPRFLTCPKASVVSVGKDATLSSQIVGNPFPQVSWEKDKQPVTAGVRFRLAQ
DGDLYRLKILDCQLSDSGQYVSRARNAHGEAFACLGLQCDAEA (SEQ ID NO:
141)

### 2. DI bispecific antibody constructed by Titin-T chain/Obscurin-O chain and assay

Construction of DI bispecific antibodies against hNGF and hRANKL: DI-2 to DI-20 comprising a first heavy chain, a second heavy chain, a first light chain, and a second light chain as described below:
the first heavy chain comprises [VH1]-[linker 1]-[Obscurin-O chain]-[linker 3]-[Fc1] in order from the N-terminus to the C-terminus,
the first light chain comprises [VL1]-[linker 2]-[Titin-T chain] in order from the N-terminus to the C-terminus,
the second heavy chain comprises [VH2]-[CH1]-[Fc2] in order from the N-terminus to the C-terminus, and
the second light chain comprises [VL2]-[CL] in order from the N-terminus to the C-terminus;
wherein VH1 and VL1 are heavy chain variable region and light chain variable region of 10, VH2 and VL2 are heavy chain variable region and light chain variable region of D0, VH1 has an amino acid sequence set forth in SEQ ID NO: 26, VL1 has an amino acid sequence set forth in SEQ ID NO: 27, VH2 has an amino acid sequence set forth in SEQ ID NO: 24, and VL2 has an amino acid sequence set forth in SEQ ID NO: 25; Fc1 (with an amino acid sequence set forth in SEQ ID NO: 177) is a first subunit of Fc of IgG1 containing a hole mutation, and Fc2 (with an amino acid sequence set forth in SEQ ID NO: 178) is a second subunit of Fc of IgG1 containing a knob mutation; CH1 has an amino acid sequence set forth in SEQ ID NO: 179, and CL has an amino acid sequence set forth in SEQ ID NO: 4; the N-terminus of the Obscurin-O chain is linked to VH1 via linker 1, the C terminus of the Obscurin-O chain is linked to Fc1 via a bond (that is, linker 3 is a bond), and the N-terminus of the Titin-T chain is linked to VL1 via linker 2. The structures of the Obscurin-O chain/Titin-T chain, linker 1, and linker 2 in different DI bispecific antibodies in this example are shown in Table 11.

**Table 11. Obscurin-O chain/Titin-T chain and linkers in DI bispecific antibodies**

| Bispecific antibody No. | First heavy chain | | First light chain | |
|---|---|---|---|---|
| | Obscurin-O chain | Linker 1 | Titin-T chain | Linker 2 |
| DI-2 | O.20 | GGGGS | T.10 | GGGGS |
| DI-3 | O.25 | GGGGS | T.16 | GGGGS |
| DI-4 | O.26 | GGGGS | T.17 | GGGGS |
| DI-5 | O.27 | GGGGS | T.18 | GGGGS |
| DI-6 | O.28 | GGGGS | T.16 | GGGGS |
| DI-7 | O.29 | GGGGS | T.17 | GGGGS |
| DI-8 | O.30 | GGGGS | T.18 | GGGGS |
| DI-9 | O.31 | GGGGS | T.16 | GGGGS |
| DI-10 | O.32 | GGGGS | T.17 | GGGGS |
| DI-11 | O.33 | GGGGS | T.18 | GGGGS |
| DI-12 | O.25 | ASTKG | T.16 | RTVAS |
| DI-13 | O.26 | ASTKG | T.17 | RTVAS |
| DI-14 | O.27 | ASTKG | T.18 | RTVAS |
| DI-15 | O.28 | ASTKG | T.16 | RTVAS |
| DI-16 | O.29 | ASTKG | T.17 | RTVAS |
| DI-17 | O.30 | ASTKG | T.18 | RTVAS |
| DI-18 | O.31 | ASTKG | T.16 | RTVAS |
| DI-19 | O.32 | ASTKG | T.17 | RTVAS |
| DI-20 | O.33 | ASTKG | T.18 | RTVAS |

The binding activity of the bispecific antibodies DI-2 to DI-20 against their antigens was assayed by the method of Test Example 4 of the present disclosure. Moreover, the thermal stability test was performed on DI-2, DI-4 to DI-8, DI-10 to DI-16, and DI-20 by the following process: the antibodies were each diluted to a concentration of 5 mg/mL with a PBS solution and tested for thermal stability with Unit (loading volume: 9 µL; parameter settings: Start Temp: 20 °C; Incubation: 0 s; Rate: 0.3 °C/min; Plate Hold: 5 s; End Temp: 95 °C). The experimental results are shown in Tables 12 and 13. The experimental results showed that the binding activity of the modified bispecific antibodies against the antigen was not significantly changed; meanwhile, DI-4 to DI-8, DI-10 to DI-16, and DI-20 had significantly improved Tm1 (°C) and Tonset (°C) as compared to DI-2, and therefore, the bispecific antibodies had better thermal stability.

**Table 12. Binding activity assay on DI bispecific antibodies**

| Bispecific antibody No. | RANKL EC50(nM) | NGF EC50(nM) |
|---|---|---|
| DI-2 | 0.3832 | 6.633 |
| DI-3 | 0.3613 | 5.7730 |
| DI-4 | 0.3959 | 6.2930 |
| DI-5 | 0.3290 | 6.1890 |
| DI-6 | 0.2509 | 5.6720 |
| DI-7 | 0.2557 | 6.6430 |
| DI-8 | 0.3643 | 7.6250 |
| DI-9 | 0.2944 | 8.4950 |
| DI-10 | 0.3460 | 7.1660 |
| DI-11 | 0.3721 | 10.9600 |
| DI-12 | 0.4125 | 6.5156 |
| DI-13 | 0.4440 | 5.5420 |
| DI-14 | 0.4182 | 3.2610 |
| DI-15 | 0.2206 | 5.2800 |
| DI-16 | 0.1474 | 5.5140 |
| DI-17 | 0.2329 | 6.7270 |
| DI-18 | 0.2662 | 5.9080 |
| DI-19 | 0.1843 | 5.9280 |
| DI-20 | 0.3184 | 6.4250 |

**Table 13. Thermal stability test results for DI bispecific antibodies**

| Bispecific antibody No. | Tm1 (°C) | Tonset (°C) |
|---|---|---|
| DI-2 | 55.6 | 48.3 |
| DI-4 | 60.1 | 52.493 |
| DI-5 | 61 | 51.967 |
| DI-6 | 60.8 | 53.012 |
| DI-7 | 60.34 | 52.003 |
| DI-8 | 60.61 | 50.425 |
| DI-10 | 60.2 | 52.766 |
| DI-11 | 57.35 | - |
| DI-12 | 59.9 | 51.726 |
| DI-13 | 61 | 50.988 |
| DI-14 | 61.2 | 52.191 |
| DI-15 | 60.41 | 50.558 |
| DI-16 | 61.5 | 50.691 |
| DI-20 | 60.7 | 51.859 |

In addition, solutions of the DI bispecific antibodies were formulated with a buffer containing 10 mM acetic acid at pH 5.5 and 9% sucrose (see Table 14 for details), and the solutions were incubated in an incubator at 40 °C for four weeks. After the end of the incubation at the fourth week, the bispecific antibodies were concentrated to the concentration at the start of the incubation, and the precipitation of the solutions was observed. The experimental results are shown in Table 14 below. The experimental results showed that there was precipitation in the solution of DI-2 bispecific antibody group, and therefore, DI-3 to DI-7 had better stability than DI-2.

**Table 14. Precipitation of solutions of DI bispecific antibodies**

| Group | Bispecific antibody No. | Initial concentration | Concentration to be achieved at week 4 | Precipitation of solutions |
|---|---|---|---|---|
| 1 | DI-2 | 20 mg/ml | 20 mg/ml | Precipitation occurred |
| 2 | DI-3 | 20 mg/ml | 20 mg/ml | No precipitation |
| 3 | DI-4 | 60 mg/ml | 60 mg/ml | No precipitation |
| 4 | DI-5 | 25 mg/ml | 25 mg/ml | No precipitation |
| 5 | DI-6 | 60 mg/ml | 60 mg/ml | No precipitation |
| 6 | DI-7 | 16 mg/ml | 16 mg/ml | No precipitation |

### 3. PL bispecific antibody constructed by Titin-T chain/Obscurin-O chain and assay

Construction of PL bispecific antibodies against hPDL1 and hCTLA4: PL-1 to PL-19 comprising a first heavy chain, a second heavy chain, a first light chain, and a second light chain as described below:
the first heavy chain comprises [VH1]-[linker 1]-[Obscurin-O chain]-[linker 3]-[Fc1] in order from the N-terminus to the C-terminus,
the first light chain comprises [VL1]-[linker 2]-[Titin-T chain] in order from the N-terminus to the C-terminus,
the second heavy chain comprises [VH2]-[CH1]-[Fc2] in order from the N-terminus to the C-terminus, and
the second light chain comprises [VL2]-[CL] in order from the N-terminus to the C-terminus;
wherein VH1 and VL1 are heavy chain variable region and light chain variable region of P0 antibody (i.e., h1831K antibody in WO2020177733A1), VH2 and VL2 are heavy chain variable region and light chain variable region of L0 antibody, VH1 has an amino acid sequence set forth in SEQ ID NO: 156, VL1 has an amino acid sequence set forth in SEQ ID NO: 155, VH2 has an amino acid sequence set forth in SEQ ID NO: 169, and VL2 has an amino acid sequence set forth in SEQ ID NO: 170; Fc1 (with an amino acid sequence set forth in SEQ ID NO: 178) is a first subunit of Fc of IgG1 containing a knob mutation, and Fc2 (with an amino acid sequence set forth in SEQ ID NO: 177) is a second subunit of Fc of IgG1 containing a hole mutation; CH1 has an amino acid sequence set forth in SEQ ID NO: 179, and CL has an amino acid sequence set forth in SEQ ID NO: 4; the N-terminus of the Obscurin-O chain is linked to VH1 via linker 1, the C terminus of the Obscurin-O chain is directly linked to Fc1 via a bond (that is, linker 3 is a bond), and the N-terminus of the Titin-T chain is linked to VL1 via linker 2. The structures of the Obscurin-O chain/Titin-T chain, linker 1, and linker 2 in different PL bispecific antibodies are shown in Table 15.

**Table 15. Obscurin-O chain/Titin-T chain and linkers in PL bispecific antibodies**

| Bispecific antibody No. | First heavy chain | | First light chain | |
|---|---|---|---|---|
| | Obscurin-O chain | Linker 1 | Titin-T chain | Linker 2 |
| PL-1 | O.20 | GGGGS | T.10 | GGGGS |
| PL-2 | O.25 | GGGGS | T.16 | GGGGS |
| PL-3 | O.26 | GGGGS | T.17 | GGGGS |
| PL-4 | O.27 | GGGGS | T.18 | GGGGS |
| PL-5 | O.28 | GGGGS | T.16 | GGGGS |
| PL-6 | O.29 | GGGGS | T.17 | GGGGS |
| PL-7 | O.30 | GGGGS | T.18 | GGGGS |
| PL-8 | O.31 | GGGGS | T.16 | GGGGS |
| PL-9 | O.32 | GGGGS | T.17 | GGGGS |
| PL-10 | O.33 | GGGGS | T.18 | GGGGS |
| PL-11 | O.25 | ASTKG | T.16 | RTVAS |
| PL-12 | O.26 | ASTKG | T.17 | RTVAS |
| PL-13 | O.27 | ASTKG | T.18 | RTVAS |
| PL-14 | O.28 | ASTKG | T.16 | RTVAS |
| PL-15 | O.29 | ASTKG | T.17 | RTVAS |
| PL-16 | O.30 | ASTKG | T.18 | RTVAS |
| PL-17 | O.31 | ASTKG | T.16 | RTVAS |
| PL-18 | O.32 | ASTKG | T.17 | RTVAS |
| PL-19 | O.33 | ASTKG | T.18 | RTVAS |

The heavy chain variable region and the light chain variable region of the anti-CTLA-4 antibody Ipilimumab (abbreviated as L0, trade name: Yervoy) are as follows:
Heavy chain variable region of L0: 
QVQLVESGGGVVQPGRSLRLSCAASGFTFSSYTMHWVRQAPGKGLEWVTFISY
DGNNKYYADSVKGRFTISRDNSKNTLYLQMNSLRAEDTAIYYCARTGWLGPFD
YWGQGTLVTVSS (SEQ ID NO: 169)
Light chain variable region of L0:

EIVLTQSPGTLSLSPGERATLSCRASQSVGSSYLAWYQQKPGQAPRLLIYGAFSR
ATGIPDRFSGSGSGTDFTLTISRLEPEDFAVYYCQQYGSSPWTFGQGTKVEIK
(SEQ ID NO: 170)

Illustratively, the sequences of four chains of PL-1 are shown as follows:
Amino acid sequence of a first heavy chain of PL-1: 
QVQLVQSGAEVKKPGASVKVSCKASGYTFTSYWMHWVRQAPGQGLEWM
GRITPSSGFAMYNEKFKNRVTMTRDTSTSTVYMELSSLRSEDTAVYYCARG
GSSYDYFDYWGQGTTVTVSSGGGGSSGAPRFLTRPLAFVVSVGKDATLSSQIV
GNPTPQVSWEKDKQPVTAGARFRLAQDGDLYRLKILDLQLSDSGQYVSRARNA
IGEAFACLGLQVDAEADKTHTCPPCPAPEAAGGPSVFLFPPKPKDTLMISRTPEV
TCVVVDVSHEDPEVKFNWYVDGVEVHNAKTKPREEQYNSTYRVVSVLTVLHQ
 
DWLNGKEYKCKVSNKALPAPIEKTISKAKGQPREPQVYTLPPCREEMTKNQVSL
WCLVKGFYPSDIAVEWESNGQPENNYKTTPPVLDSDGSFFLYSKLTVDKSRWQQ
GNVFSCSVMHEALHNHYTQKSLSLSPGK (SEQ ID NO: 142)
Amino acid sequence of a first light chain of PL-1: 
DIVLTQSPASLAVSPGQRATITCRASESVSIHGTHLMHWYQQKPGQPPKLLI
YAASKLESGVPARFSGSGSGTDFTLTINPVEAEDTANYYCQQSFEDPLTFGQ
GTKLEIKGGGGSGIPPKIECLPIDISIDEGKVLTVASAFTGEPTPEVTWSTGGRKI
HSQEQGRFHIENTDDLTTLIIKDVQKQDGGLYTLTLRNEFGSDSATVNIHIRSI
(SEQ ID NO: 143)
Amino acid sequence of a second heavy chain of PL-1: 
QVQLVESGGGVVQPGRSLRLSCAASGFTFSSYTMHWVRQAPGKGLEWVT
FISYDGNNKYYADSVKGRFTISRDNSKNTLYLQMNSLRAEDTAIYYCARTG
WLGPFDYWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPS
NTKVDKKVEPKSCDKTHTCPPCPAPEAAGGPSVFLFPPKPKDTLMISRTPEVTCV
VVDVSHEDPEVKFNWYVDGVEVHNAKTKPREEQYNSTYRVVSVLTVLHQDW
LNGKEYKCKVSNKALPAPIEKTISKAKGQPREPQVCTLPPSREEMTKNQVSLSC
AVKGFYPSDIAVEWESNGQPENNYKTTPPVLDSDGSFFLVSKLTVDKSRWQQGN
VFSCSVMHEALHNHYTQKSLSLSPGK (SEQ ID NO: 144)
Amino acid sequence of a second light chain of PL-1:

EIVLTQSPGTLSLSPGERATLSCRASQSVGSSYLAWYQQKPGQAPRLLIYGA
FSRATGIPDRFSGSGSGTDFTLTISRLEPEDFAVYYCQQYGSSPWTFGQGTK
VEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNR
GEC (SEQ ID NO: 145)

Note: In the above sequences, the bold part indicates the variable region, the dotted underlined part indicates the Fc portion, the wavy line part indicates CH1, the dashed underlined portion indicates CL, the double underlined part indicates the linker, and the single underlined part indicates the Obscurin-O chain/Titin-T chain.

The binding activity of PL bispecific antibodies was tested by the ELISA assay of Test Example 4 of the present disclosure (wherein hPDL1 and hCTLA4 antigens were purchased from Sino biology). In addition, the thermal stability test was performed on the PL bispecific antibodies using a high throughput differential scanning fluorimetry (UNCHAINED, Specification: Unit) by the following process: 9 µL, of each of the samples (PL bispecific antibody concentration: 1.4-3 mg/mL) diluted in a PBS (phosphate buffered saline) solution was loaded into the sample wells, with parameter settings as follows: Start Temp: 25 °C, Incubation: 180s, Rate: 0.3 °C/min, Plate Hold: 3s, End Temp: 95 °C, and the instrument was run. The experimental results were analyzed using the Uncle Analysis software.

The experimental results are shown in Tables 16 and 17. The experimental result showed that the PL bispecific antibodies constructed by the novel Titin-T chain/Obscurin-O chain still had good binding activity to the antigen; meanwhile, PL-2 to PL-19 had significantly improved Tm1 (°C), Tagg 266 (°C), and Tonset (°C) as compared to PL-1, and therefore, the bispecific antibodies had better thermal stability.

**Table 16. Results for antigen binding assay on the PL bispecific antibodies**

| Bispecific antibody No. | EC50 (nM) for binding to hPDL1 antigen | EC50 (nM) for binding to hCTLA4 antigen |
|---|---|---|
| PL-1 | 1.6 | 16.5 |
| PL-2 | 0.5 | 8.0 |
| PL-3 | 0.6 | 7.9 |
| PL-4 | 0.7 | 6.5 |
| PL-5 | 0.7 | 6.7 |
| PL-6 | 0.8 | 5.3 |
| PL-7 | 0.5 | 7.3 |
| PL-8 | 1.4 | 14.5 |
| PL-9 | 0.6 | 5.5 |
| PL-10 | 0.6 | 6.9 |
| PL-11 | 0.6 | 5.6 |
| PL-12 | 0.5 | 8.0 |
| PL-13 | 0.5 | 4.4 |
| PL-14 | 1.3 | 6.0 |
| PL-15 | 1.4 | 3.2 |
| PL-17 | 1.4 | 6.2 |
| PL-18 | 1.6 | 6.7 |
| PL-19 | 1.6 | 6.5 |

**Table 17. Thermal stability test results for PL bispecific antibodies**

| Bispecific antibody No. | Tm1 (°C) | Tagg 266 (°C) | Tonset (°C) |
|---|---|---|---|
| PL-1 | 61.64 | 64.82 | 52.566 |
| PL-2 | 66.20 | 66.55 | 58.317 |
| PL-3 | 64.07 | 68.28 | 57.661 |
| PL-4 | 70.71 | 67.29 | 56.246 |
| PL-5 | 74.56 | 68.11 | 58.407 |
| PL-6 | 70.43 | 70.12 | 61.069 |
| PL-7 | 68.46 | 67.41 | 63.031 |
| PL-8 | 65.00 | - | - |
| PL-9 | 69.24 | 67.83 | 58.597 |
| PL-10 | 69.63 | 68.12 | 56.788 |
| PL-11 | 65.88 | - | 57.976 |
| PL-12 | 65.54 | 67.94 | - |
| PL-13 | 71.85 | 68.17 | 58.581 |
| PL-14 | 74.18 | 69.42 | 58.589 |
| PL-15 | 70.96 | 69.91 | 58.622 |
| PL-16 | 63.48 | 68.98 | 58.702 |
| PL-17 | 70.15 | 69.86 | 56.193 |
| PL-18 | - | 69.43 | - |
| PL-19 | - | 69.52 | 57.766 |

### 4. HJ bispecific antibody constructed by Titin-T chain/Obscurin-O chain and assay

Construction of HJ bispecific antibodies against hIL5 and hTSLP: HJ-3 to HJ11 comprising a first heavy chain, a second heavy chain, a first light chain, and a second light chain as described below:
the first heavy chain comprises [VH1]-[linker 1]-[Titin-T chain]-[linker 3]-[Fc1] in order from the N-terminus to the C-terminus;
the first light chain comprises [VL1]-[linker 2]-[Obscurin-0 chain] in order from the N-terminus to the C-terminus;
the second heavy chain comprises [VH2]-[CH1]-[Fc2] in order from the N-terminus to the C-terminus; and
the second light chain comprises [VL2]-[CL] in order from the N-terminus to the C-terminus;
wherein VH1 and VL1 are heavy chain variable region and light chain variable region of H0 antibody, VH2 and VL2 are heavy chain variable region and light chain variable region of J1 antibody, VH1 has an amino acid sequence set forth in SEQ ID NO: 16, VL1 has an amino acid sequence set forth in SEQ ID NO: 17, VH2 has an amino acid sequence set forth in SEQ ID NO: 171, and VL2 has an amino acid sequence set forth in SEQ ID NO: 172; Fc1 (with an amino acid sequence set forth in SEQ ID NO: 178) is a first subunit of Fc of IgG1 containing a knob mutation, and Fc2 (with an amino acid sequence set forth in SEQ ID NO: 177) is a second subunit of Fc of IgG1 containing a hole mutation; CH1 has an amino acid sequence set forth in SEQ ID NO: 179, and CL has an amino acid sequence set forth in SEQ ID NO: 4; the N-terminus of the Titin-T chain is linked to VH1 via linker 1, the C terminus of the Titin-T chain is directly linked to Fc1 via a bond (that is, linker 3 is a bond), and the N-terminus of the Obscurin-O is linked to VL1 via linker 2. The structures of the Titin-T chain/Obscurin-O chain, linker 1, and linker 2 in different HJ bispecific antibodies are shown in Table 18.

**Table 18. Obscurin-O chain/Titin-T chain and linkers in HJ bispecific antibodies**

| Bispecific antibody No. | First heavy chain | | First light chain | |
|---|---|---|---|---|
| | Titin-T chain | Linker 1 | Obscurin-O chain | Linker 2 |
| HJ-3 | T. 10 | GGGGS | O.20 | GGGGS |
| HJ-5 | T.16 | GGGGS | O.25 | GGGGS |
| HJ-6 | T.16 | GGGGS | O.27 | GGGGS |
| HJ-7 | T.16 | GGGGS | O.28 | GGGGS |
| HJ-8 | T.16 | ASTKG | O.25 | RTVAS |
| HJ-9 | T.16 | ASTKG | O.27 | RTVAS |
| HJ-10 | T.16 | ASTKG | O.28 | RTVAS |
| HJ-11 | T.16 | ASTKG | O.29 | RTVAS |

The binding activity of the bispecific antibodies HJ-3, and HJ-5 to HJ-11 against hIL5 and hTSLP antigens was assayed by the method of Test Example 4 of the present disclosure. Moreover, the thermal stability test was performed on the bispecific antibodies by the following process: the dilutions of the HJ bispecific antibodies were formulated with a buffer containing 10 mM acetic acid at pH 5.5 and 9% sucrose; the bispecific antibodies were concentrated by ultrafiltration and concentration to obtain different concentrations of solutions (the HJ bispecific antibody concentrations are shown in Table 19-2) of the HJ bispecific antibodies; the concentrated solutions were incubated in an incubator at 40 °C, and the samples were tested for SEC purity on day 0 (i.e., before the start of incubation at 40 °C, D0), day 7 (the 7th day of incubation at 40 °C, D7), day 14 (the 14th day of incubation at 40 °C, D14), day 21 (the 21th day of incubation at 40 °C, D21), and day 28 (the 28th day of incubation at 40 °C, D28); after 28 days of incubation at 40 °C, the samples were immediately taken for the determination of non-reduced CE-SDS purity.

The experimental results are shown in Tables 19-1 and 19-2 below. The experimental results showed that the binding activity of the HJ bispecific antibodies constructed in the present disclosure against the antigen was not significantly changed; meanwhile, the HJ-5 to HJ-11 bispecific antibodies had better thermal stability than HJ-3.

**Table 19-1. Binding activity assay on HJ bispecific antibodies**

| Bispecific antibody No. | hIL5 | hTSLP | Bispecific antibody No. | hIL5 | hTSLP |
|---|---|---|---|---|---|
| | EC50(nM) | | | EC50(nM) | |
| HJ-3 | 17.38 | 3.234 | HJ-8 | 18.74 | 3.332 |
| HJ-5 | 15.96 | 2.639 | HJ-9 | 10.86 | 3.184 |
| HJ-6 | 37.05 | 2.884 | HJ-10 | 20.81 | 3.173 |
| HJ-7 | 17.69 | 2.182 | HJ-11 | 9.464 | 3.005 |

**Table 19-2. Accelerated stability test results for HJ bispecific antibodies**

| Bispecific antibody No. | D0 concentration (mg/mL) | D0 SEC purity (%) | D28 SEC purity (%) | D28 Δ SEC purity (%) | D28 non-reduced CE-SDS purity (%) |
|---|---|---|---|---|---|
| HJ-3 | 50 | 98 | 84 | 14 | 67 |
| HJ-5 | 60.8 | 98.09 | 93.86 | 4.23 | 82.93 |
| HJ-6 | 54 | 98.14 | 89.68 | 8.46 | 80.76 |
| HJ-7 | 56.8 | 97.7 | 92.72 | 4.98 | 85.47 |
| HJ-8 | 62.6 | 93.39 | 84.28 | 9.11 | 73.03 |
| HJ-9 | 53.5 | 90.01 | 85.93 | 4.08 | 80.32 |
| HJ-10 | 69.8 | 90.9 | 88.31 | 2.59 | 80.4 |
| HJ-11 | 50.4 | 92.55 | 90.89 | 1.66 | 82.96 |

| | | | | | |
|---|---|---|---|---|---|
| Note: D28 Δ SEC purity (%) = D0 SEC purity (%) - D28 SEC purity (%) | | | | | |

### Example 4. Half-Life of CH1/CL Domain-Engineered Antibody

To evaluate whether the stability of the antibodies in animals was affected after CH1/CL of the antibodies was replaced with a Titin-T chain/Obscurin-O chain or a Titin-T chain/Obscurin-like-O chain, the half-life of the antibodies in rats before and after the replacement of CH1/CL was determined. Illustratively, the half-lives in rats of the antibody F20 in which CH1/CL of F0 antibody was replaced with T.1/O.9, and the antibody C3 in which CH1/CL of C0 antibody was replaced with T.1/O.9 were determined. The specific procedures were as follows:
The antibody at 3 mpk was administered intravenously to rats, and 0.3 mL of whole blood was collected 5 min, 8 h, 1 d, 2 d, 4 d, 7 d, 10 d, 14 d, 21 d and 28 d after administration, without anticoagulation. After blood collection, the blood was left to stand at 4 °C for 30 min and centrifuged at 1000 g for 15 min, and the supernatant (serum) was placed in an EP tube and stored at -80 °C. The assay method for antibody drug concentrations at each time point was sandwich ELISA. Specifically, an ELISA plate was coated with corresponding antigens, incubated at 4 °C overnight, and then washed with a washing buffer; a blocking buffer was added, and the plate was incubated at room temperature for 1-3 h and then washed with a washing buffer; 100 µL of standard or test serum sample was added, and the plate was incubated at 37 °C for 3 h and then washed with a washing buffer; a secondary antibody anti-Human IgG FC (HRP) mouse preadsorbed (Abcam, Cat. No. ab98624, 1:10000 dilution) was added, and the plate was incubated at 37 °C for 1-1.5 h and then washed with a buffer; TMB was added, and the plate was incubated at room temperature for 10 min in the dark; a stop buffer was added, and OD450 values were read.

The experimental results are shown in Table 20. The results showed that after CH1/CL of the antibody was replaced with a Titin-T chain/Obscurin-O chain, the antibody still retained the same half-life as that of the original antibody in the rat, indicating that the antibody after CH1/CL was replaced had good stability *in vivo* as the original antibody.

**Table 20. Half-life of antibodies in which CH1/CL domain is replaced**

| Antibody name | *In vivo* half-life of rat (3 mpk) T1/2 (day) |
|---|---|
| F0 | 13.3 ± 1.0 |
| F20 | 12.8 ± 0.3 |
| C0 | 17.3 ± 2.2 |
| C3 | 15.4 ± 4.1 |

### Example 5: Construction and Detection of Bispecific Antibody

An antibody binding to a first antigen and an antibody binding to a second antigen A were used to construct bispecific antibodies by over lapextension PCR. Illustratively, a variety of bispecific antibodies were constructed.

### I. Construction of bispecific antibody

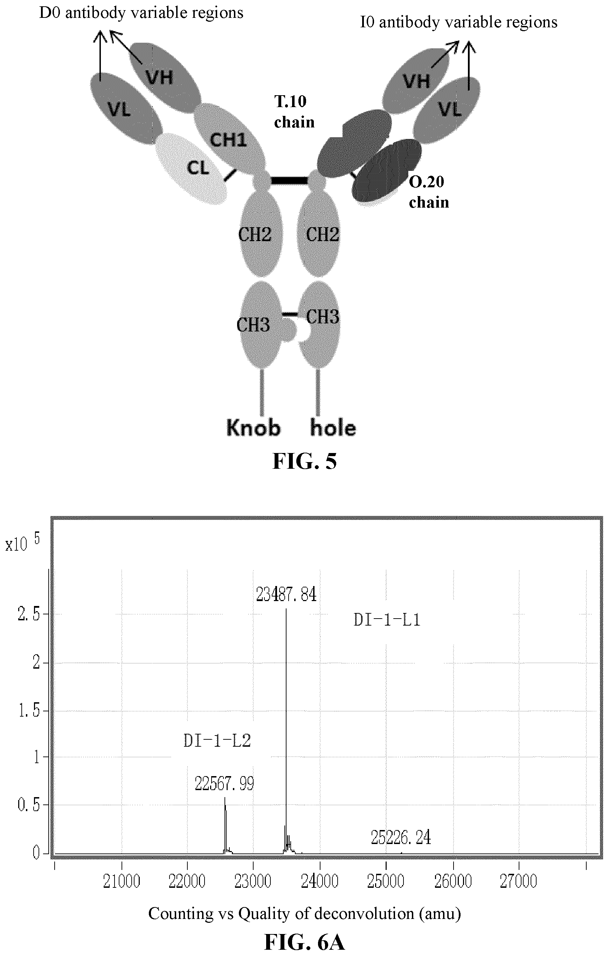

Firstly, a DI-1 bispecific antibody comprising one heavy chain (with a knob modification in the Fc region) and one light chain of a D0 antibody, and one heavy chain and one light chain of a domain-engineered 10 antibody (CH1/CL is replaced with a Titin-T chain/Obscurin-O chain, and the Fc region comprises a hole modification) was constructed. The DI-1 bispecific antibody is an IgG-like bispecific antibody, the schematic structural diagram thereof is shown in FIG. 5, and the sequences of 4 chains of DI-1 are shown as follows:
Amino acid sequence of DI-1 chain 1 (DI-1-H1): 
EVQLLESGGGLVQPGGSLRLSCAASGFTFSSYAMSWVRQAPGKGLEWVSG
ITGSGGSTYYADSVKGRFTISRDNSKNTLYLQMNSLRAEDTAVYYCAKDPG
TTVIMSWFDPWGQGTLVTVSSASTKGPSVFPLAPSSKSTSGGTAALGCLVKDY
FPEPVTVSWNSGALTSGVHTFPAVLQSSGLYSLSSVVTVPSSSLGTQTYICNVNH
KPSNTKVDKKVEPKSCDKTHTCPPCPAPEAAGGPSVFLFPPKPKDTLMISRTPEV
TCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPCREEMTKNQVSL
WCLVKGFYPSDIAVEWESNGQPENNYKTTPPVLDSDGSFFLYSKLTVDKSRWQQGN
VFSCSVMHEALHNHYTQKSLSLSPGK (SEQ ID NO: 87).
 Note: In the sequence, the bold part indicates VH of D0, the single underlined part indicates CH1, the dotted underlined part indicates CH2, and the italic part indicates CH3.
Amino acid sequence of DI-1 chain 2 (DI-1-L1): 
EIVLTQSPGTLSLSPGERATLSCRASQSVRGRYLAWYQQKPGQAPRLLIYGA
SSRATGIPDRFSGSGSGTDFTLTISRLEPEDFAVFYCQQYGSSPRTFGQGTKV
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
 EC (SEQ ID NO: 88). Note: In the sequence, the bold part indicates VL of D0, and the single underlined part indicates the light chain constant region CL of D0.
Amino acid sequence of DI-1 chain 3 (DI-1-H2): 
QVQLQESGPGLVKPSETLSLTCTVSGFSLIGYDLNWIRQPPGKGLEWIGIIW
GDGTTDYNSAVKSRVTISKDTSKNQFSLKLSSVTAADTAVYYCARGGYWYA
TSYYFDYWGQGTLVTVSSGGGGSGIPPKIECLPIDISIDEGKVLTVASAFTGEPTP
EVTWSTGGRKIHSQEQGRFHIENTDDLTTLIIKDVQKQDGGLYTLTLRNEFGSDS
ATVNIHIRSIDKTHTCPPCPAPEAAGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVCTLPPSREEMTKNQVSLSCAVKGFYP
SDIAVEWESNGQPENNYKTTPPVLDSDGSFFLVSKLTVDKSRWQQGNVFSCSVMHE
ALHNHYTQKSLSLSPGK (SEQ ID NO: 89).
 Note: In the sequence, the bold part indicates VH of 10, the single underlined part indicates T.10, the double underlined part indicates the linker sequence, the dotted line part indicates CH2, the italic part indicates CH3, and the double underlined part indicates the linker sequence.
Amino acid sequence of DI-1 chain 4 (DI-1-L2): 
DIQMTQSPSSLSASVGDRVTITCRASQSISNNLNWYQQKPGKAPKLLIYYTS
RFHSGVPSRFSGSGSGTDFTFTISSLQPEDIATYYCQQEHTLPYTFGQGTKL
EIKGGGGSSGAPRFLTRPLAFVVSVGKDATLSSQIVGNPTPQVSWEKDKQPVTA
GARFRLAQDGDLYRLKILDLQLSDSGQYVSRARNAIGEAFACLGLQVDAEA
(SEQ ID NO: 90).
 Note: In the sequence, the bold part indicates VL of 10, the single underlined part indicates O.20, and the double underlined part indicates the linker sequence.

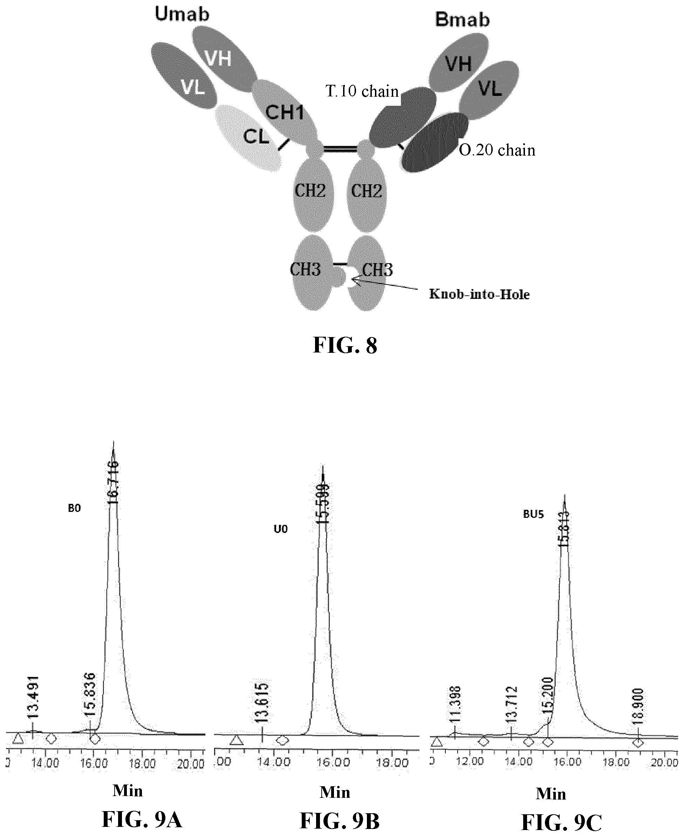

In addition, IgG-like BU bispecific antibodies were constructed from B0 or the domain-engineered antibody of B0 and U0 or the domain-engineered antibody of U0 (illustratively, the schematic structural diagram of BU5 is shown in FIG. 8). The sequences of four chains of the obtained bispecific antibodies are shown in Table 21 below:

**Table 21. Sequence listing of IgG-like BU bispecific antibodies**

| Bispecific antibody No. | Sequences of four chains of IgG-like bispecific antibodies | | | |
|---|---|---|---|---|
| | Chain 1 | Chain 2 | Chain 3 | Chain 4 |
| BU1 | Heavy chain of B0 (knob) | Light chain of B0 | Heavy chain of U0 (hole), in which CH1 is replaced with T.14-L1 | Light chain of U0, in which CL is replaced with O.22-L1 |
| | QVQLQQSGAEV KKPGSSVRVSCK ASGGTFNNNAIN | SSELTQDPAVSVA LGQTVRVTCQG DSLRSYYASWYQ | EVQLVQSGAEVK KPGESLKISCKG SGYSFTTYWLG | DIQMTQSPSSLSA SVGDRVTITCRA SQGISSWLAWYQ |
| | WVRQAPGQGLE WMGGIIPMFGT AKYSQNFQGRVA ITADESTGTASM ELSSLRSEDTAVY YCARSRDLLLFP HHALSPWGRGT MVTVSSASTKGP SVFPLAPSSKSTS GGTAALGCLVKD YFPEPVTVSWNS GALTSGVHTFPAV LQSSGLYSLSSVV TVPSSSLGTQTYI CNVNHKPSNTKV DKKVEPKSCDKT HTCPPCPAPEAAG GPSVFLFPPKPKD TLMISRTPEVTCV VVDVSHEDPEVK FNWYVDGVEVH NAKTKPREEQYN STYRVVSVLTVLH QDWLNGKEYKC KVSNKALPAPIEK TISKAKGQPREPQ VYTLPPCREEMTK NQVSLWCLVKGFY PSDIAVEWESNGQ PENNYKTTPPVLD SDGSFFLYSKLTVD KSRWQQGNVFSCS VMHEALHNHYTQ KSLSLSPGK (SEQ ID NO: 91) | QKPGQAPVLVIY GKNNRPSGIPDR FSGSSSGNTASLT ITGAQAEDEADY YCSSRDSSGNHW VFGGGTELTVLG QPKAAPSVTLFPP SSEELQANKATLV CLISDFYPGAVTV AWKADSSPVKAG VETTTPSKQSNNK YAASSYLSLTPEQ WKSHRSYSCQVT HEGSTVEKTVAPT ECS (SEQ ID NO: 92) | WVRQMPGKGL DWIGIMSPVDSD IRYSPSFQGQVT MSVDKSITTAYL QWNSLKASDTA MYYCARRRPGQ GYFDFWGQGTL VTVSSGGGGSGIP PKIECLPSDISIDE GKVLTVASAFTGE PTPEVTWSTGGR KIHSQEQGRFHIE STEDLTTLIISDVQ KQDGGLYSLSLG NEFGSDSATVNIH IRSIDKTHTCPPCP APEAAGGPSVFLF PPKPKDTLMISRT PEVTCVVVDVSH EDPEVKFNWYVD GVEVHNAKTKPR EEQYNSTYRVVS VLTVLHQDWLNG KEYKCKVSNKAL PAPIEKTISKAKG QPREPQVCTLPPS REEMTKNQVSLSC AVKGFYPSDIAVE WESNGQPENNYKT TPPVLDSDGSFFLV SKLTVDKSRWQQG NVFSCSVMHEALH NHYTQKSLSLSPG K (SEQ ID NO: 93) | QKPEKAPKSLIY AASSLQSGVPSR FSGSGSGTDFTL TISSLQPEDFATY YCQQYNIYPYTF GQGTKLEIKGGG GSSGAPRFLTRPL SYVVSVGKDASL SSQIVGNPTPQVS WEKDQLPVTAGA RFRLAQDGDLYR LKILDLQLSDSGQ YVSRARNAIGEAF ACVGLEVGAEA (SEQ ID NO: 94) |
| BU2 | Heavy chain of U0 (knob) | Light chain of U0 | Heavy chain of B0 (hole), in which CH1 is replaced with T.14-L1 | Light chain of B0, in which CL is replaced with O.23-L1 |
| | EVQLVQSGAEVK KPGESLKISCKG SGYSFTTYWLG WVRQMPGKGL DWIGIMSPVDSD IRYSPSFQGQVT MSVDKSITTAYL QWNSLKASDTA MYYCARRRPGQ GYFDFWGQGTL VTVSSASTKGPSV FPLAPSSKSTSGG TAALGCLVKDYF PEPVTVSWNSGA LTSGVHTFPAVLQ SSGLYSLSSVVTV PSSSLGTQTYICN VNHKPSNTKVDK KVEPKSCDKTHT CPPCPAPEAAGGP SVFLFPPKPKDTL | DIQMTQSPSSLS ASVGDRVTITCR ASQGISSWLAWY QQKPEKAPKSLI YAASSLQSGVPS RFSGSGSGTDFT LTISSLQPEDFAT YYCQQYNIYPYT FGQGTKLEIKRT VAAPSVFIFPPSDE QLKSGTASVVCLL NNFYPREAKVQW KVDNALQSGNSQ ESVTEQDSKDSTY SLSSTLTLSKADY EKHKVYACEVTH QGLSSPVTKSFNR GEC (SEQ ID NO: 96) | QVQLQQSGAEV KKPGSSVRVSCK ASGGTFNNNAIN WVRQAPGQGLE WMGGIIPMFGT AKYSQNFQGRVA ITADESTGTASM ELSSLRSEDTAVY YCARSRDLLLFP HHALSPWGRGT MVTVSSGGGGSG IPPKIECLPSDISID EGKVLTVASAFTG EPTPEVTWSTGG RKIHSQEQGRFHI ESTEDLTTLIISDV QKQDGGLYSLSL GNEFGSDSATVNI HIRSIDKTHTCPPC PAPEAAGGPSVFL FPPKPKDTLMISR | SSELTQDPAVSVA LGQTVRVTCQG DSLRSYYASWYQ QKPGQAPVLVIY GKNNRPSGIPDR FSGSSSGNTASLT ITGAQAEDEADY YCSSRDSSGNHW VFGGGTELTVLG GGGSDQPQFSGA PRFLTRPLSYVVS VGKDASLSSQIVG NPTPQVSWEKDQ LPVTAGARFRLA QDGDLYRLKILDL QLSDSGQYVSRA RNAIGEAFACVGL EVGAEA (SEQ ID NO: 98) |
| | MISRTPEVTCVVV DVSHEDPEVKFN WYVDGVEVHNA KTKPREEQYNST YRVVSVLTVLHQ DWLNGKEYKCK VSNKALPAPIEKTI SKAKGQPREPQV YTLPPCREEMTKN QVSLWCLVKGFYP SDIAVEWESNGQP ENNYKTTPPVLDS DGSFFLYSKLTVD KSRWQQGNVFSCS VMHEALHNHYTQ KSLSLSPGK (SEQ ID NO: 95) | | TPEVTCVVVDVS HEDPEVKFNWYV DGVEVHNAKTKP REEQYNSTYRVV SVLTVLHQDWLN GKEYKCKVSNKA LPAPIEKTISKAKG QPREPQVCTLPPS REEMTKNQVSLSC AVKGFYPSDIAVE WESNGQPENNYKT TPPVLDSDGSFFLV SKLTVDKSRWQQG NVFSCSVMHEALH NHYTQKSLSLSPG K (SEQ ID NO: 97) | |
| BU3 | Heavy chain of B0 (knob), in which CH1 is replaced with T.14-L1 | Light chain of B0, in which CL is replaced with O.23-L1 | Heavy chain of U0 (hole) | Light chain of U0 |
| | QVQLQQSGAEV KKPGSSVRVSCK ASGGTFNNNAIN WVRQAPGQGLE WMGGIIPMFGT AKYSQNFQGRVA ITADESTGTASM ELSSLRSEDTAVY YCARSRDLLLFP HHALSPWGRGT MVTVSSGGGGSG IPPKIECLPSDISID EGKVLTVASAFTG EPTPEVTWSTGG RKIHSQEQGRFHI ESTEDLTTLIISDV QKQDGGLYSLSL GNEFGSDSATVNI HIRSIDKTHTCPPC PAPEAAGGPSVFL FPPKPKDTLMISR TPEVTCVVVDVS HEDPEVKFNWYV DGVEVHNAKTKP REEQYNSTYRVV SVLTVLHQDWLN GKEYKCKVSNKA LPAPIEKTISKAKG QPREPQVYTLPPC REEMTKNQVSLWC LVKGFYPSDIAVEW ESNGQPENNYKTT PPVLDSDGSFFLYS KLTVDKSRWQQG NVFSCSVMHEALH NHYTQKSLSLSPG K (SEQ ID NO: 99) | SSELTQDPAVSVA LGQTVRVTCQG DSLRSYYASWYQ QKPGQAPVLVIY GKNNRPSGIPDR FSGSSSGNTASLT ITGAQAEDEADY YCSSRDSSGNHW VFGGGTELTVLG GGGSDQPQFSGA PRFLTRPLSYVVS VGKDASLSSQIVG NPTPQVSWEKDQ LPVTAGARFRLA QDGDLYRLKILDL QLSDSGQYVSRA RNAIGEAFACVGL EVGAEA (SEQ ID NO: 100) | EVQLVQSGAEVK KPGESLKISCKG SGYSFTTYWLG WVRQMPGKGL DWIGIMSPVDSD IRYSPSFQGQVT MSVDKSITTAYL QWNSLKASDTA MYYCARRRPGQ GYFDFWGQGTL VTVSSASTKGPSV FPLAPSSKSTSGG TAALGCLVKDYF PEPVTVSWNSGA LTSGVHTFPAVLQ SSGLYSLSSVVTV PSSSLGTQTYICN VNHKPSNTKVDK KVEPKSCDKTHT CPPCPAPEAAGGP SVFLFPPKPKDTL MISRTPEVTCVVV DVSHEDPEVKFN WYVDGVEVHNA KTKPREEQYNST YRVVSVLTVLHQ DWLNGKEYKCK VSNKALPAPIEKTI SKAKGQPREPQV CTLPPSREEMTKN QVSLSCAVKGFYPS DIAVEWESNGQPE NNYKTTPPVLDSD GSFFLVSKLTVDKS RWQQGNVFSCSV MHEALHNHYTQKS LSLSPGK (SEQ ID NO: 101) | DIQMTQSPSSLSA SVGDRVTITCRA SQGISSWLAWYQ QKPEKAPKSLIY AASSLQSGVPSR FSGSGSGTDFTL TISSLQPEDFATY YCQQYNIYPYTF GQGTKLEIKRTV AAPSVFIFPPSDEQ LKSGTASVVCLLN NFYPREAKVQWK VDNALQSGNSQE SVTEQDSKDSTYS LSSTLTLSKADYE KHKVYACEVTHQ GLSSPVTKSFNRG EC (SEQ ID NO: 96) |
| BU4 | Heavy chain of U0 (knob) | Light chain of U0 | Heavy chain of B0 (hole), in which CH1 is replaced with T.15-L1 | Light chain of B0, in which CL is replaced with O.23-L1 |
| | EVQLVQSGAEVK KPGESLKISCKG SGYSFTTYWLG WVRQMPGKGL DWIGIMSPVDSD IRYSPSFQGQVT MSVDKSITTAYL QWNSLKASDTA MYYCARRRPGQ GYFDFWGQGTL VTVSSASTKGPSV FPLAPSSKSTSGG TAALGCLVKDYF PEPVTVSWNSGA LTSGVHTFPAVLQ SSGLYSLSSVVTV PSSSLGTQTYICN VNHKPSNTKVDK KVEPKSCDKTHT CPPCPAEAAGGP SVFLFPPKPKDTL MISRTPEVTCVVV DVSHEDPEVKFN WYVDGVEVHNA KTKPREEQYNST YRVVSVLTVLHQ DWLNGKEYKCK VSNKALPAPIEKTI SKAKGQPREPQV YTLPPCREEMTKN QVSLWCLVKGFYP SDIAVEWESNGQP ENNYKTTPPVLDS DGSFFLYSKLTVD KSRWQQGNVFSCS VMHEALHNHYTQ KSLSLSPGK (SEQ ID NO: 95) | DIQMTQSPSSLS ASVGDRVTITCR ASQGISSWLAWY QQKPEKAPKSLI YAASSLQSGVPS RFSGSGSGTDFT LTISSLQPEDFAT YYCQQYNIYPYT FGQGTKLEIKRT VAAPSVFIFPPSDE QLKSGTASVVCLL NNFYPREAKVQW KVDNALQSGNSQ ESVTEQDSKDSTY SLSSTLTLSKADY EKHKVYACEVTH QGLSSPVTKSFNR GEC (SEQ ID NO: 96) | QVQLQQSGAEV KKPGSSVRVSCK ASGGTFNNNAIN WVRQAPGQGLE WMGGIIPMFGT AKYSQNFQGRVA ITADESTGTASM ELSSLRSEDTAVY YCARSRDLLLFP HHALSPWGRGT MVTVSSGGGGSK AGIRGIPPKIECLP SDISIDEGKVLTVA SAFTGEPTPEVTW STGGRKIHSQEQG RFHIESTEDLTTLII SDVQKQDGGLYS LSLGNEFGSDSAT VNIHIRSIDKTHTC PPCPAPEAAGGPS VFLFPPKPKDTLM ISRTPEVTCVVVD VSHEDPEVKFNW YVDGVEVHNAKT KPREEQYNSTYR VVSVLTVLHQDW LNGKEYKCKVSN KALPAPIEKTISKA KGQPREPQVCTLP PSREEMTKNQVSL SCAVKGFYPSDIAV EWESNGQPENNYK TTPPVLDSDGSFFL VSKLTVDKSRWQQ GNVFSCSVMHEAL HNHYTQKSLSLSP GK (SEQ ID NO: 102) | SSELTQDPAVSVA LGQTVRVTCQG DSLRSYYASWYQ QKPGQAPVLVIY GKNNRPSGIPDR FSGSSSGNTASLT ITGAQAEDEADY YCSSRDSSGNHW VFGGGTELTVLG GGGSDQPQFSGA PRFLTRPLSYVVS VGKDASLSSQIVG NPTPQVSWEKDQ LPVTAGARFRLA QDGDLYRLKILDL QLSDSGQYVSRA RNAIGEAFACVGL EVGAEA (SEQ ID NO: 100) |
| BU5 | Heavy chain of B0 (hole), in which CH1 replaced with T.14-L1 | Light chain of B0, in which CL is replaced with O.20-L1 | Heavy chain of U0 (knob) | Light chain of U0 |
| | QVQLQQSGAEV KKPGSSVRVSCK ASGGTFNNNAIN WVRQAPGQGLE WMGGIIPMFGT AKYSQNFQGRVA ITADESTGTASM ELSSLRSEDTAVY YCARSRDLLLFP HHALSPWGRGT MVTVSSGGGGS GIPPKIECLPIDISI DEGKVLTVASAFT GEPTPEVTWSTG GRKIHSQEQGRFH | SSELTQDPAVSVA LGQTVRVTCQG DSLRSYYASWYQ QKPGQAPVLVIY GKNNRPSGIPDR FSGSSSGNTASLT ITGAQAEDEADY YCSSRDSSGNHW VFGGGTELTVLG GGGSSGAPRFLTR PLAFVVSVGKDA TLSSQIVGNPTPQ VSWEKDKQPVTA GARFRLAQDGDL YRLKILDLQLSDS | EVQLVQSGAEVK KPGESLKISCKG SGYSFTTYWLG WVRQMPGKGL DWIGIMSPVDSD IRYSPSFQGQVT MSVDKSITTAYL QWNSLKASDTA MYYCARRRPGQ GYFDFWGQGTL VTVSSASTKGPSV FPLAPSSKSTSGG TAALGCLVKDYF PEPVTVSWNSGA LTSGVHTFPAVLQ | DIQMTQSPSSLSA SVGDRVTITCRA SQGISSWLAWYQ QKPEKAPKSLIY AASSLQSGVPSR FSGSGSGTDFTL TISSLQPEDFATY YCQQYNIYPYTF GQGTKLEIKRTV AAPSVFIFPPSDEQ LKSGTASVVCLLN NFYPREAKVQWK VDNALQSGNSQE SVTEQDSKDSTYS LSSTLTLSKADYE |
| | IENTDDLTTLIIKD VQKQDGGLYTLT LRNEFGSDSATVN IHIRSIDKTHTCPP CPAPEAAGGPSVF LFPPKPKDTLMIS RTPEVTCVVVDV SHEDPEVKFNWY VDGVEVHNAKTK PREEQYNSTYRV VSVLTVLHQDWL NGKEYKCKVSNK ALPAPIEKTISKAK GQPREPQVCTLPP SREEMTKNQVSLS CAVKGFYPSDIAVE WESNGQPENNYKT TPPVLDSDGSFFLV SKLTVDKSRWQQG NVFSCSVMHEALH NHYTQKSLSLSPG K (SEQ ID NO: 103) | GQYVSRARNAIG EAFACLGLQVDA EA (SEQ ID NO: 104) | SSGLYSLSSVVTV PSSSLGTQTYICN VNHKPSNTKVDK KVEPKSCDKTHT CPPCPAPEAAGGP SVFLFPPKPKDTL MISRTPEVTCVVV DVSHEDPEVKFN WYVDGVEVHNA KTKPREEQYNST YRVVSVLTVLHQ DWLNGKEYKCK VSNKALPAPIEKTI SKAKGQPREPQV YTLPPCREEMTKN QVSLWCLVKGFYP SDIAVEWESNGQP ENNYKTTPPVLDS DGSFFLYSKLTVD KSRWQQGNVFSCS VMHEALHNHYTQ KSLSLSPGK (SEQ ID NO: 95) | KHKVYACEVTHQ GLSSPVTKSFNRG EC (SEQ ID NO: 96) |

| | | | | |
|---|---|---|---|---|
| Note: In the sequence, the bold part indicates the variable region sequence, the single underlined part indicates CH1 or Titin-T chain, the dotted underlined part indicates CH2, the italic part indicates CH3, the double underlined part indicates CL or Obscurin-O chain, and the dashed underlined part indicates the linker L1. | | | | |

In addition, an FA-1 bispecific antibody was constructed. The FA-1 bispecific antibody is an IgG-like FA-1 bispecific antibody composed of a heavy chain (a knob modification in the Fc region) and a light chain of A0 antibody, and a heavy chain (a hole modification in the Fc region, replacement of CH1 with T.10-L1) and a light chain (replacement of CL with O.20-L1) of an F0 domain-engineered antibody. The sequences of four chains of FA-1 are shown in Table 22 below.

Chain 1 of FA-1 (heavy chain CH1 of F0 is replaced with T.10-L1 and the Fc region comprises a knob modification):

QVQLVQSGGGVVQPGTSLRLSCAASGFIFSSSAMHWVRQAPGKGLEWVAVI
SYDGSNKYYVDSVKGRFTISRDNSKNTLYLQMNSLRAEDTAVYYCARSARL
YASFDYWGQGALVTVSSGGGGSGIPPKIECLPIDISIDEGKVLTVASAFTGEPTPE
VTWSTGGRKIHSQEQGRFHIENTDDLTTLIIKDVQKQDGGLYTLTLRNEFGSDSA
TVNIHIRSIDKTHTCPPCPAPEAAGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIEKTISKAKGQPREPQVCTLPPSREEMTKNQVSLSCAVKGFYP
SDIAVEWESNGQPENNYKTTPPVLDSDGSFFLVSKLTVDKSRWQQGNVFSCSVMHE
ALHNHYTQKSLSLSPGK (SEQ ID NO: 107)

Chain 2 of FA-1 (light chain CL of F0 is replaced with O.20-L1):

DTVVTQEPSFSVSPGGTVTLTCGLSSGSVSTSHYPSWYQQTPGQAPRMLIYN
TNTRSSGVPDRFSGSILGNKAALTITGAQADDESDYYCAIHVDRDIWVFGGG
TKLTVLGGGGSSGAPRFLTRPLAFVVSVGKDATLSSQIVGNPTPQVSWEKDKQP

VTAGARFRLAQDGDLYRLKILDLQLSDSGQYVSRARNAIGEAFACLGLQVDAE
A (SEQ ID NO: 108)

Chain 3 of FA-1 (heavy chain of A0 comprises a knob modification):

EVQLVESGGGLVQPGGSLKLSCAASGFTFNKYAMNWVRQAPGKGLEWVA
RIRSKYNNYATYYADSVKDRFTISRDDSKNTAYLQMNNLKTEDTAVYYCVR
HGNFGNEYISYWAYWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYIC
NVNHKPSNTKVDKKVEPKSCDKTHTCPPCPAPEAAGGPSVFLFPPKPKDTLMIS
RTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPCREEMTK
NQVSLWCLVKGFYPSDIAVEWESNGQPENNYKTTPPVLDSDGSFFLYSKLTVDKSRW
QQGNVFSCSVMHEALHNHYTQKSLSLSPGK (SEQ ID NO: 109)

Chain 4 of FA-1 (light chain of A0): 
QTVVTQEPSLTVSPGGTVTLTCGSSTGAVTSGNYPNWVQQKPGQAPRGLIG
GTKFLAPGTPARFSGSLLGGKAALTLSGVQPEDEAEYYCVLWYSNRWVFG
GGTKLTVLRTVAAPSVFIFPPSDEQLKSGTASVVCLLNNFYPREAKVQWKVDN
ALQSGNSQESVTEQDSKDSTYSLSSTLTLSKADYEKHKVYACEVTHQGLSSPVT
KSFNRGEC (SEQ ID NO: 110)
 Note: In the above sequences, the bold part indicates the variable region sequence, the single underlined part indicates CH1 or Titin-T chain, the dotted underlined part indicates CH2, the italic part indicates CH3, the double underlined part indicates CL or Obscurin-O chain, and the dashed underlined part indicates the linker L1.

### II. Detection of bispecific antibody

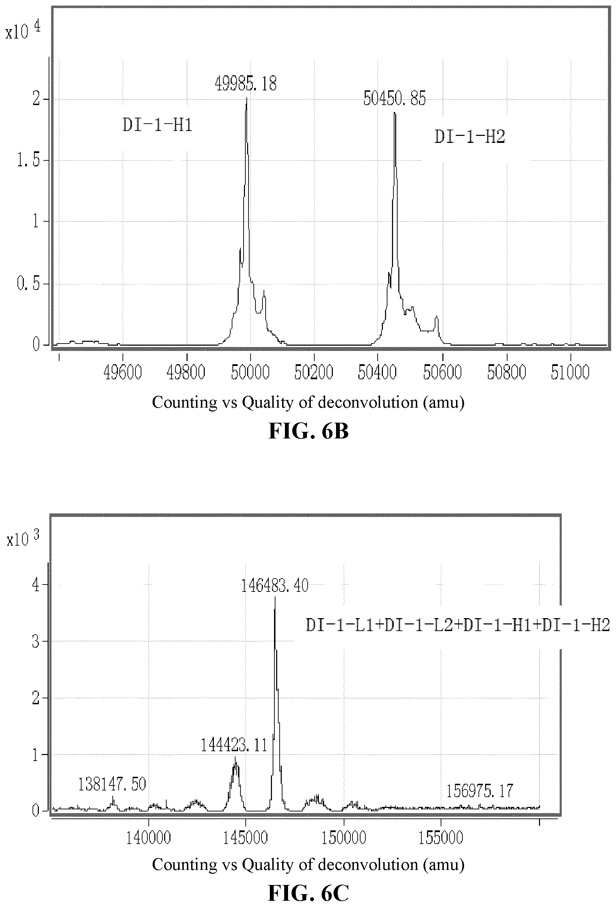

DI-1 was detected by the method of Test Example 3 of the present disclosure, and four chains of DI-1 were co-transfected into cells for expression and then subjected to mass spectrometry. The experimental results are shown in Table 22 and FIGs. 6A-6C. The mass spectrometry results showed that no homodimers and mispaired molecules were detected, that is, DI-1 was correctly assembled.

**Table 22. Mass spectrometry data for DI-1**

| DI-1 chain | Chain 1 (DI-1-H1) | Chain 2 (DI-1-L1) | Chain 3 (DI-1-H2) | Chain 4 (DI-1-L2) | DI-1-L1+DI-1-L2+ DI-1-H1+DI-1-H2 | MS results |
|---|---|---|---|---|---|---|
| Molecular weight | 49983.82 | 23487.38 | 50450.05 | 22567.45 | 146488.70 | No homodimers and mispaired molecules were detected |

In addition, the expression of BU5 was detected by the methods of Test Example 3 and Test Example 2 of the present disclosure. Firstly, the four chains of BU5 were co-transfected into cells for expression of the bispecific antibody, with B0 and U0 as controls. Then, the expression product was subjected to purity determination and mass spectrometry. The experimental results are shown in Table 23 and FIGs. 9A-9C. The experimental results showed that the bispecific antibody BU5 was successfully expressed, and four chains of BU5 were successfully assembled into the target molecule without mispairing. The bispecific antibody BU5 had a high purity, with SEC% of 88%.

**Table 23. Mass spectrometry results for BU5**

| Antibody name | Mass spectrometry |
|---|---|
| Control B0 | ✔ |
| Control U0 | ✔ |
| BU5 | ✔ |

| | |
|---|---|
| Note: In the table, "✔" indicates correct pairing. | |

In addition, the constructed BU1-BU4 bispecific antibodies were detected for purity and antigen binding activity by the methods of Test Example 2 and Test Example 4 of the present disclosure. The experimental results are shown in Table 24. The experimental results showed that the bispecific antibody in which CH1/CL was substituted still retained good antigen binding activity and had high antibody purity.

**Table 24. Detection results for antigen binding activity and purity of antibodies**

| Antibody name | Purity of antibody SEC (%) | Antigen binding EC50 (nM) | |
|---|---|---|---|
| | | Antigen BAFF | Antigen P40 |
| B0 | 99.05 | 0.2193 | - |
| U0 | 99.63 | - | 0.08881 |
| BU1 | 95.23 | 0.3561 | 0.08397 |
| BU2 | 91.09 | 0.4693 | 0.05674 |
| BU3 | 83.45 | 0.4131 | 0.2238 |
| BU4 | 91.85 | 0.5057 | 0.04429 |

### Test Examples

### Test Example 1: Determination Method for Expression Level of Antibody

HEK293E cells were transfected with expression plasmids of monoclonal antibodies or bispecific antibodies, and after 6 days, expression supernatants were collected and centrifuged at high speed to remove impurities. The supernatants were each purified using a Protein A column (GE Healthcare). The column was washed with PBS until the A280 reading dropped to a baseline, and the column was washed with a 100 mM acetate buffer (pH 3.5) and neutralized with 1 M Tris-HCl, pH 8.0. The expression level of the antibody was quantified from the amount of the antibody/expression volume obtained by the final purification.

### Test Example 2: Detection Method for Purity of Antibody

The antibody purity was monitored using SEC-HPLC. Detection was performed according to the instrument operating instructions using a Waters e2695 chromatograph, wherein Waters Xbridge BEH 200A SEC column was applied, PBS was taken as a mobile phase (pH was adjusted to 6.8 with dilute hydrochloric acid), 100 µg of protein was injected, and gradient elution was performed, with a flow rate of 0.5 mL/min. The purity of antibody was indicated as the percentage of the peak area of the main peak to the total peak area (SEC (%)).

### Test Example 3: Detection Method for Antibody by Mass Spectrometry

(I) Sample preparation:
1. Determination of deglycosylated intact molecular weight: 30 µg of the expressed antibody was taken and lyophilized before adding 10 µL of 8 M Gua-HCl, and the mixture was denatured in a water bath at 70 °C for 10 min. 90 µL of distilled water was added, from which 30 µL was taken, 0.8 µL of PNG enzyme F (peptide N-glycosidase F) was added, and the mixture was incubated in a water bath at 37 °C for 2 h. 0.5 µg of the product was taken for the determination of deglycosylated intact molecular weight.
2. Determination of deglycosylated reduced molecular weight: 30 µg of the expressed antibody was taken and lyophilized before adding 10 µL of 8 M Gua-HCl, and the mixture was denatured in a water bath at 70 °C for 10 min. 90 µL of distilled water was added, from which 30 µL was taken, 0.8 µL of PNG enzyme F (peptide N-glycosidase F) was added, and the mixture was incubated in a water bath at 37 °C for 2 h. Then, 2 µL of 0.025 M DTT was added, and the mixture was reduced for 10 min in a water bath at 70 °C. 0.5 µg of the product was taken for the determination of deglycosylated reduced molecular weight.

(II) Chromatography-mass spectrometry conditions:
Chromatography conditions:
Chromatographic column: Poroshell 300SB-C8 5 µm 2.1 × 75 mm
Mobile phase: A: 0.1% HCOOH/H₂O B: 0.1% HCOOH/CAN
Column temperature: 75 °C
Gradient: see Table 25.

**Table 25. Chromatography gradient**

| | | | | | | |
|---|---|---|---|---|---|---|
| Time (min) | 0 | 5 | 8 | 12 | 12.1 | 15 |
| B% | 5 | 5 | 95 | 95 | 5 | 5 |

Mass spectrometry conditions:
Mass spectrometry: Agilent 6530 Q-TOF LC-MS
Ionization mode ESI
Acquisition mode: 1 GHz (mass range: 500-5000 m/z)
Temperature of drying gas: 325 °C
Flow rate of drying gas: 10 L/min
Atomizer: 40 psi
Temperature of sheath gas: 350 °C
Flow rate of sheath gas: 12 L/min
Spray voltage: 500 V
Capillary voltage: 3,500 V
Cleavage voltage: 200 V
Skimmer voltage: 65 V
Rf voltage: 7500 V

The test antibody was subjected to mass spectrometry. The experimental results showed that the antibody remained correctly assembled after CH1/CL was replaced with Titin-T/Ob scurin-O.

### Test Example 4: Detection Method for Antibody Binding Activity

The binding activity of an antibody against a membrane protein target to an antigen may be detected by FACS. For example, C0, N0, F0, V0, and S0, and CH1/CL domain-engineered antibodies thereof may be detected by FACS. Cells (2×10⁶ cells/mL, 90 µL) resuspended in a FACS buffer (98% PBS, 2% FBS) were added to a 96-well U-shaped bottom plate (corning, 3795), and 10 µL of the antibody diluted in a gradient was added. The mixture was incubated at 4 °C for 1 h and washed twice with a FACS buffer, and then Alexa Fluor 488 goat anti-human IgG (H+L) (invitrogen, Cat # 2015982, 1:1000 dilution) was added to each well. The mixture was incubated at 4 °C for 1 h and washed twice, and then the cells were resuspended in a FACS buffer. Finally, fluorescence signal values were read using FACS CantoII (BD). Finally, the data were processed and analyzed using FlowJo 7.6 and Graphpad Prism 5.

For an antibody targeting a soluble protein, the binding activity of the antibody against the soluble protein was detected by ELISA. For example, D0, 10, B0, U0, H0, R0, and J0, and CH1/CL domain-engineered antibodies thereof may be detected by ELISA. The specific procedures were as follows: the protein was diluted to 1 µg/mL with a PBS buffer at pH 7.4 (BasalMedia, B320), added to a 96-well microplate (Corning, 9018) at 100 µL/well, and incubated at 4 °C overnight. After the liquid was discarded, 300 µL of 5% skim milk (BD, 232100) diluted with PBS was added to each well for blocking, and the mixture was incubated at 37 °C for 2 h. After the blocking was completed, the blocking solution was discarded; and the plate was washed 3 times with a PBST buffer (pH 7.4, PBS containing 0.1% tween-20). 100 µL of the antibody solution diluted in a gradient was added to each well, and the mixture was incubated at 37 °C for 1 h. After the incubation was completed, the plate was washed 3 times with PBST. 100 µL of mouse anti-human IgG (H+L) (Jackson ImmunoResearch, 209-035-088, 1:8000 dilution) was added to each well, and the mixture was incubated at 37 °C for 1 h. The plate was washed 3 times with PBST. 100 µL of TMB chromogenic substrate (KPL, 5120-0077) was added to each well, and the plate was incubated at room temperature for 10-15 min. The reaction was stopped by adding 50 µL of 1 M H₂SO₄ to each well. The absorbance values at 450 nm were read on a microplate reader, the binding curves of the antibodies to an antigen were fitted with software, and the EC₅₀ values were calculated.

The antigens corresponding to the antibodies in this test example were as follows: the antigen binding to D0 and a CH1/CL domain-engineered antibody thereof was hRANKL (available from Sino biological, 11682-HNCH); the antigen binding to 10 and a CH1/CL domain-engineered antibody thereof was hNGF (available from Sino biological, 11050-HNAC); the antigen binding to B0 and a CH1/CL domain-engineered antibody thereof was hBAFF (available from Sino biological, 10056-HNCH); the antigen binding to U0 and a CH1/CL domain-engineered antibody thereof was hP40 (available from Sino biological, 10052-H08H); the antigen binding to H0 and R0 and CH1/CL domain-engineered antibodies thereof was hEL-5 (available from R&D systems, 205-II,-025/CF); the antigen binding to J0 and a CH1/CL domain-engineered antibody thereof was hTSLP (available from Sino biological, 16135-H08H); the antigen binding to C0 and N0 and CH1/CL domain-engineered antibodies thereof was CEA (cells MKN45 overexpressing CEA (available from Nanjing Cobioer, CBP60488)); the antigen binding to F0, V0 and S0 and CH1/CL domain-engineered antibodies thereof was B7H3 (a recombinant cell line CT26-B7H3 overexpressing B7H3 (CT26 was derived from the Chinese Academy of Cell Bank, and the sequence of B7H3 was under ID of XP_005254757.1)).

### Test Example 5: Detection of Mispairing of CH1/CL Domain-Engineered Antibody I. Detection method for expression of light/heavy chain cross-mispaired molecules of different antibodies

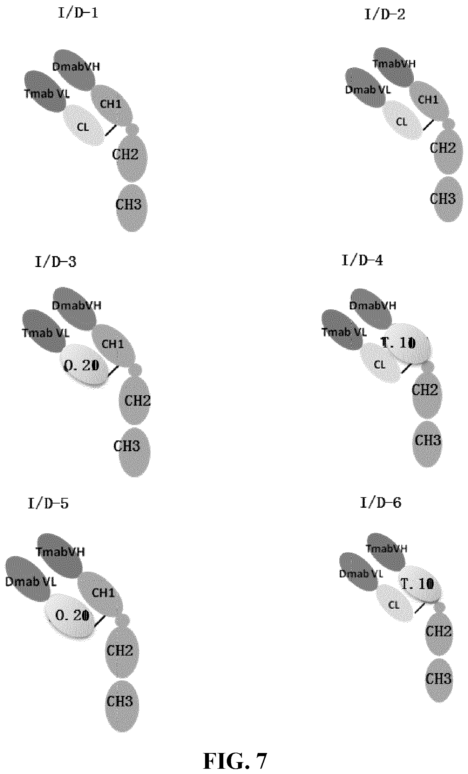

In this experiment, the expression of light/heavy chain pairings of different antibodies was analyzed to verify that replacement of CH1/CL with a Titin-T chain/Obscurin-O chain or a Titin-T chain/Obscurin-like-O chain could effectively reduce the heavy/light chain mispairings. Specifically, when the antibodies were expressed by the transfected cells, heavy/light chains of D0 and I0 were respectively exchanged (i.e., I/D-1 (heavy chain of D0 + light chain of 10); I/D-2 (heavy chain of 10 + light chain of D0)) to determine whether cross-mispaired molecules would be formed from wild-type CH1 and CL; meanwhile, heavy and light chains of domain-engineered antibody D3 in which CH1/CL of D0 was replaced with the Titin-T chain/Obscurin-O chain (heavy chain CH1 of D0 was replaced with T.10, and light chain CL was replaced with O.20) and domain-engineered antibody 13 in which CH1/CL of 10 was replaced with the Titin-T chain/Obscurin-O chain (heavy chain CH1 of 10 was replaced with T.10, and light chain CL was replaced with O.20) were respectively exchanged to construct mispaired molecules: I/D-3 (heavy chain of D0 + light chain of 13), I/D-4 (heavy chain of D3 + light chain of I0), I/D-5 (heavy chain of I0 + light chain of 13) and I/D-6 (heavy chain of 13 + light chain of D0). The schematic structural diagrams of the constructed antibodies are shown in FIG. 7.

The expression level of the antibodies was determined by the method of Test Example 1, and the expression products were subjected to mass spectrometry by the method of Test Example 3 to determine whether mispaired molecules would be formed. The experimental results are shown in Table 26. The experimental results showed that when the heavy chain of D0 + the light chain of 10 or the heavy chain of 10 + the light chain of D0 were co-expressed, intact IgG molecules (150 kDa) could be formed, with high expression levels of 74.4 mg/L and 73.8 mg/L, respectively; and LC-MS results showed that the obtained proteins were mismatched IgG molecules, indicating that if CH1-CL was not engineered or replaced, a large amount of light/heavy chain mispaired molecules could be formed when four different heavy and light chains of the bispecific antibodies were co-expressed. After CH1/CL was substituted with the Titin-T chain/Obscurin-O chain, no target molecules (150 kDa) were produced in four mispairing expression forms of I/D-3 (heavy chain of D0 + light chain of 13), I/D-4 (heavy chain of D3 + light chain of 10), I/D-5 (heavy chain of 10 + light chain of 13), and I/D-6 (heavy chain of 13 + light chain of D0), and with the exception of the combination of the heavy chain of D3 and the light chain of 10, which formed a small amount of error molecules (the molecular weight was 100 kDa) of two heavy chains, no mispaired molecules were produced in the other three forms, indicating that engineered non-homologous pairings could not form mispaired molecules in an IgG structural form. Thus, the replacement of CH1-CL with the Titin-T chain/Obscurin-O chain would effectively avoid or reduce the formation of non-homologous cross-paired molecules between the light/heavy chains of IgG-like bispecific antibodies.

**Table 26. Expression level and mass spectrometry of different light/heavy chain cross-paired antibodies**

| Name of mispairing | Heavy chain | Light chain | Expression level of antibody (mg/L) | Mass spectrometry |
|---|---|---|---|---|
| I/D-1 | Heavy chain of D0 | Light chain of I0 | 74.4 | √ |
| I/D-2 | Heavy chain of I0 | Light chain of D0 | 73.8 | √ |
| I/D-3 | Heavy chain of D0 | Light chain of I3 | 0.04 | × |
| I/D-4 | Heavy chain of D3 | Light chain of I0 | 12.75 | Only heavy chain, without light chain |
| I/D-5 | Heavy chain of I0 | Light chain of D3 | 0.04 | × |
| I/D-6 | Heavy chain of I3 | Light chain of D0 | 0.25 | × |

| | | | | |
|---|---|---|---|---|
| Note: In the table, "√" indicates that the mispaired molecule was detected, and "×" indicates that the mispaired molecule was not detected. | | | | |

### II. Detection method for expression of light/heavy chain mispairings of bispecific antibodies

In this example, to verify that replacement of CH1/CL with a Titin-T chain/Obscurin-O chain would result in no mispairing or reduce mispairing, three light/heavy chain cross-paired expression forms were designed. Specifically, two different heavy chains and one identical VL-CL light chain were co-transfected into cells for antibody expression, or two different heavy chains and one identical light chain in which CL was replaced with the Obscurin-O chain were co-transfected into cells for antibody expression, or two different heavy chains and two different light chains were co-transfected into cells for antibody expression (see Table 27 for specific pairings).

The expression product was then subjected to mass spectrometry (see Test Example 3 of the present disclosure for the method) and the antibody purity SEC was detected (see Test Example 2 of the present disclosure for the method). The experimental results are shown in Table 27. The results showed that correct molecules were formed when the light chain in which CL was replaced with the Obscurin-O chain and the light chain in which CL was not replaced were added together, while correct target molecules were not found in the cross-pairing when only one light chain in which CL was not replaced or only one light chain in which CL was replaced with the Obscurin-O chain was added. This indicated that the light chain in which CL was replaced with the Obscurin-O chain would be more easily bound to the heavy chain in which CH1 was replaced with the Titin-T chain to form a molecule, but would not be cross-mispaired with the heavy chain in which CH1 was not replaced with the Titin-T chain; the light chain in which CL was not replaced with the Obscurin-O chain was more easily bound to the heavy chain in which CH1 was not replaced with the Titin-T chain, but would not be bound to the heavy chain in which CH1 was replaced with the Titin-T chain.

**Table 27. Detection results for light/heavy chain mispairings of bispecific antibodies**

| Name of pairing | First heavy chain (B-H1) | Second heavy chain (B-H2) | First light chain (B-L1) | Second light chain (B-L2) | Mass spectrometry | | | | Purity of antibody SEC (%) | Expression level of antibody (mg/L) |
|---|---|---|---|---|---|---|---|---|---|---|
| | | | | | B-H1 | B-H1 | B-H1 | B-H2 | | |
| | | | | | B-H2 | B-H2 | B-H2 | B-H2 | | |
| | | | | | B-L1 | B-L1 | B-L2 | B-L2 | | |
| | | | | | B-L2 | B-L1 | B-L2 | B-L2 | | |
| B/U-1 | Chain formed by replacement of CH1 of heavy chain of B0 with T.10 (with a hole modification) | Heavy chain of U0 (with a knob modification) | Chain formed by replacement of CL of light chain of B0 with O.20 | Light chain of U0 | ✔ | -- | -- | -- | 91 | 25.6 |
| B/U-2 | Heavy chain of B0 (with a hole modification) | Heavy chain of U0 (with a knob modification) | Chain formed by replacement of CL of light chain of B0 with O.20 | Chain formed by replacement of CL of light chain of B0 with O.20 | -- | -- | -- | -- | -- | 0.5 |
| B/U-3 | Chain formed by replacement of CH1 of heavy chain of B0 with T.10 (with a hole modification) | Heavy chain of U0 (with a knob modification) | Light chain of U0 | Light chain of U0 | -- | -- | -- | ✔ | 33 | 15.6 |

| | | | | | | | | | | |
|---|---|---|---|---|---|---|---|---|---|---|
| Note: In the table, "✔" indicates that the four-chain-paired molecule was detected, and "-" indicates that the four-chain-paired molecule was not detected. | | | | | | | | | | |

### Test Example 6: Detection of Affinity of Bispecific Antibody for Antigen

The affinity of a test antibody for an antigen protein was determined by using a Biacore T200 (GE) instrument. According to the method in the instructions of a human anti-capture kit (GE, Cat # BR-1008-39), a Protein A biosensor chip (Cat # 29127556, GE) was used for affinity capture of a test antibody, then soluble antigens with a series of concentration gradients flowed through the surface of the chip, and a Biacore T200 instrument was used for real-time detection of reaction signals, so that an association-dissociation curve was obtained. After dissociation was completed for each cycle, the biosensor chip was washed with a glycine-hydrochloric acid regeneration solution (pH 1.5, Cat # BR-1003-54, GE). The data obtained from the experiment was fitted with BIAevaluation version 4.1, GE software using a (1:1) Langmuir model to obtain affinity values. The experimental results are shown in Table 28.

The experimental results showed that the domain-engineered bispecific antibodies of the present disclosure fully retained the affinity of the two parent monoclonal antibodies for antigens thereof. In this experiment, included were antigens hRANKL (available from Sino biological, 11682-HNCH), hNGF (available from Sino biological, 11050-HNAC), hBAFF (available from Sino biological, 10056-HNCH), hP40 (available from Sino biological, 10052-H08H), hB7H3 (prepared in the laboratory, the sequence was set forth in SEQ ID NO: 111), and hCD3 (prepared in the laboratory, a heterodimer consisting of the δ subunit (the sequence was set forth in SEQ ID NO: 112) and the ε subunit (the sequence was set forth in SEQ ID NO: 113) of hCD3).

**Table 28. Detection results for affinity of bispecific antibodies**

| Antibody | Antigen | ka (1/Ms) | kd (1/s) | KD (M) |
|---|---|---|---|---|
| B0 | hBAFF | 1.77E+05 | 1.52E-04 | 8.55E-10 |
| U0 | hP40 | 1.44E+06 | 1.14E-04 | 7.93E-11 |
| BU5 | hBAFF | 1.94E+05 | 7.62E-05 | 3.93E-10 |
| | hP40 | 1.54E+06 | 1.02E-04 | 6.64E-11 |
| BU1 | hBAFF | 3.38E+05 | 1.69E-04 | 4.99E-10 |
| | hP40 | 1.38E+06 | 4.81E-05 | 3.49E-11 |
| D0 | hRANKL | 2.36E+04 | 2.10E-04 | 8.89E-09 |
| 10 | hNGF | 1.92E+06 | 1.19E-04 | 6.21E-11 |
| DI-1 | hRANKL | 3.05E+04 | 9.36E-05 | 3.06E-09 |
| | hNGF | 2.56E+06 | 8.95E-05 | 3.50E-11 |
| A0 | hCD3 | 8.01E+04 | 4.92E-03 | 6.14E-08 |
| F0 | hB7H3 | 1.57E+05 | 2.06E-03 | 1.31E-08 |
| FA-1 | hCD3 | 7.06E+04 | 4.25E-03 | 6.02E-08 |
| | hB7H3 | 1.58E+05 | 7.12E-03 | 4.50E-08 |

### Test Example 7: Antigen Binding Assay of CH1/CL Domain-Engineered Bispecific Antibody

The affinity of a test antibody for an antigen protein was determined by using a Biacore T200 (GE) instrument to verify that the CH1-CL domain-engineered bispecific antibodies of the present disclosure could bind to two target antigens simultaneously. The specific experimental steps were as follows: a Protein A biosensor chip (Cat # 29127556, GE) was used for affinity capture of the antibody BU5, then a first antigen molecule hP40 (available from Sino biological, 10052-H08H) of the bispecific antibody flowed through the surface of the chip to reach saturation, followed by injection of a second antigen molecule hBAFF (available from Sino biological, 10056-HNCH) at a certain concentration, and a Biacore T200 instrument was used for real-time detection of reaction signals, so that an association-dissociation curve was obtained. After dissociation was completed for each cycle, the biosensor chip was washed with a glycine-hydrochloric acid regeneration solution (pH 1.5, Cat # BR-1003-54, GE). The data was fitted with BIAevaluation version 4.1, GE software using a (1:1) Langmuir model. Then, a Protein A biosensor chip (Cat # 29127556, GE) was used for affinity capture of the antibody BU5, then a first antigen molecule hP40 (available from Sino biological, 10052-H08H) of the bispecific antibody flowed through the surface of the chip to reach saturation, followed by injection of a second antigen molecule hBAFF (available from Sino biological, 10056-HNCH) at a certain concentration, and a Biacore T200 instrument was used for real-time detection of reaction signals, so that an association-dissociation curve was obtained. After dissociation was completed for each cycle, the biosensor chip was washed with a glycine-hydrochloric acid regeneration solution (pH 1.5, Cat # BR-1003-54, GE). The data was fitted with BIAevaluation version 4.1, GE software using a (1:1) Langmuir model.

The experimental results are shown in Table 29. The experimental results showed that the bispecific antibody BU5 could continue to bind to the antigen hBAFF after binding to the antigen hP40 to reach saturation, and had the ability of rebinding to hBAFF comparable to the monoclonal antibody B0. Similarly, the bispecific antibody BU5 could continue to bind to the antigen hP40 after binding to the antigen hBAFF to reach saturation, and had the ability of rebinding to hP40 comparable to U0. This indicated that the bispecific antibodies in which CH1/CL was replaced with a Titin-T chain/Obscurin-O chain of the present disclosure could bind to two target antigens simultaneously.

**Table 29. Detection results for affinity of antibodies**

| Antibody | Antigen flowed through the surface of chip | Affinity detection data | | | |
|---|---|---|---|---|---|
| | | Antigen | ka (1/Ms) | kd (1/s) | KD (M) |
| Bmab | hBAFF | hBAFF | 1.77E+05 | 1.53E-04 | 8.64E-10 |
| Umab | hP40 | hP40 | 1.59E+06 | 1.09E-04 | 6.89E-11 |
| BU5 | Injecting hBAFF for binding to reach saturation, | hP40 | 1.57E+06 | 1.04E-04 | 6.61E-11 |
| | and then injecting hP40 antigen | | | | |
| | Injecting hP40 for binding to reach saturation, | hBAFF | 1.57E+05 | 8.44E-05 | 5.37E-10 |
| | and then injecting hBAFF antigen | | | | |

### Test Example 8: Experiment of Osteoclast Differentiation of DI-1 Bispecific Antibody

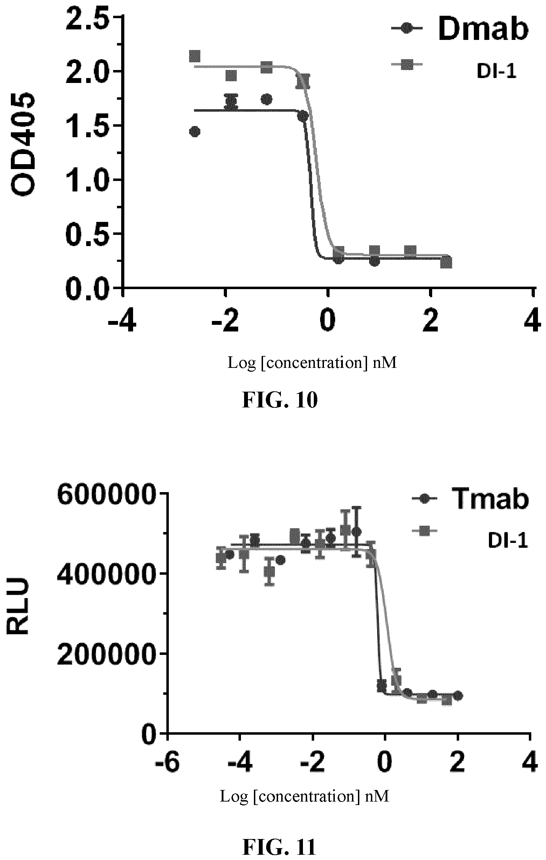

Raw264.7 cells (the Chinese Academy of Cell Bank, SCSP-5036) were digested and resuspended for counting. The cells were plated in a 24-well cell culture plate (Corning, 3524) and cultured in a cell incubator at 37 °C overnight. The next day, the antibody solution was diluted to different concentrations and mixed homogeneously with RANKL (Sino biological, 11682-HNCH), and the mixture was added to the cell culture plate to make a final concentration of 50 ng/mL, and cultured at 37 °C. After 96 h, the solution in the 24-well plate was removed, 100 µL of cell lysis buffer (Beyotime, P0013J) was added to each well, and the mixture was pipetted and mixed homogeneously. The lysate was transferred to an EP tube and centrifuged at 12000 g for 5 min, and the supernatant was taken for detection using an anti-tartaric acid phosphatase test kit (Beyotime, P0332) according to the method described in the instructions. The absorption values at 405 nm were read on a microplate reader. The determined values were fitted with software to obtain a curve, and the IC₅₀ values were calculated. The experimental results are shown in FIG. 10. The experimental results showed that the bispecific antibody DI-1 in which CH1/CL was replaced retained good activity and effectively inhibited osteoclast differentiation.

### Test Example 9: Experiment of TF1 Cell Proliferation of DI-1 Bispecific Antibody

The activity of the antibody at the cellular level was assessed in an NGF-induced TF-1 cell (ATCC, CRL-2003) proliferation experiment. The experimental procedures were as follows: TF1 cells were digested and collected, resuspended for counting, plated in a 96-well plate (Corning, 3903), and cultured in an incubator at 37 °C overnight. The next day, the antibody solution was diluted to different concentrations and mixed homogeneously with NGF (Sino biological, 11050-HNAC), and the mixture was added to the cell culture plate to make a final concentration of 10 ng/mL, and cultured in the incubator. After 72 h, the cell culture plate was removed, 50 µL of Cell-titer Glo (Promega, G755B) assay solution was added to each well, and the mixture was incubated on a shaker for 10 min and left to stand at room temperature for 10 min. Luminescence signals were detected using a microplate reader (PerkinElmer, Victor3). The detected signal values were fitted with software to obtain a curve graph, and the IC₅₀ values were calculated. The experimental results are shown in FIG. 11. The experimental results showed that the bispecific antibody DI-1 in which CH1/CL was replaced had good activity.

### Test Example 10: Detection of Light and Heavy Chain Mispairing of Bispecific Antibody in Which CH1/CL is Replaced

The following experiments were performed to analyze the light and heavy chain mispairings of bispecific antibodies in which CH1/CL was replaced with a Titin-T chain/Obscurin-O chain or with TCRα/TCRβ. The specific experiments were as follows:

### I. Experiment of light and heavy chain mispairings during co-expression of four chains of bispecific antibody

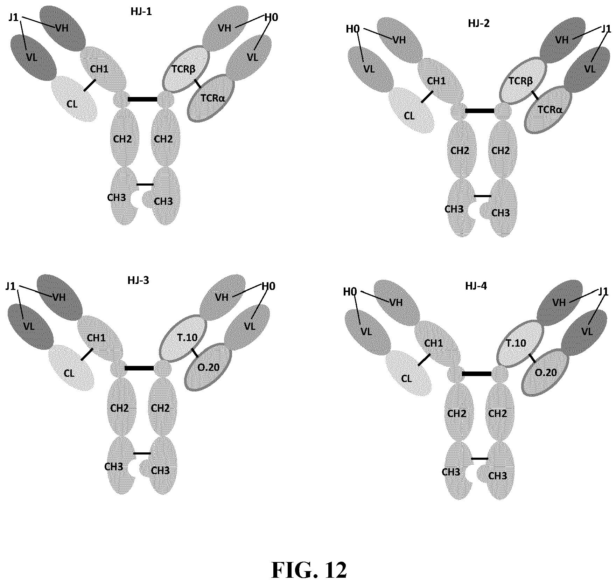

Bispecific antibodies HJ-1 and HJ-2 each consisting of four chains were constructed by replacing CH1/CL of H0 or J1 with TCRα/TCRβ, and bispecific antibodies HJ-3 and HJ-4 each consisting of four chains were constructed by replacing CH1/CL of H0 or J1 with the Titin-T chain/Obscurin-O chain. The schematic structural diagrams of the bispecific antibodies are shown in FIG. 12, and the full-length sequences of the bispecific antibodies are shown in Table 30:

**Table 30. Sequence Listing of bispecific antibodies**

| Bispecific antibody No. | Sequences of four cha ins of HJ bispecific ant ibody | | | |
|---|---|---|---|---|
| | Chain 1 | Chain 3 | Chain 2 | Chain 4 |
| HJ-1 | Heavy chain of HO (knob), in which CH1 is replaced with TCRβ | Light chain of HO, in which CL is replaced with TCRα | Heavy chain of J1 (hole) | Light chain of J1 |
| | HJ-1-H1: | HJ-1-L1: | HJ-1-H2: | HJ-1-L2: |
| | EVQLVESGGGLV QPGGSLRLSCAAS GFTFSHYYMAWV RQAPGKGLEWVT SISYEGDITYYGD SVKGRFTISRDNS KNTLYLQMNSLR AEDTATYYCASQ TLRESFDYWGQG TLVTVSSLEDLK NVFPPEVAVFEPS EAEISHTQKATL VCLATGFYPDHV ELSWWVNGKEV HSGVCTDPQPLK EQPALQDSRYAL SSRLRVSATFWQ NPRNHFRCQVQ FYGLSENDEWT QDRAKPVTQIVS AEAWGRASDKT HTCPPCPAPEAAG GPSVFLFPPKPKD TLMISRTPEVTCV VVDVSHEDPEVK FNWYVDGVEVH NAKTKPREEQYN STYRVVSVLTVLH QDWLNGKEYKC KVSNKALPAPIEK TISKAKGQPREPQ VYTLPPCREEMT KNQVSLWCLVKG FYPSDIAVEWESN GQPENNYKTTPP VLDSDGSFFLYSK LTVDKSRWQQGN VFSCSVMHEALH NHYTQKSLSLSPG KHHHHHH (SEQ ID NO: 114) | DIQMTQSPSSVSA SVGDRVTITCRAS QDIANYLSWYQQ KPGKSPKLLIYGT SNLEVGVPSRFSG SRSGTDFTLTISSL QPEDFATYYCLQD KEFPRTFGGGTKV EIKKPDIQNPDPA VYQLRDSKSSDK SVCLFTDFDSQT QVSQSKDSDVYI TDKCVLDMRSM DFKSNSAVAWSQ KSDFACANAFQN SIIPEDTFFPSPES S (SEQ ID NO: 115) | EVQLVQSGAEVK KPGSSVKVSCKA SGYTFSNYLIEWV RQAPGQGLEWIG VIDPGVGDTNYN ENFKGRATLTADK STSTAYIELSSLRS EDTAVYYCARED NTGTAFDYWGQG TTVTVSSASTKGP SVFPLAPSSKSTS GGTAALGCLVKD YFPEPVTVSWNS GALTSGVHTFPAV LQSSGLYSLSSVV TVPSSSLGTQTYI CNVNHKPSNTKV DKKVEPKSCDKT HTCPPCPAPEAAG GPSVFLFPPKPKD TLMISRTPEVTCV VVDVSHEDPEVK FNWYVDGVEVH NAKTKPREEQYN STYRVVSVLTVLH QDWLNGKEYKC KVSNKALPAPIEK TISKAKGQPREPQ VCTLPPSREEMTK NQVSLSCAVKGF YPSDIAVEWESNG QPENNYKTTPPVL DSDGSFFLVSKLT VDKSRWQQGNVF SCSVMHEALHNH YTQKSLSLSPGKD YKDDDDK (SEQ ID NO: 116) | SIVMTQTPLSLSV TPGQPASISCKAS QSVSSDVTWYLQ KPGQSPQLLIYYV SEHYTGVPDRFSG SGYGTDFTLKISR VEAEDVGVYYCQ QHHRFPLTFGQGT KLEIKRTVAAPSV FIFPPSDEQLKSGT ASVVCLLNNFYP REAKVQWKVDN ALQSGNSQESVTE QDSKDSTYSLSST LTLSKADYEKHK VYACEVTHQGLS SPVTKSFNRGEC (SEQ ID NO: 117) |
| HJ-2 | Heavy chain of HO (hole) | Light chain of HO | Heavy chain of J1 (knob), in which CH1 is replaced with TCRβ | Light chain of 11, in which CL is replaced with TCRα |
| | HJ-2-H1: | HJ-2-L1: | HJ-1-H2: | HJ-2-L2: |
| | EVQLVESGGGLV QPGGSLRLSCAAS GFTFSHYYMAWV RQAPGKGLEWVT SISYEGDITYYGD SVKGRFTISRDNS KNTLYLQMNSLR AEDTATYYCASQ TLRESFDYWGQG TLVTVSSASTKGP SVFPLAPSSKSTS GGTAALGCLVKD YFPEPVTVSWNS GALTSGVHTFPAV LQSSGLYSLSSVV TVPSSSLGTQTYI CNVNHKPSNTKV DKKVEPKSCDKT HTCPPCPAPEAAG GPSVFLFPPKPKD TLMISRTPEVTCV VVDVSHEDPEVK FNWYVDGVEVH NAKTKPREEQYN STYRVVSVLTVLH QDWLNGKEYKC KVSNKALPAPIEK TISKAKGQPREPQ VCTLPPSREEMTK NQVSLSCAVKGF YPSDIAVEWESNG QPENNYKTTPPVL DSDGSFFLVSKLT VDKSRWQQGNVF SCSVMHEALHNH YTQKSLSLSPGKD YKDDDDK (SEQ ID NO: 118) | DIQMTQSPSSVSA SVGDRVTITCRAS QDIANYLSWYQQ KPGKSPKLLIYGT SNLEVGVPSRFSG SRSGTDFTLTISSL QPEDFATYYCLQD KEFPRTFGGGTKV EIKRTVAAPSVFIF PPSDEQLKSGTAS VVCLLNNFYPRE AKVQWKVDNAL QSGNSQESVTEQ DSKDSTYSLSSTL TLSKADYEKHKV YACEVTHQGLSSP VTKSFNRGEC (SEQ ID NO: 119) | EVQLVQSGAEVK KPGSSVKVSCKA SGYTFSNYLIEWV RQAPGQGLEWIG VIDPGVGDTNYN ENFKGRATLTADK STSTAYIELSSLRS EDTAVYYCARED NTGTAFDYWGQG TTVTVSSLEDLK NVFPPEVAVFEPS EAEISHTQKATL VCLATGFYPDHV ELSWWVNGKEV HSGVCTDPQPLK EQPALQDSRYAL SSRLRVSATFWQ NPRNHFRCQVQ FYGLSENDEWT QDRAKPVTQIVS AEAWGRASDKT HTCPPCPAPEAAG GPSVFLFPPKPKD TLMISRTPEVTCV VVDVSHEDPEVK FNWYVDGVEVH NAKTKPREEQYN STYRVVSVLTVLH QDWLNGKEYKC KVSNKALPAPIEK TISKAKGQPREPQ VYTLPPCREEMT KNQVSLWCLVKG FYPSDIAVEWESN GQPENNYKTTPP VLDSDGSFFLYSK LTVDKSRWQQGN VFSCSVMHEALH NHYTQKSLSLSPG KHHHHHH (SEQ ID NO: 120) | SIVMTQTPLSLSV TPGQPASISCKAS QSVSSDVTWYLQ KPGQSPQLLIYYV SEHYTGVPDRFSG SGYGTDFTLKISR VEAEDVGVYYCQ QHHRFPLTFGQGT KLEIKKPDIQNPD PAVYQLRDSKSS DKSVCLFTDFDS QTQVSQSKDSDV YITDKCVLDMRS MDFKSNSAVAWS QKSDFACANAFQ NSIIPEDTFFPSPE SS (SEQ ID NO: 121) |
| HJ-3 | Heavy chain (knob) of H0, in which CH1 is replaced with T.10-L1 | Light chain of HO, in which CL is replaced with O.20-L1 | Heavy chain of J1 (hole) | Light chain of J1 |
| | HJ-3-H1: | HJ-3-L1: | HJ-3-H2: | HJ-3-L2: |
| | EVQLVESGGGLV QPGGSLRLSCAAS GFTFSHYYMAWV RQAPGKGLEWVT SISYEGDITYYGD SVKGRFTISRDNS KNTLYLQMNSLR AEDTATYYCASQ TLRESFDYWGQG | DIQMTQSPSSVSA SVGDRVTITCRAS QDIANYLSWYQQ KPGKSPKLLIYGT SNLEVGVPSRFSG SRSGTDFTLTISSL QPEDFATYYCLQD KEFPRTFGGGTKV EIKGGGGSSGAP | EVQLVQSGAEVK KPGSSVKVSCKA SGYTFSNYLIEWV RQAPGQGLEWIG VIDPGVGDTNYN ENFKGRATLTADK STSTAYIELSSLRS EDTAVYYCARED NTGTAFDYWGQG | SIVMTQTPLSLSV TPGQPASISCKAS QSVSSDVTWYLQ KPGQSPQLLIYYV SEHYTGVPDRFSG SGYGTDFTLKISR VEAEDVGVYYCQ QHHRFPLTFGQGT KLEIKRTVAAPSV |
| | TLVTVSSGGGGS GIPPKIECLPIDIS IDEGKVLTVASA FTGEPTPEVTWS TGGRKIHSQEQG RFHIENTDDLTT LIIKDVQKQDGG LYTLTLRNEFGS DSATVNIHIRSID KTHTCPPCPAPEA AGGPSVFLFPPKP KDTLMISRTPEVT CVVVDVSHEDPE VKFNWYVDGVE VHNAKTKPREEQ YNSTYRVVSVLT VLHQDWLNGKE YKCKVSNKALPA PIEKTISKAKGQP REPQVYTLPPCRE EMTKNQVSLWCL VKGFYPSDIAVEW ESNGQPENNYKT TPPVLDSDGSFFL YSKLTVDKSRWQ QGNVFSCSVMHE ALHNHYTQKSLS LSPGKHHHHHH (SEQ ID NO: 122) | RFLTRPLAFVVS VGKDATLSSQIV GNPTPQVSWEK DKQPVTAGARFR LAQDGDLYRLKI LDLQLSDSGQYV SRARNAIGEAFA CLGLQVDAEA (SEQ ID NO: 123) | TTVTVSSASTKGP SVFPLAPSSKSTS GGTAALGCLVKD YFPEPVTVSWNS GALTSGVHTFPAV LQSSGLYSLSSVV TVPSSSLGTQTYI CNVNHKPSNTKV DKKVEPKSCDKT HTCPPCPAPEAAG GPSVFLFPPKPKD TLMISRTPEVTCV VVDVSHEDPEVK FNWYVDGVEVH NAKTKPREEQYN STYRVVSVLTVLH QDWLNGKEYKC KVSNKALPAPIEK TISKAKGQPREPQ VCTLPPSREEMTK NQVSLSCAVKGF YPSDIAVEWESNG QPENNYKTTPPVL DSDGSFFLVSKLT VDKSRWQQGNVF SCSVMHEALHNH YTQKSLSLSPGKD YKDDDDK (SEQ ID NO: 116) | FIFPPSDEQLKSGT ASVVCLLNNFYP REAKVQWKVDN ALQSGNSQESVTE QDSKDSTYSLSST LTLSKADYEKHK VYACEVTHQGLS SPVTKSFNRGEC (SEQ ID NO: 117) |
| HJ-4 | Heavy chain of HO (hole) | Light chain of HO | Heavy chain (knob) of J1, in which CH1 is replaced with T.10-L1 | Light chain of 11, in which CL is replaced with O.20-L1 |
| | HJ-4-H1: | HJ-4-L1: | HJ-4-H2: | HJ-4-L2: |
| | EVQLVESGGGLV QPGGSLRLSCAAS GFTFSHYYMAWV RQAPGKGLEWVT SISYEGDITYYGD SVKGRFTISRDNS KNTLYLQMNSLR AEDTATYYCASQ TLRESFDYWGQG TLVTVSSASTKGP SVFPLAPSSKSTS GGTAALGCLVKD YFPEPVTVSWNS GALTSGVHTFPAV LQSSGLYSLSSVV TVPSSSLGTQTYI CNVNHKPSNTKV DKKVEPKSCDKT HTCPPCPAPEAAG GPSVFLFPPKPKD TLMISRTPEVTCV VVDVSHEDPEVK FNWYVDGVEVH NAKTKPREEQYN STYRVVSVLTVLH QDWLNGKEYKC | DIQMTQSPSSVSA SVGDRVTITCRAS QDIANYLSWYQQ KPGKSPKLLIYGT SNLEVGVPSRFSG SRSGTDFTLTISSL QPEDFATYYCLQD KEFPRTFGGGTKV EIKRTVAAPSVFIF PPSDEQLKSGTAS VVCLLNNFYPRE AKVQWKVDNAL QSGNSQESVTEQ DSKDSTYSLSSTL TLSKADYEKHKV YACEVTHQGLSSP VTKSFNRGEC (SEQ ID NO: 119) | EVQLVQSGAEVK KPGSSVKVSCKA SGYTFSNYLIEWV RQAPGQGLEWIG VIDPGVGDTNYN ENFKGRATLTADK STSTAYIELSSLRS EDTAVYYCARED NTGTAFDYWGQG TTVTVSSGGGGS GIPPKIECLPIDIS IDEGKVLTVASA FTGEPTPEVTWS TGGRKIHSQEQG RFHIENTDDLTT LIIKDVQKQDGG LYTLTLRNEFGS DSATVNIHIRSID KTHTCPPCPAPEA AGGPSVFLFPPKP KDTLMISRTPEVT CVVVDVSHEDPE VKFNWYVDGVE VHNAKTKPREEQ YNSTYRVVSVLT VLHQDWLNGKE | SIVMTQTPLSLSV TPGQPASISCKAS QSVSSDVTWYLQ KPGQSPQLLIYYV SEHYTGVPDRFSG SGYGTDFTLKISR VEAEDVGVYYCQ QHHRFPLTFGQGT KLEIKGGGGSSG APRFLTRPLAFV VSVGKDATLSSQ IVGNPTPQVSWE KDKQPVTAGAR FRLAQDGDLYRL KILDLQLSDSGQ YVSRARNAIGEA FACLGLQVDAEA (SEQ ID NO: 125) |
| | KVSNKALPAPIEK TISKAKGQPREPQ VCTLPPSREEMTK NQVSLSCAVKGF YPSDIAVEWESNG QPENNYKTTPPVL DSDGSFFLVSKLT VDKSRWQQGNVF SCSVMHEALHNH YTQKSLSLSPGKD YKDDDDK (SEQ ID NO: 118) | | YKCKVSNKALPA PIEKTISKAKGQP REPQVYTLPPCRE EMTKNQVSLWCL VKGFYPSDIAVEW ESNGQPENNYKT TPPVLDSDGSFFL YSKLTVDKSRWQ QGNVFSCSVMHE ALHNHYTQKSLS LSPGKHHHHHH (SEQ ID NO: 124) | |

| | | | | |
|---|---|---|---|---|
| Note: In the sequences, the bold part indicates the Titin-T chain/Obscurin-O chain portion in the TCR sequence, the dotted underlined part indicates the Fc or CL constant region portion, the single underlined part indicates the variable region portion, and the dashed underlined part indicates the linker L1. | | | | |

Heavy chain of 11 : 
EVQLVQSGAEVKKPGSSVKVSCKASGYTFSNYLIEWVRQAPGQGLEWIGVIDP
GVGDTNYNENFKGRATLTADKSTSTAYIELSSLRSEDTAVYYCAREDNTGTAFD
YWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKK
VEPKSCDKTHTCPPCPAPELLGGPSVFLFPPKPKDTLYITREPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVM
HEALHNHYTQKSLSLSPGK
(SEQ ID NO: 126).
Light chain of J1: 
SIVMTQTPLSLSVTPGQPASISCKASQSVSSDVTWYLQKPGQSPQLLIYYVSEHY
TGVPDRFSGSGYGTDFTLKISRVEAEDVGVYYCQQHHRFPLTFGQGTKLEIKRT
VAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
(SEQ ID NO: 117).
Heavy chain variable region of J1: 
EVQLVQSGAEVKKPGSSVKVSCKASGYTFSNYLIEWVRQAPGQGLEWIGVIDP
GVGDTNYNENFKGRATLTADKSTSTAYIELSSLRSEDTAVYYCAREDNTGTAFD
YWGQGTTVTVSS
(SEQ ID NO: 171).
Light chain variable region of J1:

SIVMTQTPLSLSVTPGQPASISCKASQSVSSDVTWYLQKPGQSPQLLIYYVSEHY
TGVPDRFSGSGYGTDFTLKISRVEAEDVGVYYCQQHHRFPLTFGQGTKLEIK
(SEQ ID NO: 172).

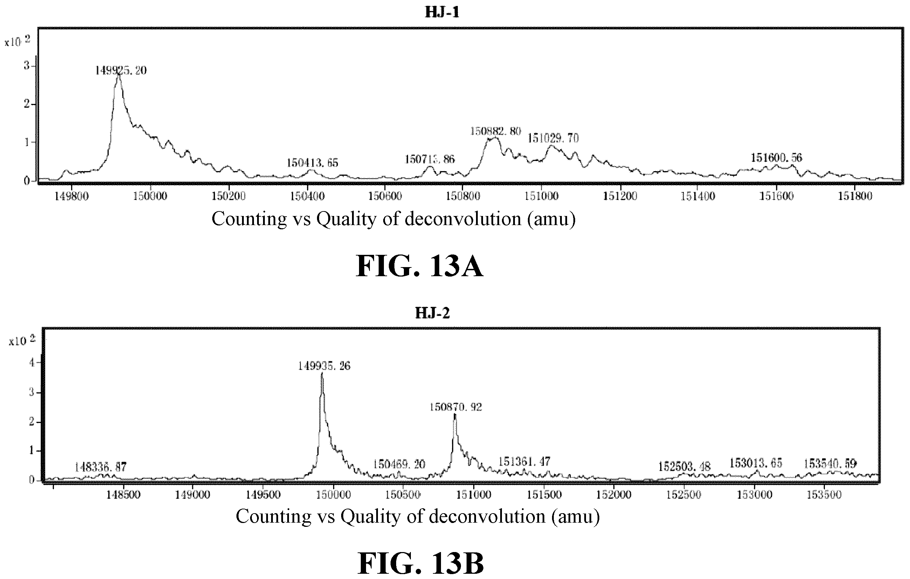

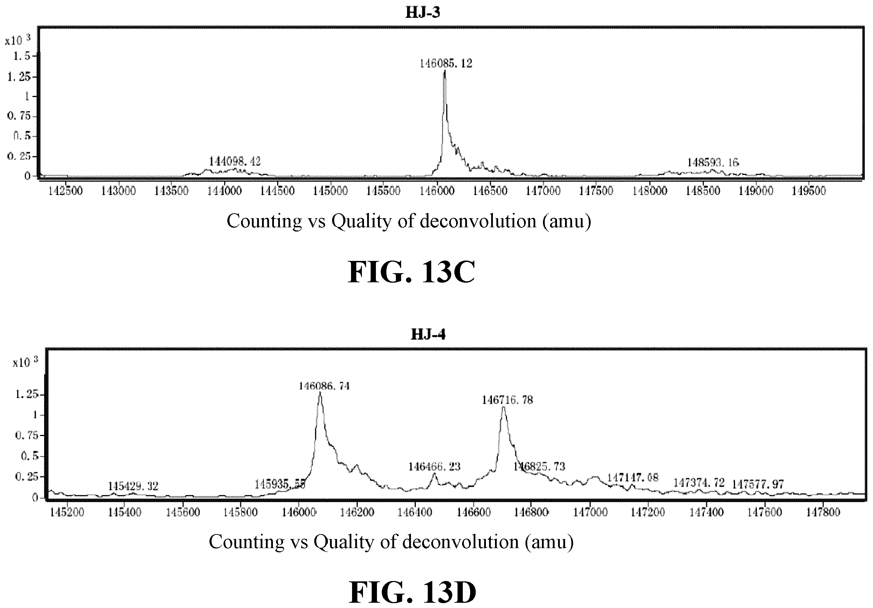

The four chains of HJ-1, HJ-2, HJ-3, and HJ-4 were co-transfected into cells for expression, and then the expression products were subjected to mass spectrometry (see Test Example 3 of the present disclosure for the method) to determine whether there were light and heavy chain mispaired molecules. The experimental results are shown in FIGs. 13A-13D and Table 31.

**Table 31. Experimental results for mass spectrometry of expression products of bispecific antibodies**

| Bispecific antibody No. | Co-transfected chains | Results for mass spectrometry | | |
|---|---|---|---|---|
| | | HJ (1+2+3+4) | Mispaired molecule 1 (1 + 2 + 3 + 3) | Mispaired molecule 2 (1 + 2 + 4 + 4) |
| HJ-1 | 1: HJ-1-H1 | √ | × | √ |
| | 2: HJ-1-H2 | | | |
| | 3: HJ-1-L1 | | | |
| | 4: HJ-1-L2 | | | |
| HJ-2 | 1: HJ-2-H2 | √ | × | √ |
| | 2: HJ-2-H1 | | | |
| | 3: HJ-2-L2 | | | |
| | 4: HJ-2-L1 | | | |
| HJ-3 | 1: HJ-3-H1 | √ | × | × |
| | 2: HJ-3-H2 | | | |
| | 3: HJ-3-L1 | | | |
| | 4: HJ-3-L2 | | | |
| HJ-4 | 1: HJ-4-H2 | √ | × | × |
| | 2: HJ-4-H1 | | | |
| | 3: HJ-4-L2 | | | |
| | 4: HJ-4-L1 | | | |

| | | | | |
|---|---|---|---|---|
| Note: In the table, "√" indicates that the molecule was detected, "×" indicates that the molecule was not detected, "HJ (1 + 2 + 3 + 4)" indicates a bispecific antibody formed by the four corresponding chains numbered 1, 2, 3 and 4 on the left side thereof, "mispaired molecule 1 (1 + 2 + 3 + 3)" indicates a mispaired molecule 1 formed by the four corresponding chains numbered 1, 2, 3 and 3 on the left side thereof; and "mispaired molecule 2 (1 + 2 + 4 + 4)" indicates a mispaired molecule 2 formed by the four corresponding chains numbered 1, 2, 4 and 4 on the left side thereof. | | | | |

The experimental results showed that when four chains of the bispecific antibody in which CH1/CL was replaced with TCRβ/TCRα were co-transfected for expression, the light and heavy chains of the antibody were mispaired. However, the bispecific antibody in which CH1/CL was replaced with the Titin-T chain/Obscurin-O chain had no light and heavy chain mispairings. This indicated that the bispecific antibodies in which CH1/CL was replaced with a Titin-T chain/Obscurin-O chain of the present disclosure had excellent ability of reducing light and heavy chain mispairings.

### II. Experiment of bispecific antibodies with three chains co-expressed

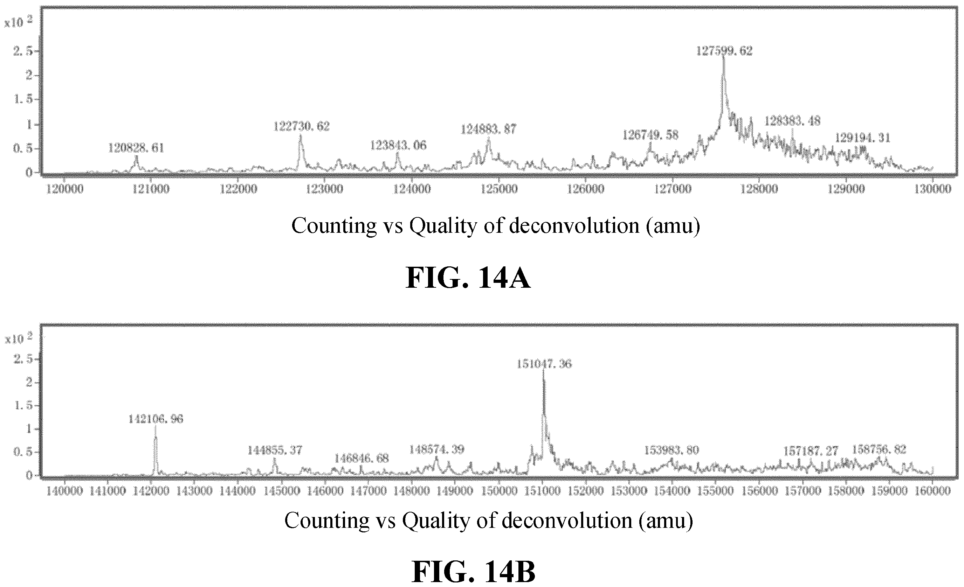

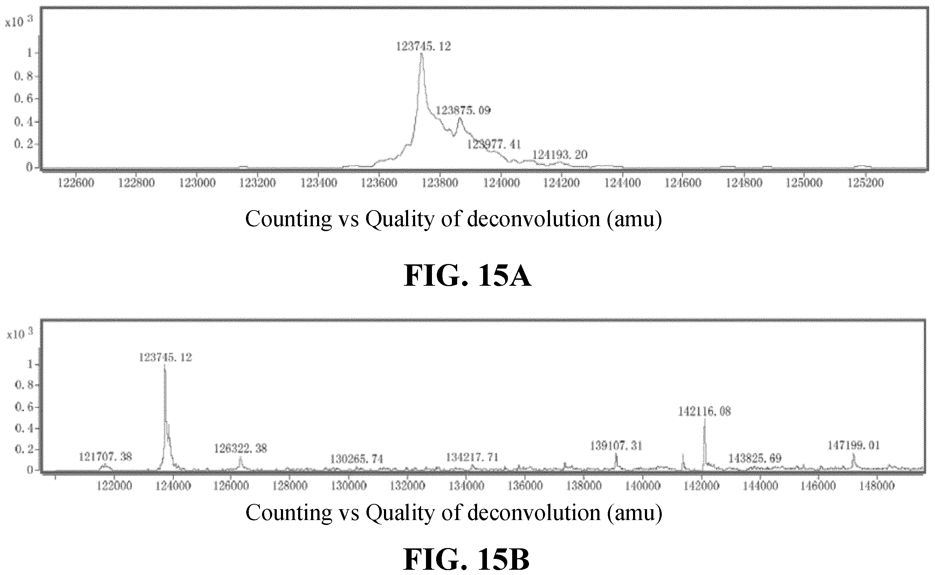

In this experiment, three chains (1 heavy chain in which CH1 was replaced with TCRβ or Titin-T chain, 1 heavy chain in which CH1 was not replaced, and 1 wild-type light chain (VL-CL) in which CL was not replaced; specific sequences are shown in Table 30) were co-transfected into cells for expression, the expression products were subjected to mass spectrometry (see Test Example 3 of the present disclosure for the method), and the antibody purity SEC was determined (see Test Example 2 of the present disclosure for the method), so as to verify whether the wild-type light chain (VL-CL) would form a mispaired molecule in combination with the heavy chain in which CH1 was replaced with TCRβ or Titin-T chain. The experimental results are shown in FIG. 14A, FIG. 14B, FIG. 15A, FIG. 15B, and Table 32.

**Table 32. Experimental results for bispecific antibodies with three chains co-expressed**

| Group | Co-transfected chains | Antibody purity SEC (%) | Results for mass spectrometry | |
|---|---|---|---|---|
| | | | Molecule (1 + 2 + 3) | Mispaired molecule (1 + 2 + 3 + 3) |
| 1 | 1: HJ-1-H1 | 75.85 | √ | √ |
| | 2: HJ-1-H2 | | | |
| | 3: HJ-1-L2 | | | |
| 2 | 1: HJ-3-H1 | 96.82 | √ | × |
| | 2: HJ-3-H2 | | | |
| | 3: HJ-3-L2 | | | |

| | | | | |
|---|---|---|---|---|
| Note: In the table, "√" indicates that the molecule was detected, "X" indicates that the molecule was not detected, "molecule (1 + 2 + 3)" indicates a molecule formed by the three corresponding chains numbered 1, 2 and 3 on the left side thereof, and "mispaired molecule (1 + 2 + 3 + 3)" indicates a mispaired molecule formed by the four corresponding chains 1, 2, 3 and 3 on the left side thereof. | | | | |

The experimental results showed that VL-CL was readily bound to the heavy chain in which CH1 was replaced with TCRβ to express a mispaired molecule with two identical light chains. However, the VL-CL light chain did not bind to the heavy chain in which CH1 was replaced with the Titin-T chain to form a mispaired molecule with two identical light chains, which indirectly indicated that the bispecific antibody in which CH1 of the heavy chain was replaced with the Titin-T chain of the present disclosure had more excellent ability of reducing light and heavy chain mispairings as compared to the bispecific antibody in which CH1 of the heavy chain was replaced with TCRβ.

### Test Example 11: (FabV)₂-IgG Bispecific Antibody in Which CH1/CL is Replaced

### I. Construction of PDL1-TIGIT bispecific antibodies in which CH1/CL was replaced

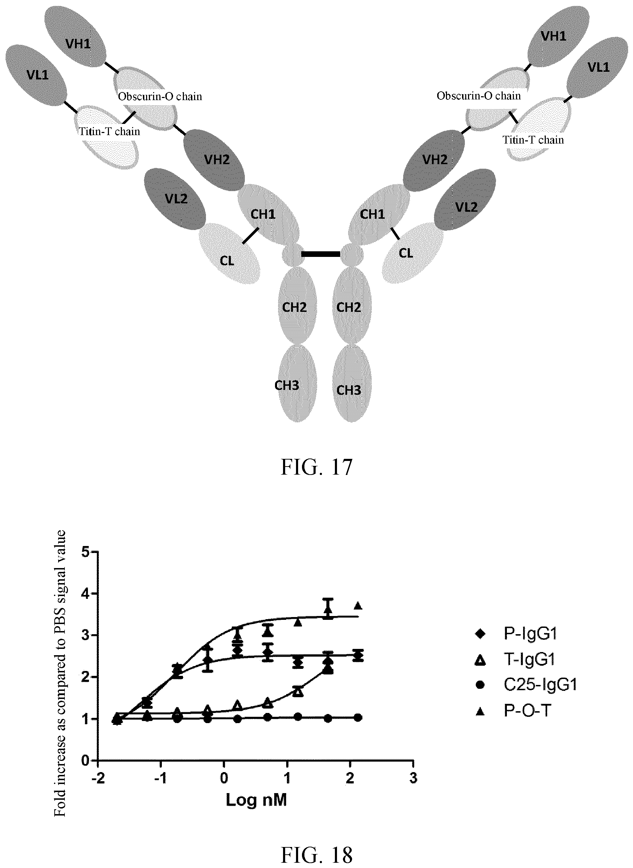

An anti-PDL1-TIGIT bispecific antibody P-O-T in a (FabV)₂-IgG form in which CH1/CL was replaced with a Titin-T chain/Obscurin-O chain (T.10/O.24) was constructed (the schematic structural diagram is shown in FIG. 17) using an anti-TIGIT antibody disclosed in International Patent Application WO2019062832A1 (antibody name: h1708-04) and an anti-PDL1 antibody disclosed in International Patent Application WO2020177733A1 (antibody name: h1831K). P-O-T comprises 2 identical light chains 1, 2 identical light chains 2, and 2 identical heavy chains, and the amino acid sequences of the polypeptide chains of P-O-T are shown as follows:
Light chain 1 of P-O-T: 
DIVLTQSPASLAVSPGQRATITCRASESVSIHGTHLMHWYQQKPGQPPKLLIYAA
SKLESGVPARFSGSGSGTDFTLTINPVEAEDTANYYCQQSFEDPLTFGQGTKLEIK
GGGGSGIPPKIECLPIDISIDEGKVLTVASAFTGEPTPEVTWSTGGRKIHSQEQGRF
 
HIENTDDLTTLIIKDVQKQDGGLYTLTLRNEFGSDSATVNIHIRSI (SEQ ID NO:
146)
 (Note: The single underlined part indicates the light chain variable region VL1 portion (same as VL of h1831K), the dotted underlined part indicates the linker portion, and the dotted line indicates the T. 10 portion)
Light chain 2 of P-O-T: 
DIQMTQSPSSLSASVGDRVTITCRASENIYSYLAWYQQKPGKSPKLLIYNARTLA
EGVPSRFSGSGSGTDFTLTISSLQPEDFATYYCQYHSGSPLPFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC (SEQ
ID NO: 147)
 (Note: The single underlined part indicates the light chain variable region VL2 portion (same as VL of h1708-04), and the wavy line part indicates the light chain constant region (same as CL of h1708-04))
Heavy chain of P-O-T: 
QVQLVQSGAEVKKPGASVKVSCKASGYTFTSYWMHWVRQAPGQGLEWMGRI
TPSSGFAMYNEKFKNRVTMTRDTSTSTVYMELSSLRSEDTAVYYCARGGSYD
YFDYWGQGTTVTVSSGGGGSSGAPRFLTRPKAFVVSVGKDATLSSQIVGNPFPQ
VSWEKDKQPVTAGVRFRLAQDGDLYRLKILDLQLSDSGQYVSRARNAIGEAFA
CLGLQVDAEAGGGGSEVQLVQSGAEVKKPGASVKVSCKASGYTFTNYWMHW
VRQAPGQGLEWMGRIDPDSTGSKYNEKFKTRVTMTRDTSTSTVYMELSSLRSE
DTAVYYCAREGAYGYYFDYWGQGTLVTVSSASTKGPSVFPLAPSSKSTSGGTA
ALGCLVKDYFPEPVTVSWNSGALTSGVHTFPAVLQSSGLYSLSSVVTVPSSSLGT
QTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLT
VDKSRWQQGNVFSCSVMHEALHNHYTQKSLSLSPGK (SEQ ID NO: 148)
 (Note: The single underlined part indicates the heavy chain variable region VH1 portion (same as VH of h1831K), the double underlined part indicates the heavy chain variable region VH2 portion (same as VH of h1708-04), the dotted line part indicates the O.24 portion, the dotted line part indicates the linker portion, and the wavy line part indicates the heavy chain constant region portion (same as the heavy chain constant region of h1708-04))

The sequences of h1831K (P-IgG1) and h1708-04 (T-IgG1) are as follows:
Light chain of h1708-04: 
DIQMTQSPSSLSASVGDRVTITCRASENIYSYLAWYQQKPGKSPKLLIYNARTLA
EGVPSRFSGSGSGTDFTLTISSLQPEDFATYYCQYHSGSPLPFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC (SEQ
 ID NO: 149)
Heavy chain of h1708-04: 
EVQLVQSGAEVKKPGASVKVSCKASGYTFTNYWMHWVRQAPGQGLEWMGRI
DPDSTGSKYNEKFKTRVTMTRDTSTSTVYMELSSLRSEDTAVYYCAREGAYGY
YFDYWGQGTLVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK (SEQ ID NO: 150)
Light chain of h1831K: 
DIVLTQSPASLAVSPGQRATITCRASESVSIHGTHLMHWYQQKPGQPPKLLIYAA
SKLESGVPARFSGSGSGTDFTLTINPVEAEDTANYYCQQSFEDPLTFGQGTKLEIK
RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC
(SEQ ID NO: 151)
Heavy chain of h1831K:

QVQLVQSGAEVKKPGASVKVSCKASGYTFTSYWMHWVRQAPGQGLEWMGRI
TPSSGFAMYNEKFKNRVTMTRDTSTSTVYMELSSLRSEDTAVYYCARGGSSYD
YFDYWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK (SEQ ID NO: 152)

Note: In the above sequences, the single underlined part indicates the variable region, and the wavy line part indicates the constant region.

In addition, the sequences of the variable regions and CDRs of h1708-04 and h1831K (see Table 33) are as follows:
Light chain variable region of h1708-04: 
DIQMTQSPSSLSASVGDRVTITCRASENIYSYLAWYQQKPGKSPKLLIYNARTLA
EGVPSRFSGSGSGTDFTLTISSLQPEDFATYYCQYHSGSPLPFGGGTKVEIK (SEQ
ID NO: 153)
Heavy chain variable region of h1708-04: 
EVQLVQSGAEVKKPGASVKVSCKASGYTFTNYWMHWVRQAPGQGLEWMGRI
DPDSTGSKYNEKFKTRVTMTRDTSTSTVYMELSSLRSEDTAVYYCAREGAYGY
YFDYWGQGTLVTVSS (SEQ ID NO: 154)
Light chain variable region of h1831K: DIVLTQSPASLAVSPGQRATITCRASESVSIHGTHLMHWYQQKPGQPPKLLIYAA 
SKLESGVPARFSGSGSGTDFTLTINPVEAEDTANYYCQQSFEDPLTFGQGTKLEIK
(SEQ ID NO: 155)
Heavy chain variable region of h1831K:

QVQLVQSGAEVKKPGASVKVSCKASGYTFTSYWMHWVRQAPGQGLEWMGRI
TPSSGFAMYNEKFKNRVTMTRDTSTSTVYMELSSLRSEDTAVYYCARGGSSYD
YFDYWGQGTTVTVSS (SEQ ID NO: 156)

**Table 33. Sequences of antibody heavy and light chain CDR regions**

| Antibody | Heavy chain | | Light chain | |
|---|---|---|---|---|
| h1708-04 | HCDR1 | NYWMH | LCDR1 | RASENIYSYLA |
| | | SEQ ID NO: 157 | | SEQ ID NO: 160 |
| | HCDR2 | RIDPDSTGSKYNEKFKT | LCDR2 | NARTLAE |
| | | SEQ ID NO: 158 | | SEQ ID NO: 161 |
| | HCDR3 | EGAYGYYFDY | LCDR3 | QYHSGSPLP |
| | | SEQ ID NO: 159 | | SEQ ID NO: 162 |
| h1831K | HCDR1 | SYWMH | LCDR1 | RASESVSIHGTHLMH |
| | | SEQ ID NO: 163 | | SEQ ID NO: 166 |
| | HCDR2 | RITPSSGFAMYNEKFKN | LCDR2 | AASKLES |
| | | SEQ ID NO: 164 | | SEQ ID NO: 167 |
| | HCDR3 | GGSSYDYFDY | LCDR3 | QQSFEDPLT |
| | | SEQ ID NO: 165 | | SEQ ID NO: 168 |

| | | | | |
|---|---|---|---|---|
| Note: The sequences of the CDR regions were determined according to the Kabat numbering scheme. | | | | |

### II. Assay of ability of PDL1-TIGIT bispecific antibodies in which CH1/CL is replaced to bind to PDL1 and TIGIT

The affinity of the bispecific molecules P-O-T, human PDL1-his (hPDL1, Cat. No. 10084-H08H, S.B), and human TIGIT-his (hTIGIT, Cat. No. 10917-H08H, S.B) of the present disclosure was determined by using Biacore T200. Specifically, a Protein A biosensor chip (Cat. No. 29127556, GE) was used for affinity capture of IgG, then high-concentration antigen 1 (hPDL1 100 nM or hTIGIT 100 nM) flowed through the surface of the chip over 180 s to saturation of sites on the antibody for the antigen 1, followed by the injection of antigen 2 (hTIGIT or hPDL1), and a Biacore T200 instrument was used for real-time detection of reaction signals, so that an association-dissociation curve was obtained. After dissociation was completed for each cycle, the biosensor chip was washed with 10 mM Gly-HCl at pH 1.5 for regeneration. Data were fitted using a 1:1 model. The results are shown in Table 34 below:

**Table 34. Assay results for affinity of the test antibodies hPDL1 and hTIGIT**

| Antibody | Antigen 1 | Antigen 2 (analyzed antigen) | ka (1/Ms) | kd (1/s) | KD (M) |
|---|---|---|---|---|---|
| P-O-T | | hPDL1 | 3.71E+06 | 9.53E-05 | 2.57E-11 |
| P-O-T | TIGIT | hPDL1 | 2.68E+06 | 8.12E-05 | 3.03E-11 |
| P-O-T | | TIGIT | 1.27E+06 | 1.23E-04 | 9.70E-11 |
| P-O-T | hPDL1 | TIGIT | 1.26E+06 | 1.20E-04 | 9.54E-11 |
| P-IgG1 | | hPDL1 | 2.08E+06 | 8.67E-05 | 4.17E-11 |
| T-IgG1 | | TIGIT | 2.14E+06 | 1.39E-04 | 6.51E-11 |

The assay results showed that the (FabV)₂-IgG bispecific antibody P-O-T in which CH1/CL was replaced with a Titin-T chain/Obscurin-O chain had good binding ability to hPDL1 and hTIGIT, and meanwhile, the binding of the P-O-T to one antigen did not affect the binding of the P-O-T to the other antigen.

### III. Assay of blocking effect of PDL1-TIGIT bispecific antibodies in which CH1/CL is replaced on the binding of PD-L1 to PD-1 and the binding of TIGIT to CD155

The *in vitro* cell assay described below can be used to determine the blocking effect of test antibodies on the binding of PD-L1 to PD-1 and TIGIT to CD155, the activity of which can be expressed as EC₅₀ values. On the first day of the experiment, CHOK1/PD-L1 cells (Promega, CS187108) stably expressing human CD155 were seeded in a 96-well plate with F-12 Nutrient Mixture (Gibco, 11765-054) complete medium containing 10% FBS (Gibco, 10099-141) and antibiotics at a density of 4000 cells/well, with 100 µL of cell suspension in each well. The plate was placed in a cell incubator at 37 °C with 5% CO₂ for culturing overnight. The next day, the medium in the plate was discarded by pipetting, 40 µL of test antibody diluted in a gradient formulated with RPMI (Gibco, 11875119) containing 2% FBS (Gibco, 10099-141) and 40 µL of Jurkat/PD-1/NFAT-luc2 cells (Promega, CS187102) stably expressing human TIGIT and human CD226 resuspended in the same medium were added to each well, with 50,000 cells in each well. The final concentration of the antibody was 9 concentration points obtained by 3-fold gradient dilution from 100 nM, and antibody-free control cell wells and cell-free control wells were set. The plate was placed in an incubator at 37 °C with 5% CO₂ for culturing for 6 h. After 6 h, the 96-well cell culture plate was taken, and 40 µL of the substrate in Bio-Glo^{™} Luciferase Assay (Promega, G7573) was added to each well. After the plate was left to stand for 10 min, luminescence signal values were read on a microplate reader (PerkinElmer, VICTOR 3). The relative activation level was calculated for each concentration of the antibody by control wells. Curve fitting was performed using GraphPad Prism based on log concentration and the relative activation level of the antibody, and the EC₅₀ values were calculated.

The results are shown in FIG. 18. The experimental results showed that the PDL1-TIGIT bispecific antibodies in which CH1/CL is replaced had superimposed blocking activity as compared to the PDL1 monoclonal antibody and the TIGIT monoclonal antibody.

### IV. Activation of antibody on tuberculin-stimulated peripheral blood mononuclear cells

The *in vitro* cell assay described below was used to determine the activation of the test antibodies on the IFN-γ secretion by tuberculin-stimulated peripheral blood mononuclear cells (PBMCs), the activity of which can be expressed as EC₅₀ values. On the first day of the experiment, PBMCs were separated from fresh healthy human blood using a SepMateTM-50 separation tube (STEMCELL, 86450) according to the instructions for use. The cells were seeded in a 6-well plate with a RPMI medium (Gibco, 11875119) containing 10% FBS (Gibco, 10099-141) and tuberculin subjected to 800-fold dilution (Synbiotics, 97-8800) at a density of 4×10⁶ cells/well, with 2 mL of cell suspension in each well. The plate was placed in an incubator at 37 °C with 5% CO₂ cell for culturing for 5 days. On the fourth day, a human CD155/PVR protein (Aero, CD5-H5223) was diluted to 2.5 µg/mL with PBS and added to a 96-well cell culture plate at 100 µL/well, and the plate was placed in a refrigerator at 4 °C for coating overnight. On the fifth day, the protein-coated 96-well cell culture plate was washed twice with PBS, and tuberculin-stimulated PBMCs were collected and seeded into the 96-well plate with a RPMI medium containing 10% FBS at a density of 1×10⁵ cells/well, with 90 µL of cell suspension in each well. Then, 10 µL of the test antibody diluted with PBS was added to each well. The final concentration of the antibody was 9 concentration points obtained by 3-fold serial dilution from 200 nM. Antibody-free control cell wells were set. The well plate was placed in a cell incubator at 37 °C with 5% CO₂ for culturing for 3 days. On the eighth day, the 96-well cell culture plate was centrifuged at 250 g for 5 min, the supernatant was transferred to a new 96-well plate and subjected to 20-fold dilution with a Human IFN-γ ELISA kit (NeoBioscience, EHC102g.96.10). The content of IFN-γ in the supernatant was determined. The relative activation level was calculated for the antibody treatment group by control wells. Curve fitting was performed using Graphpad Prism 5 based on the concentration and the corresponding relative activation level of the antibody, and the EC₅₀ values were calculated.

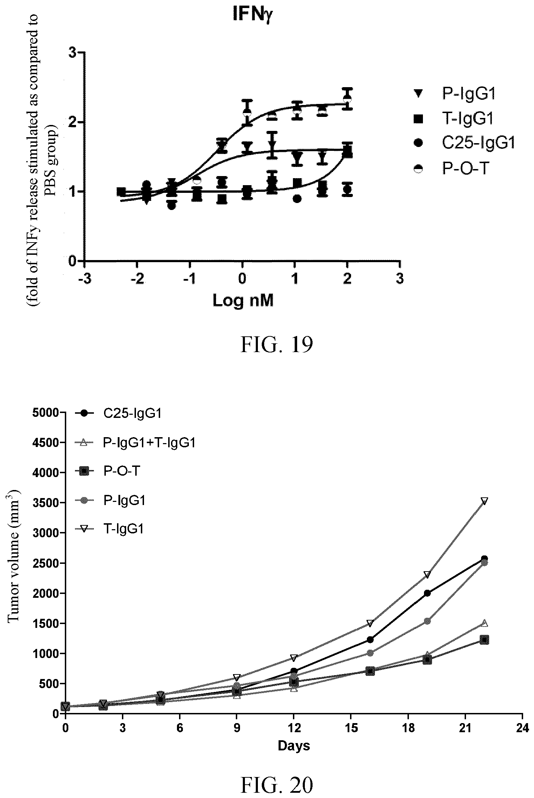

The experimental results are shown in FIG. 19. The experimental results showed that the PDL1-TIGIT bispecific antibodies in which CH1/CL is replaced could better block immunosuppression and promote the secretion of IFN-γ as compared to the monoclonal antibodies.

### V. Assay of in vivo efficacy of PDL1-TIGIT bispecific antibodies in which CH1/CL is replaced

In this experiment, human PD-1-TIGIT double-transgenic mice were inoculated with MC38-HL1 cells stably transfected with human PD-L1, and grouped when the tumor formed, so as to compare the efficacy of a combination of PD-L1 and TIGIT with that of the PDL1-TIGIT bispecific antibody.

82 female PD-1-TIGIT double-transgenic mice, aged 6-8 weeks and weighing about 16-18 g, were purchased from Biocytogen, and housed at 5 mice/cage in an SPF-grade environment at the temperature of 20-25 °C and humidity of 40-60%, with license number of SCXK (Beijing) 2015-0008. The mice were acclimatized for about 10 days. 100 µL of MC38-HL1 cells (2.0×10⁵ cells) were subcutaneously inoculated into the right flank of 82 hPD-1/TIGIT double-transgenic mice. After the tumor (about 110 mm³) formed, the mice with too large or too small tumor were removed, and the rest of mice were randomly divided into 5 groups according to the tumor volume, i.e., negative control C25-IgG1 (irrelevant target IgG1 protein) group, P-IgG1 + T-IgG1 group, P-O-T group, P-IgG1 group, and T-IgG1 group, with 8 mice in each group. The specific information is shown in Table 35. On the day of the experiment, the antibody was administered by intraperitoneal injection once every week for a total of 3 weeks. The tumor volumes and body weights were measured twice a week, and data were recorded.

**Table 35. Grouping for tumor inhibition experiment of xenograft tumors in MC38-HL1 mice**

| Group | Dose of administration (mg/kg) | Route of administration | | Period of administration | |
|---|---|---|---|---|---|
| C25-IgG1 | 9 mg/kg | i.p | qw | | 21 days |
| P-IgG1+T-IgG1 | P-IgG1 9mg/kg +T-IgG1 9mg/kg | i.p | qw | | 21 days |
| P-O-T | 5 mg/kg | i.p | qw | | 21 days |
| P-IgG1 | 9 mg/kg | i.p | qw | | 21 days |
| T-IgG1 | 9 mg/kg | i.p | qw | | 21 days |

| | | | | | |
|---|---|---|---|---|---|
| Note: In the table, i.p indicates intraperitoneal injection, and qw indicates once a week. | | | | | |

Data were recorded using Excel statistical software: the average values were calculated as avg; the SD values were calculated as STDEV; the SEM values were calculated as STDEV/SQRT (number of animals per group); GraphPad Prism software was used for plotting, and statistical analysis of the data was performed using Two-way ANOVA, One-way ANOVA, or t-test.

Tumor volume (V) was calculated as: V = 1/2 × L_{long} × Lₛₕₒᵣₜ²
Relative tumor proliferation rate T/C (%) = (T - T₀)/(C - C₀) × 100%, where T and C are the tumor volumes of animals at the end of the experiment in the treatment group and control group, respectively; T₀ and C₀ are the tumor volumes of animals at the beginning of the experiment in the treatment group and control group, respectively.

The results are shown in Table 36 and FIG. 20.

**Table 36. Tumor growth inhibition (%) of antibodies against xenograft tumors in MC38-HL1 mice**

| Group | Tumor growth inhibition (TGI %) |
|---|---|
| C25-IgG1 | / |
| P-IgG1 +T-IgG1 | 43% |
| P-O-T | 55% |
| P-IgG1 | 3% |
| T-IgG1 | -39% |

The experimental result showed that on day 22, as compared to the negative control C25-IgG1 group, the combination group of P-IgG1 at 9 mg/kg and T -IgG 1 at 9 mg/kg had the tumor growth inhibition of 43%, and the group of P-O-T at 5 mg/kg had the tumor inhibition rate of 55%. Therefore, they could significantly inhibit the growth of MC38-HL1 tumors, with a tumor inhibition effect being significantly superior to that of the groups of P-IgG1 alone and T-IgG1 alone.

## Claims

1. A dimerized polypeptide comprising a Titin-T chain and an Obscurin-O chain, or a Titin-T chain and an Obscurin-like-O chain, wherein,
i) the Titin-T chain is a variant of SEQ ID NO: 32, wherein the variant has amino acid residue substitutions at one or more positions selected from the group consisting of positions 60 and 64 as compared to SEQ ID NO: 32, and/or
ii) the Obscurin-O chain is a variant of SEQ ID NO: 33, wherein the variant has amino acid residue substitutions at one or more positions selected from the group consisting of positions 13, 32, 48, 66, 82, and 93 as compared to SEQ ID NO: 33;
with the proviso that:
a) when the variant does not have an amino acid residue substitution at position 13, 48, 66, 82, or 93, and has an amino acid residue substitution at position 32, the amino acid substitution at position 32 is not 32P;
b) when the variant does not have an amino acid residue substitution at position 32, 48, 66, 82, or 93, and has an amino acid residue substitution at position 13, the amino acid substitution at position 13 is not 13Y; and
c) when the variant does not have an amino acid residue substitution at position 48, 66, 82, or 93, and has amino acid residue substitutions at positions 13 and 32, the amino acid residue substitution at position 13 is not 13Y, and the amino acid residue substitution at position 32 is not 32P.

2. The dimerized polypeptide according to claim 1, wherein the variant of SEQ ID NO: 32 has one or more amino acid residue substitutions selected from the group consisting of 60S and 64T, and/or the variant of SEQ ID NO: 33 has one or more amino acid residue substitutions selected from the group consisting of 13S, 32F, 48V, 66C, 82H, and 93C;
preferably, the variant of SEQ ID NO: 32 has amino acid residue substitutions of 60S and 64T, and/or the variant of SEQ ID NO: 33 has amino acid residue substitutions selected from any one of a) to c):
a) 32F and 48V;
b) 13S, 32F, 48V, and 82H; and
c) 13S, 32F, 48V, 66C, 82H, and 93C.

3. The dimerized polypeptide according to claim 1 or 2, wherein the variant of SEQ ID NO: 32 further has amino acid residue substitutions at one or more positions selected from the group consisting of positions 3, 8, 11, 13, 20, 22, 25, 26, 39, 40, 42, 45, 47, 49, 56, 58, 66, 70, 75, 77, 79, 81, 82, 83, and 84 as compared to SEQ ID NO: 32;
preferably, the variant of SEQ ID NO: 32 further comprises one or more amino acid residue substitutions selected from the group consisting of 3W, 8C, 11I, 13L, 20C, 22M/22C, 25S, 26C, 39T, 40S, 42K, 45S, 47E, 49G, 56S, 58E, 66S/66K, 70R, 75V, 77S, 79T, 81R, 82M, 83D, and 84L, as compared to SEQ ID NO: 32;
more preferably, the variant of SEQ ID NO: 32 further comprises amino acid residue substitutions selected from any one of a) to l) as compared to SEQ ID NO: 32:
a) 8C, 25S, and 39T;
b) 20C, 25S, and 39T;
c) 25S, 26C, and 39T;
d) 22C, 25S, and 39T;
e) 8C, 25S, 39T, 66S, and 77S;
f) 8C, 25S, 39T, 66K, 70R, 79T, and 81R;
g) 3W, 8C, 11I, 13L, 22M, 25S, 39T, and 82M;
h) 8C, 11I, 25S, 39T, 66K, 79T, and 81R;
i) 8C, 25S, 39T, 40S, 42K, 45S, 47E, 49G, 56S, 58E, 75V, 83D, and 84L;
j) 8C, 25S, 39T, 47E, 49G, 56S, 58E, and 75V;
k) 8C, 25S, 39T, 56S, 58E, and 75V; and
l) 8C, 25S, 39T, 56S, 58E, 66S, and 77S;
most preferably, the variant of SEQ ID NO: 32 has amino acid residue substitutions selected from any one of A) to C) as compared to SEQ ID NO: 32:
A) 8C, 11I, 25S, 39T, 60S, 64T, 66K, 79T, and 81R;
B) 8C, 11I, 20C, 25S, 39T, 60S, 64T, 66K, 79T, and 81R; and
C) 8C, 11I, 25S, 26C, 39T, 60S, 64T, 66K, 79T, and 81R.

4. The dimerized polypeptide according to any one of claims 1 to 3, wherein the variant of SEQ ID NO: 33 further has amino acid residue substitutions at one or more positions selected from the group consisting of positions 2, 3, 7, 9, 11, 12, 13, 14, 17, 20, 22, 25, 30, 32, 34, 36, 41, 42, 44, 45, 53, 58, 62, 67, 69, 76, 88, 89, 92, 94, and 97 as compared to SEQ ID NO: 33;
preferably,
the variant of SEQ ID NO: 33 further has one or more amino acid residue substitutions selected from the group consisting of 2E, 3C, 7K/7R, 9C, 11L, 12S, 13Y, 14T, 17E, 20L, 22M/22S, 25S, 30D, 32P, 34E, 36T, 41K, 42L, 44I, 45T, 53L, 58V, 62E/62K/62H, 67Q/67T, 69S, 76S, 88C, 89L, 92E, 94G, and 97G as compared to SEQ ID NO: 33;
more preferably, the variant of SEQ ID NO: 33 further has amino acid residue substitutions selected from any one of A)-R) as compared to SEQ ID NO: 33:
A) 88C;
B) 3C;
C) 9C;
D) 25S, 76S, and 88C;
E) 25S, 76S, and 3C;
F) 25S, 76S, and 9C;
G) 7K, 25S, 62K, 76S, and 88C;
H) 7K, 25S, 62H, 76S, and 88C;
I) 7R, 25S, 62K, 76S, and 88C;
G) 7R, 25S, 62H, 76S, and 88C;
K) 11L, 25S, 62K, 76S, and 88C;
L) 11L, 25S, 62H, 76S, and 88C;
M) 12S, 13Y, 14T, 22S, 25S, 62K, 76S, and 88C;
N) 2E, 11L, 17E, 25S, 30D, 32P, 34E, 36T, 44I, 45T, 58V, 62E, 67Q, 69S, 76S, 88C, and 97G;
O) 11L, 20L, 22M, 25S, 53L, 62K, 76S, and 88C;
P) 11L, 25S, 41K, 45T, 62K, 67Q, 69S, 76S, 88C, and 89L;
Q) 11L, 25S, 42L, 45T, 62K, 67T, 69S, 76S, 88C, 92E, and 94G; and
R) 11L, 12S, 13Y, 22S, 25S, 42L, 45T, 62K, 67Q, 69S, 76S, 88C, 92E, and 94G;
most preferably, the variant of SEQ ID NO: 33 has amino acid residue substitutions selected from any one of a) to j) as compared to SEQ ID NO: 33:
a) 25S, 32F, 41K, 45T, 48V, 62K, 67Q, 69S, 76S, 88C, and 89L;
b) 13S, 25S, 32F, 41K, 45T, 48V, 62K, 67Q, 69S, 76S, 82H, 88C, and 89L;
c) 3C, 13S, 25S, 32F, 41K, 45T, 48V, 62K, 67Q, 69S, 76S, 82H, 88C, and 89L;
d) 9C, 13S, 25S, 32F, 41K, 45T, 48V, 62K, 67Q, 69S, 76S, 82H, 88C, and 89L;
e) 13S, 32F, 41K, 45T, 48V, 62K, 67Q, 69S, 82H, 88C, and 89L;
f) 3C, 13S, 32F, 41K, 45T, 48V, 62K, 67Q, 69S, 82H, 88C, and 89L;
g) 9C, 13S, 32F, 41K, 45T, 48V, 62K, 67Q, 69S, 82H, 88C, and 89L;
h) 13S, 25S, 32F, 41K, 45T, 48V, 62K, 66C, 67Q, 69S, 76S, 82H, 88C, 89L, and 93C;
i) 3C, 13S, 25S, 32F, 41K, 45T, 48V, 62K, 66C, 67Q, 69S, 76S, 82H, 88C, 89L, and 93C; and
j) 9C, 13S, 25S, 32F, 41K, 45T, 48V, 62K, 66C, 67Q, 69S, 76S, 82H, 88C, 89L, and 93C.

5. The dimerized polypeptide according to any one of claims 1 to 3, wherein the Obscurin-like-O chain is SEQ ID NO: 34 or a variant thereof, wherein the variant of SEQ ID NO: 34 has amino acid residue substitutions at one or more positions selected from the group consisting of positions 6, 26, 74, 77, 84, and 86;
preferably, the variant of SEQ ID NO: 34 has one or more amino acid residue substitutions selected from the group consisting of 6E, 26S, 74C, 77S, 84C, and 86C; more preferably, the variant of SEQ ID NO: 34 has amino acid residue substitutions selected from any one of A) to F):
A) 6E and 74C;
B) 6E and 84C;
C) 6E and 86C;
D) 6E, 26S, 77S, and 74C;
E) 6E, 26S, 77S, and 84C; and
F) 6E, 26S, 77S, and 86C.

6. The dimerized polypeptide according to any one of claims 1 to 4, wherein,
the Titin-T chain is a variant of SEQ ID NO: 32, 68, or 127, wherein the variant has one or more amino acid residue substitutions selected from the group consisting of 60S and 64T; and the Obscurin-O chain is a variant of SEQ ID NO: 33, 80, or 128, wherein the variant has one or more amino acid residue substitutions selected from the group consisting of 13S, 32F, 48V, 66C, 82H, and 93C;
preferably, the Titin-T chain has at least 85% sequence identity to any one of amino acid sequences of SEQ ID NO: 129 to SEQ ID NO: 131, and the Obscurin-O chain has at least 85% sequence identity to any one of amino acid sequences of SEQ ID NO: 132 to SEQ ID NO: 141;
more preferably, the Titin-T chain has an amino acid sequence set forth in any one of SEQ ID NO: 129 to SEQ ID NO: 131, and the Obscurin-O chain has an amino acid sequence set forth in any one of SEQ ID NO: 132 to SEQ ID NO: 141.

7. An antigen-binding molecule comprising the dimerized polypeptide according to any one of claims 1 to 6.

8. The antigen-binding molecule of claim 7, comprising a first antigen-binding moiety, wherein the first antigen-binding moiety comprises a domain-engineered Fab, wherein the domain-engineered Fab comprises a heavy chain variable region VH1, a light chain variable region VL1, and the dimerized polypeptide, but does not comprise a light chain constant region CL or a heavy chain constant region CH1, wherein the VH1 and the VL1 are each linked to any one of the peptide chains of the dimerized polypeptide via a linker;
preferably, the C-terminus of the VH1 is fused to the N-terminus of the Titin-T chain of the dimerized polypeptide according to any one of claims 1 to 6 via a linker, and the C-terminus of the VL1 is fused to the N-terminus of the Obscurin-O chain or Obscurin-like-O chain of the dimerized polypeptide according to any one of claims 1 to 6 via a linker; or
the C-terminus of VL1 is fused to the N-terminus of the Titin-T chain of the dimerized polypeptide according to any one of claims 1 to 6 via a linker, and the C-terminus of VH1 is fused to the N-terminus of the Obscurin-O chain or Obscurin-like-O chain of the dimerized polypeptide according to any one of claims 1 to 6 via a linker.

9. The antigen-binding molecule according to claim 7 or 8, comprising a first antigen-binding moiety, wherein the first antigen-binding moiety comprises:
a. a peptide chain of [VH1]-[linker 1]-[Titin-T chain] in order from the N-terminus to the C-terminus, and a peptide chain of [VL1]-[linker 2]-[Obscurin-O chain or Obscurin-like-O chain] in order from the N-terminus to the C-terminus; or
b. a peptide chain of [VH1]-[linker 1]-[Obscurin-O chain or Obscurin-like-O chain] in order from the N-terminus to the C-terminus, and a peptide chain of [VL1]-[linker 2]-[Titin-T chain] in order from the N-terminus to the C-terminus;
wherein the linker 1 and the linker 2 are identical or different;
the Titin-T chain and the Obscurin-O chain or the Obscurin-like-O chain are as defined in any one of claims 1 to 6;
preferably,
A) the linker 1 and the linker 2 are both (GₓS)_{y} linkers, wherein x is selected from the group consisting of integers from 1 to 5, and y is selected from the group consisting of integers from 0 to 6, or
B) the linker 1 is a C-terminal truncated sequence of CH1, and the linker 2 is a C-terminal truncated sequence of CL;
more preferably,
A) the linker 1 is set forth in SEQ ID NO: 173; the linker 2 is set forth in SEQ ID NO: 174; or
B) the linker 1 and the linker 2 are both set forth in SEQ ID NO: 175; or
C) the linker 1 and the linker 2 are both set forth in SEQ ID NO: 176.

10. The antigen-binding molecule according to any one of claims 7 to 9, further comprising an Fc region, wherein the Fc region comprises a first subunit Fc1 and a second subunit Fc2 capable of associating with each other;
preferably, the Fc region has one or more amino acid substitutions that reduce homodimerization; and/or the Fc region has one or more amino acid substitutions capable of reducing binding of the Fc region to an Fc receptor;
more preferably, the Fc1 has a protuberance structure according to the knob-and-hole technique, and the Fc2 has a pore structure according to the knob-and-hole technique; or the Fc2 has a protuberance structure according to the knob-and-hole technique, and the Fc1 has a pore structure according to the knob-and-hole technique;
most preferably, the Fc1 has a sequence set forth in SEQ ID NO: 177, and the Fc2 has a sequence set forth in SEQ ID NO: 178; or the Fc1 has a sequence set forth in SEQ ID NO: 178, and the Fc2 has a sequence set forth in SEQ ID NO: 177.

11. The antigen-binding molecule according to any one of claims 7 to 10, comprising the first antigen-binding moiety and a second antigen-binding moiety, wherein the second antigen-binding moiety comprises a heavy chain variable region VH2 and a light chain variable region VL2, and the first antigen-binding moiety and the second antigen-binding moiety bind to different antigens or different epitopes on the same antigen;
preferably, the second antigen-binding moiety comprises a Fab.

12. The antigen-binding molecule according to claim 11, comprising a first heavy chain, a first light chain, a second heavy chain, and a second light chain; wherein
a. the first heavy chain comprises [VH1]-[linker 1]-[Titin-T chain]-[linker 3]-[Fc1] in order from the N-terminus to the C-terminus,
the first light chain comprises [VL1]-[linker 2]-[Obscurin-0 chain or Obscurin-like-O chain] in order from the N-terminus to the C-terminus,
the second heavy chain comprises [VH2]-[CH1]-[Fc2] in order from the N-terminus to the C-terminus, and
the second light chain comprises [VL2]-[CL] in order from the N-terminus to the C-terminus; or
b. the first heavy chain comprises [VH1]-[linker 1]-[Obscurin-O chain or Obscurin-like-O chain]-[linker 3]-[Fc1] in order from the N-terminus to the C-terminus, the first light chain comprises [VL1]-[linker 2]-[Titin-T chain] in order from the N-terminus to the C-terminus,
the second heavy chain comprises [VH2]-[CH1]-[Fc2] in order from the N-terminus to the C-terminus, and
the second light chain comprises [VL2]-[CL] in order from the N-terminus to the C-terminus;
wherein the linker 1, the linker 2, and the linker 3 are identical or different;
preferably, the Fc1 and the Fc2 each independently have one or more amino acid substitutions that reduce homodimerization;
more preferably,
A) the linker 1, the linker 2, and the linker 3 are all (GₓS)_{y}, wherein x is selected from the group consisting of integers from 1 to 5, and y is selected from the group consisting of integers from 0 to 6, or
B) the linker 1 is a C-terminal truncated sequence of CH1, the linker 2 is a C-terminal truncated sequence of CL, and the linker 3 is (GₓS)_{y}, wherein x is selected from the group consisting of integers from 1 to 5, and y is selected from the group consisting of integers from 0 to 6;
most preferably,
A) the linker 1 is set forth in SEQ ID NO: 173; the linker 2 is set forth in SEQ ID NO: 174; linker 3 is a bond; or
B) the linker 1 and the linker 2 are both set forth in SEQ ID NO: 175; the linker 3 is a bond; or
C) the linker 1 and the linker 2 are both set forth in SEQ ID NO: 176; the linker 3 is a bond.

13. The antigen-binding molecule according to claim 12, wherein:
(I) the antigen-binding molecule is capable of binding to NGF and RANKL;
preferably,
the antigen-binding molecule comprises a first heavy chain, a first light chain, a second heavy chain, and a second light chain, wherein:
the first heavy chain comprises [VH1]-[linker 1]-[Obscurin-O chain]-[linker 3]-[Fc1] in order from the N-terminus to the C-terminus,
the first light chain comprises [VL1]-[linker 2]-[Titin-T chain] in order from the N-terminus to the C-terminus,
the second heavy chain comprises [VH2]-[CH1]-[Fc2] in order from the N-terminus to the C-terminus, and
the second light chain comprises [VL2]-[CL] in order from the N-terminus to the C-terminus;
wherein: the VH1 forms a first antigen-binding moiety binding to NGF with the VL1, and the VH2 forms a second antigen-binding moiety binding to RANKL with the VL2; or
the VH1 forms a first antigen-binding moiety binding to RANKL with the VL1, and the VH2 forms a second antigen-binding moiety binding to NGF with the VL2;
more preferably,
the VH1 has a sequence set forth in SEQ ID NO: 26, the VL1 has a sequence set forth in SEQ ID NO: 27, the VH2 has a sequence set forth in SEQ ID NO: 24, and the VL2 has a sequence set forth in SEQ ID NO: 25; or
the VH1 has a sequence set forth in SEQ ID NO: 24, the VL1 has a sequence set forth in SEQ ID NO: 25, the VH2 has a sequence set forth in SEQ ID NO: 26, and the VL2 has a sequence set forth in SEQ ID NO: 27;
and the Obscurin-O chain has a sequence set forth in any one of SEQ ID NOs: 132-141, and the Titin-T chain has a sequence set forth in any one of SEQ ID NOs: 129-131;
most preferably,
the Fc1 has a sequence set forth in SEQ ID NO: 177; the Fc2 has a sequence set forth in SEQ ID NO: 178; the CH1 has a sequence set forth in SEQ ID NO: 179; the CL has a sequence set forth in SEQ ID NO: 4; the linker 3 is a bond; the linker 1 and the linker 2 are selected from the group consisting of: a) linker 1 and linker 2 both set forth in SEQ ID NO: 175; and b) linker 1 set forth in SEQ ID NO: 173 and linker 2 set forth in SEQ ID NO: 174;
(II) the antigen-binding molecule is capable of binding to PDL1 and CTLA4;
preferably,
the antigen-binding molecule comprises a first heavy chain, a first light chain, a second heavy chain, and a second light chain, wherein:
the first heavy chain comprises [VH1]-[linker 1]-[Obscurin-O chain]-[linker 3]-[Fc1] in order from the N-terminus to the C-terminus,
the first light chain comprises [VL1]-[linker 2]-[Titin-T chain] in order from the N-terminus to the C-terminus,
the second heavy chain comprises [VH2]-[CH1]-[Fc2] in order from the N-terminus to the C-terminus, and
the second light chain comprises [VL2]-[CL] in order from the N-terminus to the C-terminus; wherein:
the VH1 forms a first antigen-binding moiety binding to PDL1 with the VL1, and the VH2 forms a second antigen-binding moiety binding to CTLA4 with the VL2; or
the VH1 forms a first antigen-binding moiety binding to CTLA4 with the VL1, and the VH2 forms a second antigen-binding moiety binding to PDL1 with the VL2;
more preferably,
the VH1 has a sequence set forth in SEQ ID NO: 156, the VL1 has a sequence set forth in SEQ ID NO: 155, the VH2 has a sequence set forth in SEQ ID NO: 169, and the VL2 has a sequence set forth in SEQ ID NO: 170; or
the VH1 has a sequence set forth in SEQ ID NO: 169, the VL1 has a sequence set forth in SEQ ID NO: 170, the VH2 has a sequence set forth in SEQ ID NO: 156, and the VL2 has a sequence set forth in SEQ ID NO: 155;
and the Obscurin-O chain has a sequence set forth in any one of SEQ ID NOs: 132-141, and the Titin-T chain has a sequence set forth in any one of SEQ ID NOs: 129-131; most preferably,
the Fc1 has a sequence set forth in SEQ ID NO: 178; the Fc2 has a sequence set forth in SEQ ID NO: 177; the CH1 has a sequence set forth in SEQ ID NO: 179; the CL has a sequence set forth in SEQ ID NO: 4; the linker 3 is a bond; the linker 1 and the linker 2 are selected from the group consisting of: a) linker 1 and linker 2 both set forth in SEQ ID NO: 175; and b) linker 1 set forth in SEQ ID NO: 173 and linker 2 set forth in SEQ ID NO: 174; or
(III) the antigen-binding molecule is capable of binding to IL5 and TSLP;
preferably,
the antigen-binding molecule comprises a first heavy chain, a first light chain, a second heavy chain, and a second light chain, wherein:
the first heavy chain comprises [VH1]-[linker 1]-[Titin-T chain]-[linker 3]-[Fc1] in order from the N-terminus to the C-terminus;
the first light chain comprises [VL1]-[linker 2]-[Obscurin-O chain] in order from the N-terminus to the C-terminus;
the second heavy chain comprises [VH2]-[CH1]-[Fc2] in order from the N-terminus to the C-terminus; and
the second light chain comprises [VL2]-[CL] in order from the N-terminus to the C-terminus; wherein:
the VH1 forms a first antigen-binding moiety binding to IL5 with the VL1, and the VH2 forms a second antigen-binding moiety binding to TSLP with the VL2; or
the VH1 forms a first antigen-binding moiety binding to TSLP with the VL1, and the VH2 forms a second antigen-binding moiety binding to IL5 with the VL2;
more preferably,
the VH1 has a sequence set forth in SEQ ID NO: 16, the VL1 has a sequence set forth in SEQ ID NO: 17, the VH2 has a sequence set forth in SEQ ID NO: 171, and the VL2 has a sequence set forth in SEQ ID NO: 172; or
the VH1 has a sequence set forth in SEQ ID NO: 171, the VL1 has a sequence set forth in SEQ ID NO: 172, the VH2 has a sequence set forth in SEQ ID NO: 16, and the VL2 has a sequence set forth in SEQ ID NO: 17;
and the Obscurin-O chain has a sequence set forth in any one of SEQ ID NOs: 132-141,
and the Titin-T chain has a sequence set forth in any one of SEQ ID NOs: 129-131; most preferably,
the Fc1 has a sequence set forth in SEQ ID NO: 178; the Fc2 has a sequence set forth in SEQ ID NO: 177; the CH1 has a sequence set forth in SEQ ID NO: 179; the CL has a sequence set forth in SEQ ID NO: 4; the linker 3 is a bond; the linker 1 and the linker 2 are selected from the group consisting of: a) linker 1 and linker 2 both having sequences set forth in SEQ ID NO: 175; and b) linker 1 having a sequence set forth in SEQ ID NO: 173 and linker 2 having a sequence set forth in SEQ ID NO: 174.

14. The antigen-binding molecule according to claim 11, comprising:
a. a first heavy chain comprising [VH1]-[linker 1]-[Titin-T chain]-[linker 3]-[VH2]-[CH1]-[Fc1] in order from the N-terminus to the C-terminus;
a second heavy chain comprising [VH1]-[linker 1]-[Titin-T chain]-[linker 3]-[VH2]-[CH1]-[Fc2] in order from the N-terminus to the C-terminus;
a first light chain comprising [VL1]-[linker 2]-[Obscurin-O chain or Obscurin-like-O chain] in order from the N-terminus to the C-terminus; and
a second light chain comprising [VL2]-[CL] in order from the N-terminus to the C-terminus; or
b. a first heavy chain comprising [VH1]-[linker 1]-[Obscurin-O chain or Obscurin-like-O chain]-[linker 3]-[VH2]-[CH1]-[Fc1] in order from the N-terminus to the C-terminus;
a second heavy chain comprising [VH1]-[linker 1]-[Obscurin-O chain or Obscurin-like-O chain]-[linker 3]-[VH2]-[CH1]-[Fc2] in order from the N-terminus to the C-terminus;
a first light chain comprising [VL1]-[linker 2]-[Titin-T chain] in order from the N-terminus to the C-terminus; and
a second light chain comprising [VL2]-[CL] in order from the N-terminus to the C-terminus;
wherein the linker 1, the linker 2, and the linker 3 are identical or different;
preferably, the Fc1 and the Fc2 are identical, or the Fc1 and the Fc2 each independently have one or more amino acid substitutions that reduce homodimerization;
more preferably,
A) the linker 1, the linker 2, and the linker 3 are all (GₓS)_{y} linkers, wherein x is selected from the group consisting of integers from 1 to 5, and y is selected from the group consisting of integers from 0 to 6; preferably, all are set forth in SEQ ID NO: 175 or SEQ ID NO: 176, or
B) the linker 1 is a C-terminal truncated sequence of CH1, and preferably, the linker 1 is set forth in SEQ ID NO: 173; the linker 2 is a C-terminal truncated sequence of CL, and preferably, the linker 2 is set forth in SEQ ID NO: 174; the linker 3 is a (GₓS)_{y} linker, wherein x is selected from the group consisting of integers from 1 to 5, and y is selected from the group consisting of integers from 0 to 6, and preferably, the linker 3 is set forth in SEQ ID NO: 175 or SEQ ID NO: 176;
most preferably,
the antigen-binding molecule is capable of binding to PDL1 and TIGIT.

15. An antigen-binding molecule comprising a first antigen-binding moiety capable of specifically binding to PDL1 and a second antigen-binding moiety capable of specifically binding to TIGIT, wherein the first antigen-binding moiety comprises a heavy chain variable region VH1 and a light chain variable region VL1, and the second antigen-binding moiety comprises a heavy chain variable region VH2 and a light chain variable region VL2; wherein,
the VH1 comprises HCDR1, HCDR2, and HCDR3 set forth in SEQ ID NOs: 163, SEQ ID NO: 164, and SEQ ID NO: 165, respectively, and the VL1 comprises LCDR1, LCDR2, and LCDR3 set forth in SEQ ID NO: 166, SEQ ID NO: 167, and SEQ ID NO: 168, respectively; and/or
the VH2 comprises HCDR1, HCDR2, and HCDR3 set forth in SEQ ID NO: 157, SEQ ID NO: 158, and SEQ ID NO: 159, respectively, and the VL2 comprises LCDR1, LCDR2, and LCDR3 set forth in SEQ ID NO: 160, SEQ ID NO: 161, and SEQ ID NO: 162, respectively;
preferably,
the VH1 has a sequence set forth in SEQ ID NO: 156 or a sequence having at least 90% sequence identity to SEQ ID NO: 156, and the VL1 has a sequence set forth in SEQ ID NO: 155 or a sequence having at least 90% sequence identity to SEQ ID NO: 155; and/or
the VH2 has a sequence set forth in SEQ ID NO: 154 or a sequence having at least 90% sequence identity to SEQ ID NO: 154, and the VL2 has a sequence set forth in SEQ ID NO: 153 or a sequence having at least 90% sequence identity to SEQ ID NO: 153; more preferably, the antigen-binding molecule has:
a heavy chain having a sequence set forth in SEQ ID NO: 148 or a sequence having at least 90% sequence identity to SEQ ID NO: 148;
a first light chain having a sequence set forth in SEQ ID NO: 146 or a sequence having at least 90% sequence identity to SEQ ID NO: 146; and
a second light chain having a sequence set forth in SEQ ID NO: 147 or a sequence having at least 90% sequence identity to SEQ ID NO: 147.

16. A domain-engineered antibody, being an antibody in which a heavy chain constant region CH1 and a light chain constant region CL are replaced with the dimerized polypeptide according to any one of claims 1 to 6, wherein
preferably, the heavy chain constant region CH1 is replaced with a Titin-T chain, and the light chain constant region CL is replaced with an Obscurin-O chain; or the light chain constant region CL is replaced with a Titin-T chain, and the heavy chain constant region CH1 is replaced with an Obscurin-O chain.

17. A pharmaceutical composition comprising the antigen-binding molecule according to any one of claims 7 to 15 or the domain-engineered antibody according to claim 16, and one or more pharmaceutically acceptable carriers, diluents, or excipients.

18. Use of the dimerized polypeptide according to any one of claims 1 to 6 in the reduction of light/heavy chain mispairings during preparation of a multispecific antibody,
preferably in the reduction of light chain/heavy chain mispairings during preparation of a bispecific antibody.

19. A nucleic acid molecule encoding the dimerized polypeptide according to any one of claims 1 to 6, the antigen-binding molecule according to any one of claims 7 to 15, or the domain-engineered antibody according to claim 16.

20. A host cell comprising the nucleic acid molecule according to claim 19.

21. A method for preparing the dimerized polypeptide according to any one of claims 1 to 6, the antigen-binding molecule according to any one of claims 7 to 15, or the domain-engineered antibody according to claim 16, comprising the steps of: culturing the host cell according to claim 20, and purifying and isolating the dimerized polypeptide, the antigen-binding molecule, or the domain-engineered antibody.

22. Use of the antigen-binding molecule according to any one of claims 7 to 15, the domain-engineered antibody according to claim 16, or the pharmaceutical composition according to claim 17 in the preparation of a medicament for treating or preventing a disease or condition.
